# EUROPÄISCHE PATENTSCHRIFT

(11) **EP 2 195 868 B1**
(45) Veröffentlichungstag und Bekanntmachung des Hinweises auf die Patenterteilung: **02.01.2013**
(21) Anmeldenummer: 08837984.7
(22) Anmeldetag: 26.09.2008
(51) Int. Cl.: H01L 51/54, H01L 51/50, H01L 51/00

(54) **VERWENDUNG VON ACRIDINDERIVATEN ALS MATRIXMATERIALIEN UND/ODER ELEKTRONENBLOCKER IN OLEDS**
USE OF ACRIDINE DERIVATIVES AS MATRIX MATERIALS AND/OR AN ELECTRON BLOCKER IN OLEDS
UTILISATION DE DÉRIVÉS D'ACRIDINE COMME MATÉRIAUX DE MATRICE ET/OU COMME BLOQUEURS D'ÉLECTRONS DANS DES DIODES ÉLECTROLUMINESCENTES ORGANIQUES

(30) Priorität: 02.10.2007 EP 07117770
(43) Veröffentlichungstag der Anmeldung: 16.06.2010
(73) Patentinhaber: BASF SE, 67056 Ludwigshafen (DE)
(72) Erfinder: KAHLE, Klaus, 67069 Ludwigshafen (DE); MOLT, Oliver, 69493 Hirschberg (DE); LANGER, Nicolle, 64646 Heppenheim (DE); LENNARTZ, Christian, 67105 Schifferstadt (DE); SCHILDKNECHT, Christian, 68305 Mannheim (DE); NORD, Simon, 67354 Römerberg (DE); FUCHS, Evelyn, 68199 Mannheim (DE); RUDOLPH, Jens, 67547 Worms (DE)
(86) Internationale Anmeldenummer: PCT/EP2008/062976
(87) Internationale Veröffentlichungsnummer: WO 2009/047147

(56) Entgegenhaltungen:
- EP-A- 1 840 120
- WO-A-2006/033563
- WO-A-2006/080640
- JP-A- 8 259 937
- US-A1- 2004 219 386

## Beschreibung

Die vorliegende Erfindung betrifft die Verwendung von (Hetero)Aryl-substituierten Acridinderivaten als Matrixmaterialien in einer Licht-emittierenden Schicht von organischen Leuchtdioden (OLEDs) und/oder als Elektronenblocker in OLEDs. Weiterhin betrifft die vorliegende Erfindung eine Licht-emittierende Schicht, die mindestens ein Emittermaterial und mindestens ein Matrixmaterial enthält, wobei als Matrixmaterial mindestens ein (Hetero)Aryl-substituiertes Acridinderivat eingesetzt wird, sowie eine organische Leuchtdiode, die mindestens eine erfindungsgemäße Licht-emittierende Schicht und/oder eine Blockschicht für Elektronen, die die (Hetero)Aryl-substituierten Acridinderivate gemäß der vorliegenden Anmeldung enthält, enthält und eine Vorrichtung ausgewählt aus stationären und mobilen Bildschirmen und Beleuchtungseinheiten, die mindestens eine erfindungsgemäße organische Leuchtdiode enthält.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignen sich Vorrichtungen enthaltend OLEDs insbesondere für mobile Anwendungen, zum Beispiel für Anwendungen in Handys, Laptops, Digitalkameras, sowie zur Beleuchtung, usw.

Die Grundprinzipien der Funktionsweise von OLEDs sowie geeignete Aufbauten (Schichten) von OLEDs sind dem Fachmann bekannt und zum Beispiel in WO 2005/113704 und der darin zitierten Literatur genannt. Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (Triplett-Emitter) (M. A. Baldow et al., Appl. Phys. Lett. 1999, 75, 4 - 6).

Aus quantenmechanischen Gründen ist bei der Verwendung der Triplett-Emitter (Phosphoreszenz-Emitter) eine bis zu vierfache Quanten-, Energie- und Leistungseffizienz möglich. Um die Vorteile des Einsatzes der metallorganischen Triplett-Emitter in die Praxis umzusetzen, ist es erforderlich, Device-Kompositionen bereitzustellen, die eine hohe operative Lebensdauer, eine hohe Stabilität gegenüber Temperaturbelastung und eine niedrige Einsatz- und Betriebsspannung aufweisen.

Solche Device-Kompositionen können zum Beispiel Matrixmaterialien enthalten, in denen der eigentliche Lichtemitter in verteilter Form vorliegt. Dabei hat die Auswahl des Matrixmaterials einen wesentlichen Einfluss unter anderem auf die Leuchtdichten- und Quantenausbeuten der OLEDs. Auch die Auswahl der weiteren Schichten, z. B. der Blockschicht für Elektronen, der OLEDs hat einen Einfluss auf die Performance der OLEDs, so dass es sinnvoll ist, z. B. die Blockschicht für Elektronen an den Lichtemitter anzupassen.

Im Stand der Technik werden zahlreiche verschiedene Materialien für den Einsatz in OLEDs vorgeschlagen. Unter den vorgeschlagenen Materialien sind auch (Hetero)Arylsubstituierte Acridinderivate, wobei der Einsatz von Acridinderivaten in verschiedenen Schichten der OLEDs offenbart ist.

So betrifft US 2004/0219386 A1 den Einsatz von heterogenen Spiroverbindungen in OLEDs, wobei diese als Licht-emittierende Schicht und/oder eine oder mehrere Ladungstransportschichten, oder als Wirtsmaterial (Matrixmaterial) für eine oder mehrere dieser Schichten eingesetzt werden können. Unter die Gesamtoffenbarung in US 2004/0219386 A1 fallen auch solche Spiroverbindungen, die Acridin-Einheiten aufweisen.

WO 2006/080640 beschreibt die Verwendung von Acridinderivaten als Matrixmaterialien in einer Licht-emittierenden Schicht von organischen Leuchtdioden.

JP 2004/171986 A2 betrifft OLEDs, worin polycyclische Verbindungen in der Elektronentransportschicht oder als Matrixmaterial in der Licht-emittierenden Schicht eingesetzt werden. Bei den polycyclischen Verbindungen handelt es sich um Spiroverbindungen, wobei fast alle Beispielverbindungen Spirobifluorene betreffen. Unter anderem ist jedoch ein Beispiel erwähnt, worin ein spirocyclisches Acridinderivat offenbart ist. Dieses Acridinderivat ist am Stickstoff mit einer Methylgruppe substituiert und weist in der 9-Position eine aliphatische Spiroverknüpfung zu einem weiteren Acridinderivat auf. Die in JP 2004/171986 A2 genannten polycyclischen Verbindungen sind als Matrixmaterial für Triplett-Emitter geeignet.

EP 1 341 403 A1 betrifft organische Leuchtdioden, die spezielle spirocyclische organische Verbindungen enthalten. Bei den spirocyclischen organischen Verbindungen kann es sich unter anderem um ein Acridinderivat handeln, das am Stickstoffatom mit einer Methylgruppe substituiert ist und in 9-Position eine Spiroverknüpfung zu einem Fluorenyl-Rest aufweist. Die in EP 1 341 403 A1 offenbarten Spiroverbindungen werden bevorzugt in der Elektronentransportschicht bzw. Lochblockierschicht eingesetzt.

Neben den vorstehend genannten Spiroverbindungen ist der Einsatz von nichtspirocyclischen Acridinderivaten in OLEDs ebenfalls im Stand der Technik bekannt. So betrifft JP 06-293883 A Verbindungen, die in ihrer Licht-emittierenden Schicht tricyclische organische Verbindungen aufweisen. Gemäß der allgemeinen Definition in JP 06-293883 A kann es sich bei den Verbindungen um Acridinderivate handeln. In JP 06-293883 A werden jedoch keine Beispiele für geeignete Acridinderivate offenbart, sondern lediglich Acridonderivate.

JP 07-109449 A betrifft den Einsatz von Verbindungen mit einem tricyclischen Grundkörper in OLEDs. Die in JP 07-109449 A offenbarten Verbindungen weisen die gleiche Grundstruktur auf, wie die in JP 06-293883 A offenbarten Verbindungen. Sie werden gemäß JP 07-109449 A in der Licht-emittierenden Schicht eingesetzt. Zwar umfasst die in JP 07-109449 A offenbarte Grundstruktur auch Acridinderivate, jedoch sind keine Beispiele für geeignete Acridinderivate offenbart, sondern lediglich Beispiele für Acridonderivate.

JP 08-259937 A betrifft ein Elektronentransportmaterial für OLEDs, wobei es sich um ein in 9-Position mit zwei Triarylresten substituiertes Acridinderivat handeln kann. Der Einsatz der in JP 08-259937 A offenbarten Verbindungen als Matrixmaterialien ist in JP 08-259937 A nicht offenbart.

JP 10-338871 A betrifft den Einsatz der bereits in JP 08-259937 A offenbarten Verbindungen in der Licht-emittierenden Schicht als elektrolumineszierendes Material. Der Einsatz der in JP 10-338871 A offenbarten Acridinderivate als Matrixmaterial ist in JP 10-338871 nicht offenbart.

JP 09-310066 A betrifft spezielle Aminverbindungen, die in der Lochtransportschicht eines OLEDs eingesetzt werden können. Bei den Aminverbindungen gemäß JP 09-310066A kann es sich um Acridinderivate handeln. Dabei tragen die in JP 09-310066 A offenbarten Acridinderivate in ihrer 9-Position Alkylreste, zum Beispiel Methylgruppen.

JP 10-226785 A betrifft den Einsatz von speziellen Triarylaminverbindungen als Lochinjektions oder Lochtransportmaterialien in organischen Leuchtdioden. Gemäß JP 10-226785 A können die Triarylaminverbindungen zu der Licht-emittierenden Schicht oder einer anderen Schicht (Lochinjektionsschicht oder Lochtransportschicht) des OLEDs gegeben werden. Bei den Triarylaminverbindungen gemäß JP 10-226785 A kann es sich unter anderem um Acridinderivate handeln, wobei diese in der 9-Position Alkylreste tragen, zum Beispiel Methylreste.

JP 2001-244076 A betrifft u. a. die Verwendung von über eine Arylgruppe verknüpften Bis-Acridinderivaten in organischen Leuchtdioden. Die in JP 2001-244076 A genannten Acridinderivate können unter anderem als Matrixmaterial in der Licht-emittierenden Schicht eingesetzt werden. Es werden jedoch in JP 2001-244076 A keine konkreten Acridinderivate (sondern lediglich Acridonderivate) erwähnt.

Damit OLEDs mit besonders guten Eigenschaften bereitgestellt werden können, ist es erforderlich, die in den OLEDs eingesetzten Materialien zu optimieren. Dabei ist es unter anderem sinnvoll, die in der Licht-emittierenden Schicht eingesetzten Matrixmaterialien oder die in einer Blockschicht für Elektronen eingesetzten Materialien auf das eingesetzte Emittermaterial abzustimmen. Aufgabe der vorliegenden Anmeldung ist es daher, neuartige Matrixmaterialien für den Einsatz in der Licht-emittierenden Schicht in OLEDs sowie Elektronenblocker für den Einsatz in OLEDs bereitzustellen, die bevorzugt als Matrixmaterialien und/oder Elektronenblocker für Triplett-Emitter dienen. Die Matrixmaterialien und Elektronenblocker sollen leicht zugänglich sein und in Kombination mit dem (den) eigentlichen Emitter(n) gute Leuchtdichten und Quantenausbeuten in OLEDs bewirken.

Diese Aufgabe wird gelöst durch die Verwendung von Acridinderivaten der allgemeinen Formel (I) als Matrixmaterialien in einer Licht-emittierenden Schicht und/oder Elektronenblocker in einer organischen Leuchtdiode 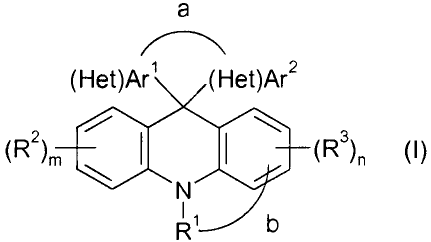 worin bedeuten:
- (Het)Ar¹, (Het)Ar²: unabhängig voneinander ein unsubstituierter oder substituierter Arylrest oder ein unsubstituierter oder substituierter Heteroarylrest;
- R¹: ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest;
- R², R³: unabhängig voneinander ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest oder ein Silylrest;
- n, m: unabhängig voneinander 0, 1, 2, 3 oder 4, wobei in dem Fall, wenn n bzw. m 0 sind, alle mit R² bzw. R³ substituierbaren Positionen des Acridin-Grundgerüsts Wasserstoff bedeuten; 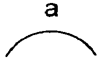 nicht vorhanden; 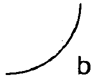 eine optionale Brücke aus 0, 1 oder 2 Atomen, die den Rest R¹ kovalent mit einem der substituierbaren Kohlenstoffatome des Acridin-Gerüsts verknüpft,
dadurch gekennzeichnet, dass die Acridinderivate der Formel (I) eine Energie des niedrigsten Triplettzustandes von mindestens 2,6 eV aufweisen, und als Matrixmaterialien und/oder Elektronenblocker für Triplett-Emitter eingesetzt werden.

Die erfindungsgemäß eingesetzten Verbindungen der Formel (I) werden in der Licht-emittierenden Schicht eines OLEDs gemeinsam mit dem eigentlichen Emitter eingesetzt. Der Emitter, der mit den Matrixmaterialien der Formel (I) gemeinsam in der Licht-emittierenden Schicht eines OLEDs eingesetzt wird, ist ein Triplett-Emitter. Des Weiteren können die Verbindungen der Formel (I) in einer Blockschicht für Elektronen eingesetzt werden, die bevorzugt in direkter Nachbarschaft (direktem Kontakt) zur Emitterschicht in den OLEDs vorliegt. Bei dem Emitter handelt es sich auch in diesem Fall um einen Triplett-Emitter.

Ein Gegenstand der vorliegenden Erfindung ist daher die Verwendung der Acridinderivate der Formel (I) als Matrixmaterialien für Triplett-Emitter.

Ein weiterer Gegenstand der vorliegenden Erfindung ist des Weiteren die Verwendung der Acridinderivate der Formel (I) in einer Blockschicht für Elektronen, wobei die Blockschicht für Elektronen in einer OLED vorliegt und in direktem Kontakt zu der Emitterschicht der OLED steht, wobei die Emitterschicht einen Triplett-Emitter enthält. Somit ist ein weiterer Gegenstand der vorliegenden Erfindung ist daher die Verwendung der Acridinderivate der Formel (I) als Elektronenblocker für Triplett-Emitter.

Die Acridinderivate der Formel (I) zeichnen sich insbesondere dadurch aus, dass sie in Kombination mit dem (den) eigentlichen Emitter(n) in Form von Matrixmaterial und/oder in Form von Elektronenblockern gute Leuchtdichten und Quantenausbeuten bei Einsatz in der Emitterschicht von OLEDs aufweisen. Weiterhin sind Acridinderivate der Formel (I) gut verdampfbar und bilden stabile amorphe Filme. Sie lassen sich außerdem synthetisch gut an den verwendeten Emitter anpassen.

Wesentlich für den Einsatz von organischen Verbindungen als Matrixmaterialien und/oder Elektronenblocker für Triplett-Emitter in organischen Leucht-Dioden ist die Energie des niedrigsten Triplettzustands. Diese weist in den erfindungsgemäß verwendeten Acridinderivaten der Formel (I) mindestens 2,6 eV auf, bevorzugt beträgt die Energie des niedrigsten Triplettzustands von 2,6 bis 3,3 eV, besonders bevorzugt von 2,7 bis 3,0 eV. Die Energie des niedrigsten Triplettzustands ist definiert als kurzwelligster Peak der Triplett-Emission.

Die vorstehend genannten Reste (Het)Ar¹, (Het)Ar², R¹, R² und R³ sowie die vorstehend genannten optionalen Brücken können unsubstituiert oder substituiert sein. In dem Fall, dass die vorstehend genannten Gruppen unsubstituiert sind, sind alle substituierbaren Positionen mit Wasserstoffatomen besetzt. In dem Fall, dass die vorstehend genannten Gruppen substituiert sind, können die Gruppen unabhängig voneinander einen oder mehrere Substituenten tragen, wobei die maximale Anzahl an Substituenten sich aus der Anzahl der substituierbaren Positionen in den jeweiligen vorstehend genannten Gruppen ergibt. Bevorzugte Ausführungsformen bezüglich der Substitution der vorstehend genannten Reste sind nachstehend genannt.

Geeignete Substituenten sind Alkylreste, die linear oder verzweigt sein können, Cycloalkylreste, Heterocycloalkylreste, Alkoxyreste, Cyanogruppen, Aryl- oder Heteroarylcarbonyloxy, Alkylcarbonyloxy, Silylreste, Arylreste oder Heteroarylreste. Bevorzugte Alkyl-, Alkoxy-, Cycloalkyl-, Heterocycloalkyl-, Silyl-, Aryl- und Heteroarylreste sind nachstehend genannt.

Besonders bevorzugt werden die folgenden Reste als Substituenten eingesetzt: C₁- bis C₈-Alkylreste, die linear oder verzweigt sein können, bevorzugt C₁- bis C₄-Alkylreste, die linear oder verzweigt sein können, besonders bevorzugt Methyl, Ethyl, n-Propyl, iso-Propyl, n-Butyl, iso-Butyl, sec-Butyl oder tert-Butyl. Die Alkylreste können unsubstituiert oder substituiert sein, wobei geeignete Substituenten bevorzugt aus der Gruppe bestehend aus Aryl, Alkoxy, Aryloxy und Halogen ausgewählt sind. Beispiele für als Substituenten geeignete substituierte Alkylreste sind CF₃, Phenoxymethyl und Benzyl. Weitere bevorzugte Substituenten sind C₆- bis C₁₄-Arylreste, die wiederum mit den für die Alkylreste genannten Substituenten substituiert oder unsubstituiert sein können, zum Beispiel Phenyl oder Biphenyl. Weitere bevorzugt geeignete Substituenten sind Heteroarylreste mit 5 bis 14 Ringatomen, die wiederum mit den vorstehend für die Alkylreste genannten Substituenten substituiert sein können oder unsubstituiert sein können. Bevorzugte Heteroarylreste sind Pyridyl, Pyrimidyl, Triazinyl, Oxadiazolyl, Benzofuryl, Dibenzofuryl, Benzothienyl, Dibenzothienyl, Carbazolyl, das über das N-Atom oder über eines seiner C-Atome mit dem Acridin-Grundgerüst verknüpft sein kann, Acridyl, das über das N-Atom oder über eines seiner C-Atome mit dem Acridin-Grundgerüst verknüpft sein kann, Phenothiazin-S,S-dioxidyl, das über das N-Atom oder über eines seiner C-Atome mit dem Acridin-Grundgerüst verknüpft sein kann, sowie ggf. substituierte Imidazole und Benzimidazole, die über N oder C verknüpft sein können. Weitere bevorzugt geeignete Substituenten sind Alkoxy-Substituenten, wobei bevorzugte Alkylreste der Alkoxy-Substituenten bereits vorstehend unter den als Substituenten geeigneten Alkylresten genannt sind. Besonders bevorzugt wird als Alkoxy-Substituent OCH₃ eingesetzt. Weitere bevorzugte Substituenten sind Aryl- und Heteroarylcarbonyloxy, wobei geeignete Arylreste und Heteroarylreste den vorstehend als Substituenten genannten Arylresten und Heteroarylresten entsprechen. Weitere bevorzugt als Substituenten geeignete Reste sind CN-Gruppen sowie Silylgruppen. Die als Substituenten genannten Reste können selbst wiederum einen oder mehrere Substituenten ausgewählt aus der vorstehend genannten Gruppe tragen. Z.B. können die genannten Carbazolylreste mit Silylgruppen substituiert sein.

Wie bereits vorstehend erwähnt können die Alkylreste sowie die Alkylreste der Alkoxygruppen gemäß der vorliegenden Anmeldung sowohl geradkettig als auch verzweigt sein und weisen bevorzugt 1 bis 8 Kohlenstoffatome, besonders bevorzugt 1 bis 4 Kohlenstoffatome auf. Ganz besonders bevorzugt handelt es sich bei den Alkylresten um Methyl, Ethyl, n-Propyl, iso-Propyl, n-Butyl, iso-Butyl, sec-Butyl oder tert-Butyl. Die Alkylreste können unsubstituiert oder substituiert sein, wobei bevorzugte Substituenten ausgewählt sind aus der Gruppe bestehend aus Aryl, Alkoxy und Halogen. Weitere geeignete Substituenten der Alkylreste sind vorstehend genannt. Beispiele für geeignete substituierte Alkylreste sind CF₃, Benzyl und Phenoxymethyl.

Bei den Cycloalkylresten gemäß der vorliegenden Anmeldung handelt es sich bevorzugt um C₃- bis C₁₄-Cycloalkylreste, das heißt Cycloalkylreste, die 3 bis 14 Kohlenstoffatome in ihrem Ringgerüst aufweisen. Besonders bevorzugt sind C₅- bis C₁₀-Cycloalkylreste. Die Cycloalkylreste können mono-, bi- oder polycyclisch sein. Bevorzugt handelt es sich bei den Cycloalkylresten um mono- oder bicyclische Cycloalkylreste, besonders bevorzugt monocyclische Cycloalkylreste. Wie bereits vorstehend erwähnt, können die Cycloalkylreste substituiert oder unsubstituiert sein. Geeignete Substituenten sind bevorzugt ausgewählt aus der Gruppe bestehend aus Aryl, Alkoxy und Halogen. Weitere bevorzugt geeignete Substituenten wurden bereits vorstehend genannt. Beispiele für geeignete Cycloalkylreste sind monocyclische Cycloalkylreste wie Cyclopropyl, Cyclobutyl, Cyclopentyl, Cyclohexyl, Cycloheptyl, Cyclooctyl, Cyclononyl und Cyclodecyl sowie polycyclische Cycloalkylreste wie Decalinyl, Norbornanyl, Bornanyl oder Adamantyl.

Unter Heterocycloalkylresten sind Reste zu verstehen, die sich von den vorstehend genannten Cycloalkylresten dadurch unterscheiden, dass in dem cyclischen Alkyl-Grundgerüst mindestens ein Kohlenstoffatom durch ein Heteroatom ersetzt ist. Bevorzugte Heteroatome sind N, O und S. Das Heterocycloalkyl-Grundgerüst kann - wie das Cycloalkyl-Grundgerüst - an einer, mehreren oder allen substituierbaren Positionen substituiert sein, wobei geeignete Substituenten dieselben sind, die bereits unter der Definition von Cycloalkyl genannt wurden. Die Zahl der Ringatome entspricht ebenfalls der bezüglich der Cycloalkylreste genannten Zahl der Kohlenstoffatome. Bevorzugte Heterocycloalkylreste sind stickstoffhaltige Reste wie Pyrrolidin-2-yl, Pyrrolidin-3-yl, Piperidin-2-yl, Piperidin-3-yl, Piperidin-4-yl.

Geeignete Halogensubstituenten sind Fluor, Chlor, Brom und Jod, bevorzugt Fluor, Chlor und Brom, besonders bevorzugt Fluor und Chlor, wobei Fluor ganz besonders bevorzugt ist.

Geeignete Alkoxygruppen leiten sich von den entsprechenden Alkylresten ab, wie sie vorstehend definiert wurden. Beispiele für geeignete Alkoxygruppen sind OCH₃, OC₂H₅, OC₃H₇ und OC₄H₉. Dabei sind unter C₃H₇ und C₄H₉ sowohl die n-Isomere als auch die verzweigten Isomere wie iso-Propyl, iso-Butyl, sec-Butyl und tert-Butyl zu verstehen.

Als Aryl werden in der vorliegenden Anmeldung Reste bezeichnet, die von mono- oder polycyclischen Aromaten, bevorzugt mono-, bi- oder tricyclischen Aromaten, besonders bevorzugt mono- oder bicyclischen Aromaten abgeleitet sind und keine Ringheteroatome enthalten. Sofern es sich nicht um monocyclische Systeme handelt, ist bei der Bezeichnung Aryl für den zweiten oder weiteren Ring auch die gesättigte Form (Perhydroform) oder die teilweise ungesättigte Form (beispielsweise Dihydroform oder Tetrahydroform), sofern die jeweiligen Formen bekannt und stabil sind, möglich. Das heißt, die Bezeichnung Aryl umfasst in der vorliegenden Anmeldung beispielsweise auch bicyclische Reste, in denen sowohl beide Reste aromatisch sind als auch bicyclische Reste, in denen nur ein Ring aromatisch ist. Beispiele für geeignete Arylreste sind: Phenyl, Naphthyl, Indanyl, 1,2-Dihydronaphthenyl, 1,4-Dihydronaphthenyl, Indenyl oder 1,2,3,4-Tetrahydronaphthyl. Besonders bevorzugt sind Phenyl oder Naphthyl, ganz besonders bevorzugt ist Phenyl.

Die Arylreste können unsubstituiert oder mit einem oder mehreren weiteren Resten substituiert sein. Geeignete weitere Reste sind ausgewählt aus der Gruppe bestehend aus Alkyl, Aryl, Alkoxy, Aryloxy, Arylcarbonyloxy, Heteroaryl, Hydroxy, Amino und Halogen. Bevorzugte Alkyl-, Aryl-, Alkoxy- und Halogenreste sowie geeignete weitere Substituenten sind bereits vorstehend genannt. Die Substitution der Arylreste ist unter anderem abhängig von ihrer Position in dem Acridin-Grundgerüst der Formel (I). Weitere in Abhängigkeit von ihrer Position substituierte Arylreste sind somit nachstehend bezüglich der Definitionen der einzelnen Substituenten an dem Acridin-Grundgerüst der Formel (I) genannt.

Geeignete Aryloxy- und Arylcarbonyloxy-Gruppen leiten sich von den entsprechenden Arylresten ab, wie sie vorstehend definiert wurden. Besonders bevorzugt sind Phenoxy und Phenylcarbonyloxy.

Geeignete Aminogruppen weisen die allgemeine Formel -NR'R" auf, wobei R' und R" Aryl bedeuten. Geeignete Arylreste, die jeweils gegebenenfalls substituiert sein können, sind vorstehend genannt. Beispiele für geeignete Aminogruppen sind Diarylaminogruppen wie Diphenylamino.

Unter Heteroaryl sind mono-, bi- oder polycyclische heteroaromatische Gruppen zu verstehen, bevorzugt mono-, bi- oder tricyclische heteroaromatische Gruppen, die sich zum Teil vom vorstehend genannten Aryl ableiten lassen, in dem im Arylgrundgerüst mindestens ein Kohlenstoffatom durch ein Heteroatom ersetzt ist. Bevorzugte Heteroatome sind N, O und S. Bevorzugte monocyclische Heteroarylreste sind abgeleitet von Pyridin, Pyrimidin und Triazin, wobei die Stickstoffatome in den Pyrimidin- und Triazin-Resten beliebig angeordnet sein können, Pyrrol, Furan, Thiophen, Imidazol und Oxadiazol. Das Grundgerüst kann an einer, mehreren oder allen substituierbaren Positionen substituiert sein, wobei geeignete Substituenten die bezüglich der Definition von Aryl genannten Substituenten sind. Des Weiteren kann das monocyclische Heteroaryl-Grundgerüst mit einer oder zwei Arylgruppen anelliert sein, wobei bi- oder tricyclische Heteroarylreste gebildet werden. Geeignete bi- oder tricyclische Heteroarylreste sind Benzofuryl, Dibenzofuryl, Benzothienyl, Dibenzothienyl, Carbazolyl, Benzimidazolyl, Phenothiazin-S,S-dioxid-yl, Acridyl, wobei die Verknüpfung der Carbazolyl, Phenothiazin-S,S-dioxid-yl sowie der Acridyl-Reste entweder über das N-Atom oder über eines der C-Atome der entsprechenden Reste erfolgen kann. Besonders bevorzugte Heteroarylsubstituenten sind abhängig von der Position der Substituenten an dem Acridyl-Grundgerüst der Formel (I) und sind nachstehend bezüglich der Definitionen der einzelnen Reste des Acridyl-Grundgerüsts der Formel (I) genannt.

Unter Silylresten werden im Sinne der vorliegenden Anmeldung Arylsilylreste, Arylalyklsilylreste und Alkylsilylreste verstanden, wobei geeignete Aryl- und Alkylgruppen vorstehend genannt sind. Bevorzugte Arylgruppen in den Silylresten sind C₆-Arylreste, die substituiert oder unsubstituiert sein können, besonders bevorzugt sind unsubstituierte Phenylreste. Bevorzugte Alkylgruppen sind C₁-C₈-Alkylreste, besonders bevorzugt C₁-C₄-Alkylreste, besonders bevorzugt Methyl- oder Ethylreste und ganz besonders bevorzugt Methylreste. Beispiele für bevorzugte Silylreste sind Triphenylsilyl, Diphenylmethylsilyl, Trimethylsilyl und Phenyldimethylsilyl.

Der Rest R¹ ist ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest oder ein unsubstituierter oder substituierter Heteroarylrest. Geeignete und bevorzugte Alkylreste, Cycloalkylreste, Heterocycloalkylreste, Arylreste, Heteroarylreste sowie geeignete Substituenten sind bereits vorstehend genannt. Bevorzugt handelt es sich bei dem Rest R¹ um einen unsubstituierten oder substituierten C₁- bis C₈-Alkylrest, der linear oder verzweigt sein kann, besonders bevorzugt um einen unsubstituierten C₁- bis C₄-Alkylrest, der linear oder verzweigt sein kann; einen unsubstituierten oder substituierten C₆- bis C₁₄-Arylrest, besonders bevorzugt einen unsubstituierten oder substituierten Phenylrest oder einen unsubstituierten oder substituierten Biphenylrest; einen unsubstituierten oder substituierten Heteroarylrest mit 5 bis 14 Ringatomen, besonders bevorzugt einen unsubstituierten oder substituierten Pyridylrest, einen unsubstituierten oder substituierten Pyrimidylrest, einen unsubstituierten oder substituierten Benzofurylrest, einen unsubstituierten oder substituierten Dibenzofurylrest, einen unsubstituierten oder substituierten Benzothienylrest, einen unsubstituierten oder substituierten Dibenzothienylrest, einen unsubstituierten oder substituierten Carbazolylrest oder einen unsubstituierten oder substituierten Triazinrest. In einer bevorzugten Ausführungsform ist der Rest R¹ ein unsubstituierter oder substituierter Arylrest, wobei bevorzugte Arylreste vorstehend genannt sind und geeignete Beispiele für Arylreste R¹ nachstehend genannt sind. Geeignete Substituenten der vorstehend genannten bevorzugten Reste sind bereits vorstehend genannt. Beispiele für geeignete Reste R¹ sind Methyl, Ethyl, n-Propyl, iso-Propyl, n-Butyl, iso-Butyl, sec-Butyl, tert-Butyl sowie Reste der folgenden allgemeinen Formeln 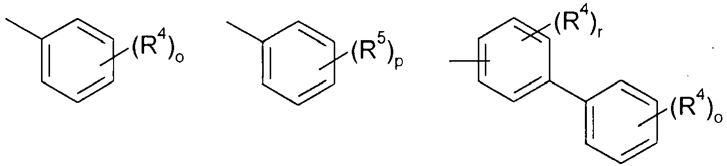 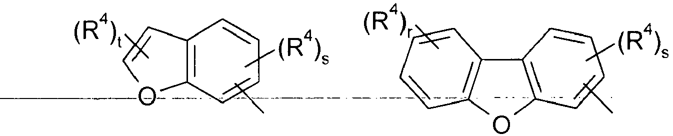 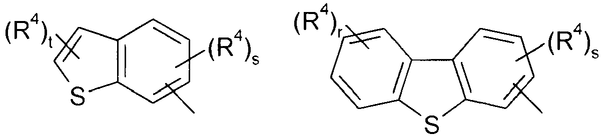 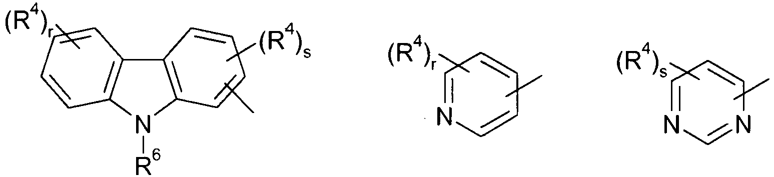 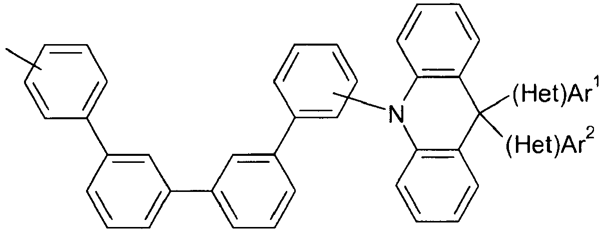 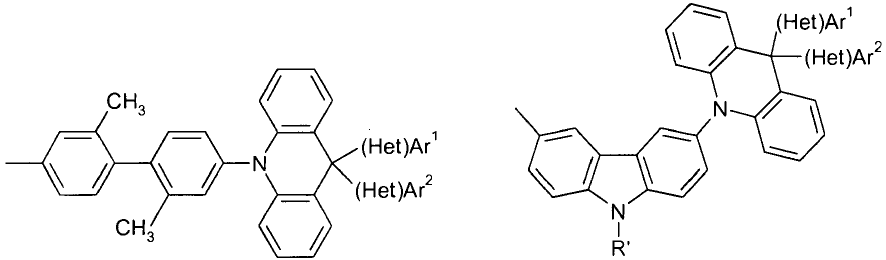 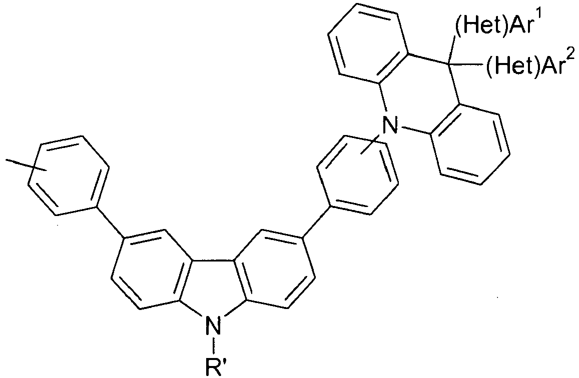 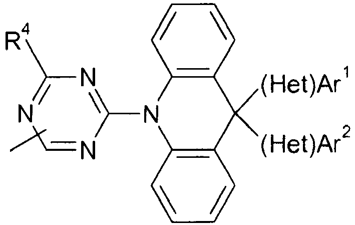 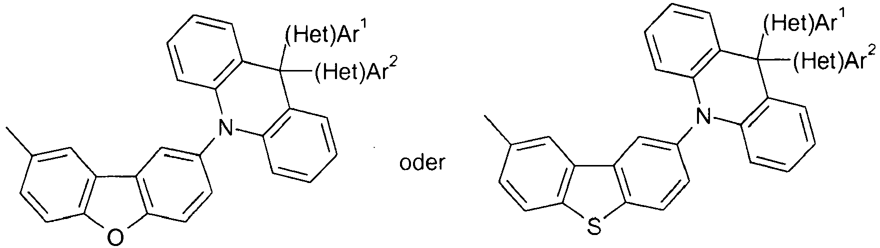 worin bedeuten:
- R⁴: ein Substituent ausgewählt aus der Gruppe bestehend aus Alkyl, Aryl, Heteroaryl, Alkoxy, Aryloxy, Arylcarbonyloxy, Silyl und Halogen, wobei bevorzugte Alkyl, Aryl, Heteroaryl, Alkoxy, Aryloxy, Aryloxycarbonyloxy, Silyl und Halogen-Gruppen vorstehend genannt sind. Ganz besonders bevorzugte Reste R⁴ sind Methyl, Methoxy, CF₃, CN, Silyl (bevorzugt Triphenylsilyl, Diphenylmethylsilyl, Phenyldimethylsilyl).
- R⁵: Alkyl, das substituiert oder unsubstituiert und verzweigt oder unverzweigt sein kann, bevorzugt C₁- bis C₈-Alkyl, besonders bevorzugt C₁- bis C₄-Alkyl, ganz besonders bevorzugt Methyl, Ethyl, n-Propyl, iso-Propyl, n-Butyl, iso-Butyl, sec-Butyl, tert-Butyl, insbesondere bevorzugt Methyl, CF₃: Alkoxy, bevorzugt C₁- bis C₄-Alkoxy, besonders bevorzugt Methoxy; Cyano, Silyl; Arylcarbonyloxy, bevorzugt Phenylcarbonyloxy, Heteroarylcarbonyloxy, wobei bevorzugte Heteroarylgruppen vorstehend genannt sind, oder ein Rest der nachstehend genannten Formeln:

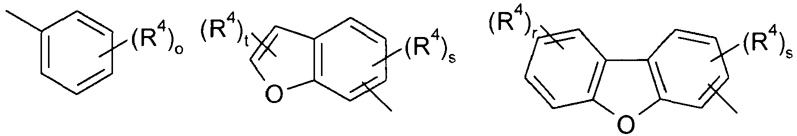

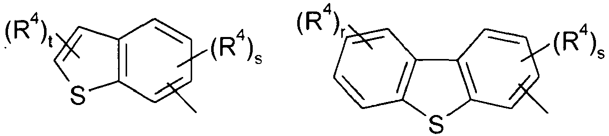

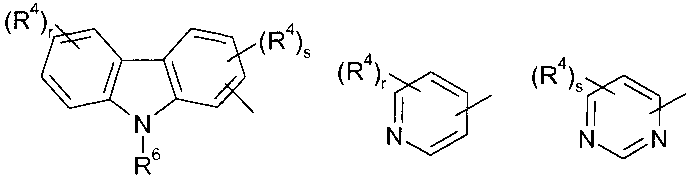

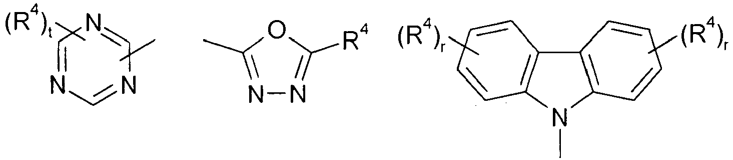

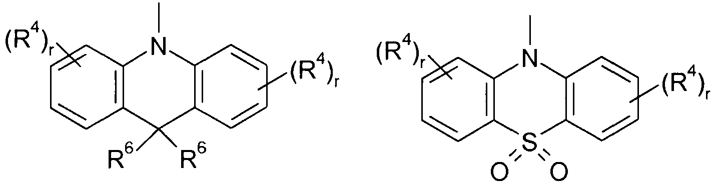

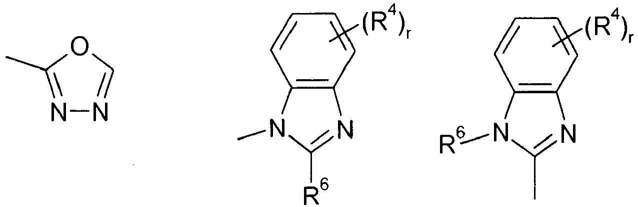

- R⁶: Wasserstoff, Alkyl, Cycloalkyl, Aryl oder Heteroaryl, wobei geeignete Alkyl-, Cycloalkyl-, Aryl- und Heteroarylreste vorstehend genannt sind;
- R': ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest, wobei geeignete Alkyl-, Cycloalkyl-, Aryl- und Heteroarylreste vorstehend genannt sind;
- o, p: 0, 1, 2, 3, 4, 5, bevorzugt 0, 1, 2, 3, besonders bevorzugt 0 oder 1,
- r: 0, 1, 2, 3, 4, bevorzugt 0, 1 oder 2, besonders bevorzugt 0 oder 1;
- s: 0, 1, 2, 3, bevorzugt 0 oder 1;
- t: 0, 1, 2, bevorzugt 2;
wobei in den Fällen, worin o, p, r, s oder t 0 bedeuten, die vorstehend genannten Grundgerüste an ihren substituierbaren Positionen Wasserstoffatome tragen und (Het)Ar¹ und (Het)Ar² die nachstehend genannten Bedeutungen aufweisen.

In einer bevorzugten Ausführungsform bedeuten in dem Rest R¹ sowie in den unten genannten Resten (Het)Ar¹ und (Het)Ar² o, r und s 0, t 2 und p 1.

(Het)Ar¹ und (Het)Ar² bedeuten unabhängig voneinander einen unsubstituierten oder substituierten Arylrest oder einen unsubstituierten oder substituierten Heteroarylrest.

Bevorzugt sind die Reste (Het)Ar¹ und (Het)Ar² unabhängig voneinander ausgewählt aus der Gruppe bestehend aus unsubstituierten oder substituierten C₆-C₁₄-Arylresten, bevorzugt unsubstituiertem oder substituiertem Phenyl oder unsubstituiertem oder substituiertem Biphenyl; und unsubstituierten oder substituierten Heteroarylresten mit 5 bis 14 Ringatomen, bevorzugt unsubstituiertem oder substituiertem Pyridyl, unsubstituiertem oder substituiertem Pyrimidyl, unsubstituiertem oder substituiertem Triazinyl, unsubstituiertem oder substituiertem Benzofuryl, unsubstituiertem oder substituiertem Benzothienyl, unsubstituiertem oder substituiertem Benzimidazolyl, unsubstituiertem oder substituiertem Dibenzofuryl, unsubstituiertem oder substituiertem Dibenzothienyl oder unsubstituiertem oder substituiertem Carbazolyl, wobei der Carbazolylrest über das Stickstoffatom oder eines seiner Kohlenstoffatome mit dem Acridin-Grundgerüst der Formel (I) verknüpft sein kann. Geeignete Substituenten der vorstehend genannten Reste (Het)Ar¹ und (Het)Ar² sind bereits vorstehend genannt.

Besonders bevorzugte Reste (Het)Ar¹ und (Het)Ar² sind unabhängig voneinander ausgewählt aus den nachfolgend dargestellten Resten: 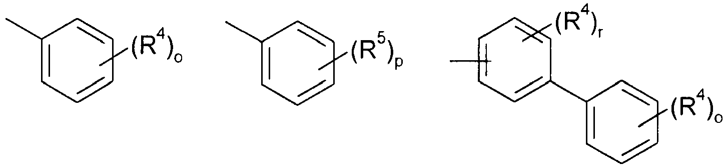 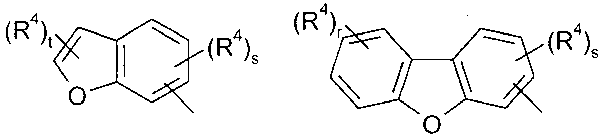 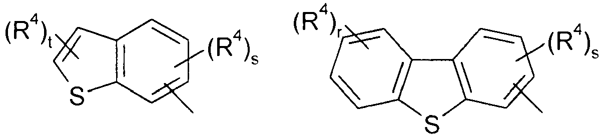 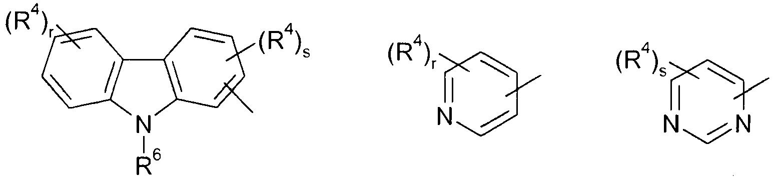 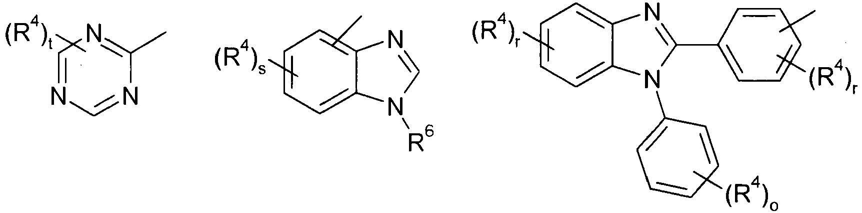 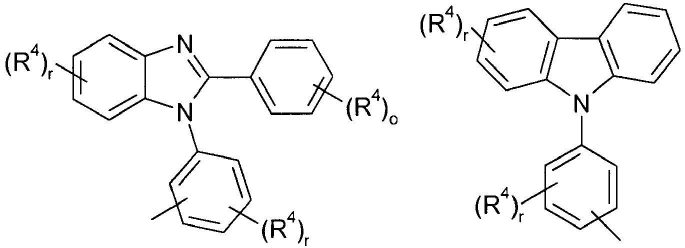 wobei die Reste R⁴, R⁵ und R⁶ sowie die Indices o, p, r, s und t die vorstehend bezüglich der Reste R¹ genannten Bedeutungen aufweisen.

In einer besonders bevorzugten Ausführungsform der vorliegenden Erfindung sind die Gruppen (Het)Ar¹ und (Het)Ar² in den Acridinderivaten der Formel (I) identisch.

R² und R³ bedeuten in den Acridinderivaten der Formel (I) unabhängig voneinander ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest oder ein unsubstituierter oder substituierter Heteroarylrest sowie ein Silylrest. Geeignete Alkylreste, Cycloalkylreste, Heterocycloalkylreste, Arylreste und Heteroarylreste sind vorstehend genannt. Geeignete Substituenten sind ebenfalls bereits vorstehend genannt. Bevorzugt bedeuten R² und R³ unabhängig voneinander einen unsubstituierten oder substituierten Alkylrest, der linear oder verzweigt sein kann, einen unsubstituierten oder substituierten Arylrest oder einen unsubstituierten oder substituierten Heteroarylrest. Besonders bevorzugt sind R² und R³ unabhängig voneinander C₁- bis C₈-Alkyl. das unsubstituiert oder substituiert und linear oder verzweigt sein kann, bevorzugt C₁-bis C₄-Alkyl, das linear oder verzweigt sein kann und unsubstituiert ist, zum Beispiel Methyl, Ethyl, n-Propyl, iso-Propyl, n-Butyl, iso-Butyl, sec-Butyl, tert-Butyl; unsubstituiertes oder substituiertes C₆- bis C₁₄-Aryl, bevorzugt unsubstituiertes oder substituiertes Phenyl, besonders bevorzugt unsubstituiertes Phenyl; unsubstituiertes oder substituiertes Heteroaryl mit 5 bis 14 Ringatomen, bevorzugt unsubstituiertes oder substituiertes Pyridyl, unsubstituiertes oder substituiertes Carbazolyl, besonders bevorzugt unsubstituiertes Pyridyl, unsubstituiertes oder mit C₁- bis C₈-Alkylresten und/oder Silylresten substituiertes Carbazolyl oder Silyl, bevorzugt Triphenylsilyl, Diphenylmethylsilyl, Phenyldimethylsilyl.

n und m sind in den Acridinderivaten der allgemeinen Formel (I) unabhängig voneinander 0, 1, 2, 3 oder 4, wobei in dem Fall, wenn n bzw. m 0 sind, alle mit R² bzw. R³ substituierbaren Positionen des Acridingerüsts Wasserstoff bedeuten. Bevorzugt sind n und m unabhängig voneinander 0, 1 oder 2, besonders bevorzugt 0 oder 1, ganz besonders bevorzugt 0.

Das Acridinderivat der allgemeinen Formel (I) kann eine optionale Brücke 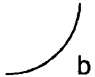 aufweisen, die 0, 1 oder 2, bevorzugt 0 oder 1, ganz besonders bevorzugt 1 Atom(e) aufweist, und die den Rest R¹ kovalent mit einem der substituierbaren Kohlenstoffatome des Acridingerüsts verknüpft.

Dabei bedeutet eine Brücke aus 0 Atomen, dass eine direkte Bindung zwischen den Gruppen (Het)Ar¹ und (Het)Ar² gebildet wird. Bei den die Brücke bildenden Atomen kann es sich um Kohlenstoffatome oder Heteroatome, zum Beispiel O, N oder S, handeln. Wenn die Brücke mehr als ein Atom enthält, kann es sich bei den Atomen entweder um Kohlenstoff- oder um Heteroatome handeln, so dass sowohl Brücken, die aus reinen Kohlenstoffatomketten (Alkylenbrücke) aufgebaut sind als auch Brücken, die neben Kohlenstoffatomen Heteroatome (Heteroalkylenbrücke) aufweisen, umfasst sind. Die Kohlenstoffatome oder optional die Heteroatome, zum Beispiel N, können mit Wasserstoffatomen, Alkylresten, zum Beispiel C₁- bis C₄-Alkylresten, bevorzugt Methyl; oder Arylresten, z.B. C₆- bis C₁₄-Arylresten, bevorzugt Phenyl, substituiert sein. Des Weiteren kann es sich bei der Brücke um eine Alkenylengruppe handeln, wenn die Brücke mindestens zwei Kohlenstoffatome aufweist. Diese Alkenylengruppe kann ebenfalls mit Wasserstoff oder den vorstehend genannten Resten substituiert sein. Bevorzugt ist eine Brücke, die 0 oder 1 Atom aufweist, wobei eine Brücke aus 1 Atom, ggf. ein Heteroatom aufweist. Besonders bevorzugt ist die Brücke aus Kohlenstoffatomen aufgebaut oder eine Bindung, ganz besonders bevorzugt ist eine aus einem oder mehreren Kohlenstoffatom(en) aufgebaute Brücke

Die Acridinderivate der allgemeinen Formel (I) weisen keine Brücke 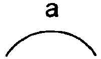 auf, das heißt, die Acridinderivate der Formel (I) sind keine Spiroverbindungen. Es wurde gefunden, dass Acridinderivate der Formel (I), die keine Spiroverbindungen darstellen, eine gute Beständigkeit des amorphen Zustandes bei Betrieb einer OLED aufweisen.

Die Beständigkeit des amorphen Zustandes stellt ein wichtiges Kriterium für den Einsatz in OLEDs geeignete Matrixmaterialien dar. Die thermische Beanspruchung während des Betriebs einer OLED kann zu einem Übergang des metastabilen amorphen Zustandes zum thermodynamisch stabilen Kristall führen. Dies hat weitreichende Konsequenzen für die Lebensdauer des Bauteils. Die Korngrenzen einzelner Kristallite stellen Fehlstellen dar, an denen der Transport von Ladungsträgern zum Erliegen kommt. Ebenso führt die mit der Kristallisation einhergehende Umstrukturierung der Schichten zu einem verminderten Kontakt der Schichten untereinander und mit den Elektroden. Dies führt beim Betrieb mit der Zeit letztlich zur Zerstörung der OLED. Es ist bekannt, dass die Stabilität des amorphen Zustandes in Spiroverbindungen besonders hoch ist. Es wurde gefunden, dass Acridinderivate der Formel (I), die keine

Spiroverbindungen darstellen, das heißt, worin 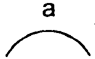 nicht vorliegt, eine gute Stabilität des amorphen Zustandes aufweisen und somit für den Einsatz als Matrixmaterialien und/oder Elektronenblockermaterialien in OLEDs gut geeignet sind. In einer weiteren bevorzugten Ausführungsform der vorliegenden Erfindung weisen die Acridinderivate der Formel (I) weder eine Brücke 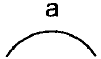 noch eine Brücke 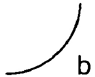 auf.

In einer weiteren bevorzugten Ausführungsform betrifft die vorliegende Erfindung Acridinderivate der allgemeinen Formel (I), worin die Reste und Symbole unabhängig voneinander die folgenden Bedeutungen aufweisen:
- (Het)Ar¹, (Het)Ar²: unabhängig voneinander ausgewählt aus der Gruppe bestehend aus unsubstituierten oder substituierten C₆-C₁₄-Arylresten und unsubstituierten oder substituierten Heteroarylresten mit 5 bis 14 Ringatomen, bevorzugt ausgewählt aus der Gruppe bestehend aus unsubstituiertem oder substituiertem Phenyl, unsubstituiertem oder substituiertem Biphenyl, unsubstituiertem oder substituiertem Pyridyl, unsubstituiertem oder substituiertem Pyrimidyl, unsubstituiertem oder substituiertem Triazinyl, unsubstituiertem oder substituiertem Benzofuryl, unsubstituiertem oder substituiertem Benzimidazolyl, unsubstituiertem oder substituiertem Benzothienyl, unsubstituiertem oder substituiertem Dibenzofuryl, unsubstituiertem oder substituiertem Dibenzothienyl und unsubstituiertem oder substituiertem Carbazolyl, besonders bevorzugt Phenyl, das unsubstituiert ist, oder mit einem oder mehreren Resten ausgewählt aus CN, Phenylcarbonyloxy, Carbazolyl, das über das N-Atom oder über ein C-Atom mit dem Acridin-Grundgerüst verknüpft sein kann, Pyridyl, CF₃, 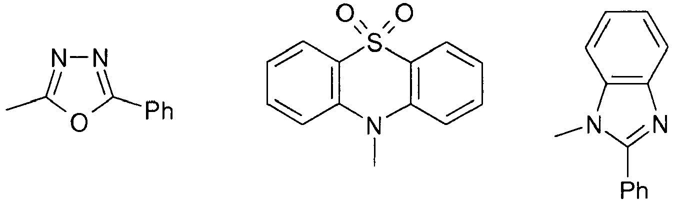 oder 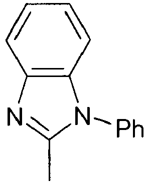 substituiert ist; Pyridyl, Pyrimidyl, Triazinyl, Oxazolyl, Oxadiazolyl, das jeweils unsubstituiert oder mit Phenyl substituiert ist; Benzimidazolyl, das unsubstituiert oder mit Phenyl substituiert ist; Benzofuryl, Dibenzofuryl. Benzothienyl, Dibenzothienyl und Carbazolyl, das jeweils unsubstituiert oder mit Phenyl substituiert ist;
- R¹: ein unsubstituierter oder substituierter C₁-C₈-Alkylrest, der linear oder verzweigt sein kann, bevorzugt ein unsubstituierter C₁- bis C₄-Alkylrest, der linear oder verzweigt sein kann; ein unsubstituierter oder substituierter C₆-bis C₁₄-Arylrest, bevorzugt ein unsubstituierter oder substituierter Phenylrest oder ein unsubstituierter oder substituierter Biphenylrest; ein unsubstituierter oder substituierter Heteroarylrest mit 5 bis 14 Ringatomen, bevorzugt ein unsubstituierter oder substituierter Pyridylrest, ein unsubstituierter oder substituierter Pyrimidylrest, ein unsubstituierter oder substituierter Benzofurylrest, ein unsubstituierter oder substituierter Benzothienylrest, ein unsubstituierter oder substituierter Dibenzofurylrest, ein unsubstituierter oder substituierter Dibenzothienylrest oder ein unsubstituierter oder substituierter Carbazolylrest, der über das N-Atom oder über ein C-Atom mit dem Acridin-Grundgerüst der Formel (I) verknüpft sein kann; besonders bevorzugt bedeutet R¹ Phenyl, das unsubstituiert ist oder mit einem mehreren Resten ausgewählt aus CH₃, CN, OCH₃, Carbazolyl, das über das N-Atom oder über ein C-Atom mit dem Acridin-Grundgerüst der Formel (I) verknüpft sein kann, 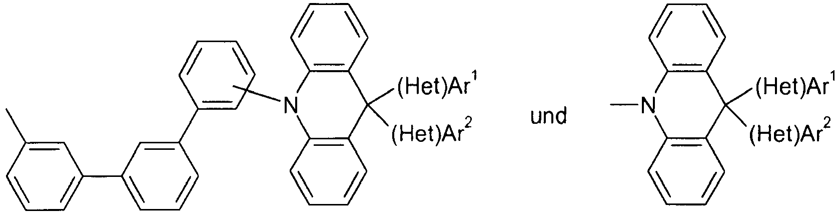 substituiert sein kann; Biphenyl, Benzofuryl oder ein Rest der Formeln 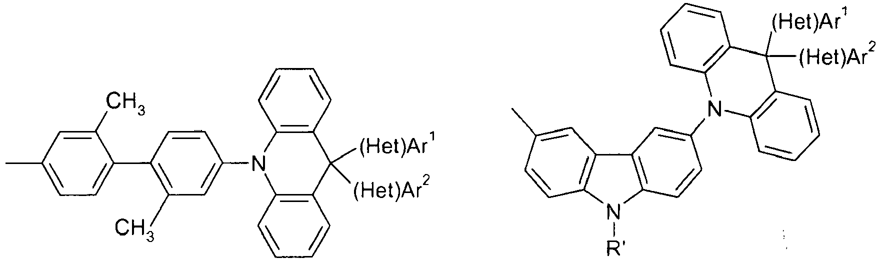 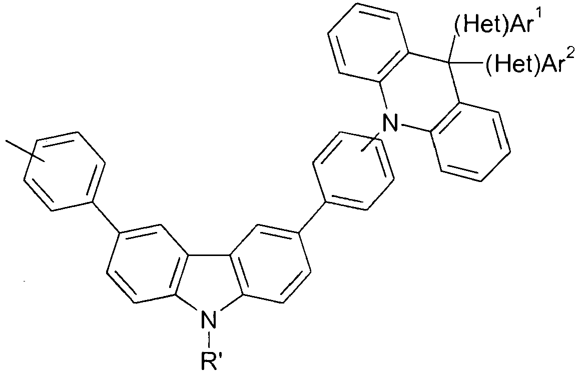 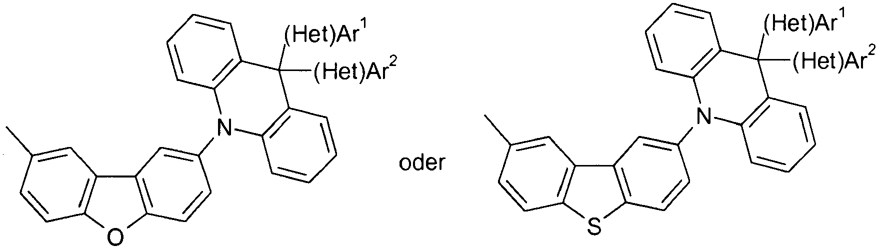 wobei (Het)Ar¹ und (Het)Ar² vorstehend definiert sind, und
R' ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest ist;
ganz besonders bevorzugt ist R' Phenyl, das unsubstituiert ist oder mit einem oder mehreren der vorstehend genannten Reste substituiert ist,
R², R³ unabhängig voneinander ein unsubstituierter oder substituierter C₁-C₈-Alkylrest, der linear oder verzweigt sein kann, bevorzugt ein unsubstituierter C₁-C₄-Alkylrest; ein unsubstituierter oder substituierter C₆- bis C₁₄-Arylrest, bevorzugt ein unsubstituierter oder substituierter Phenylrest oder ein unsubstituierter oder substituierter Biphenylrest; ein unsubstituierter oder substituierter Heteroarylrest mit 5 bis 14 Ringatomen, bevorzugt ein unsubstituierter oder substituierter Pyridylrest, bevorzugt ein unsubstituierter oder substituierter Carbazolylrest oder ein Silylrest, bevorzugt Triphenylsilyl, Diphenylmethylsilyl, Phenyldimethylsilyl; in einer besonders bevorzugten Ausführungsform weisen die Acridinderivate der Formel (I) jeweils keinen oder jeweils einen Rest R² bzw. R³ auf (n, m = 0,1), wobei, wenn n und m = 1 bedeuten, die Reste R² und R³ jeweils in para-Stellung zu dem N-Atom der Acridinderivate der Formel (I) angeordnet sind;
n, m 0, 1, 2 oder 3, bevorzugt 0 oder 1, besonders bevorzugt 0; 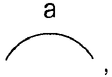 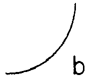
nicht vorhanden.

Geeignete Substituenten sowie weitere bevorzugte geeignete Alkylreste, Arylreste und Heteroarylreste sind vorstehend genannt.

Besonders bevorzugte Acridinderivate der Formel (I) weisen die folgenden Formeln auf:

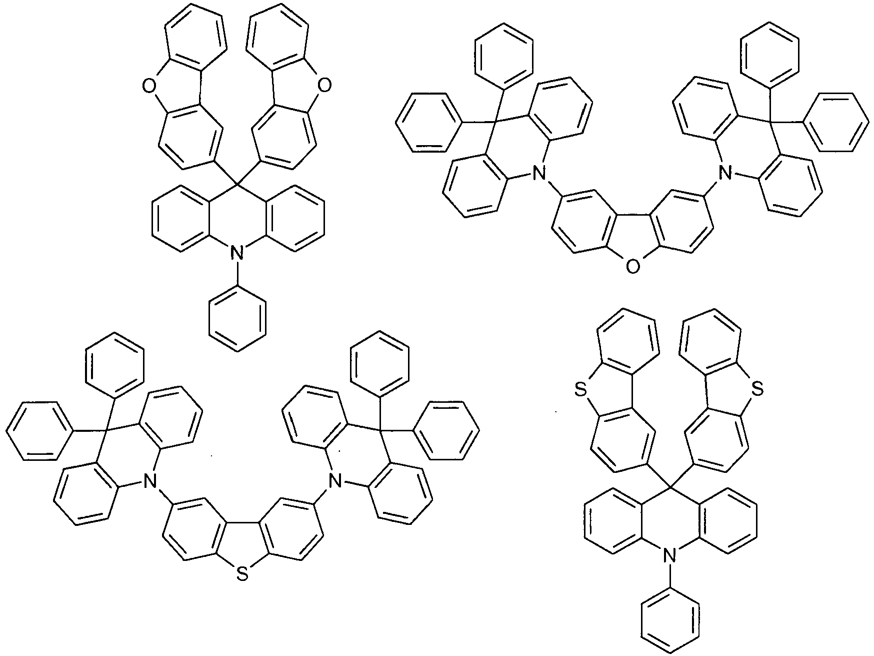

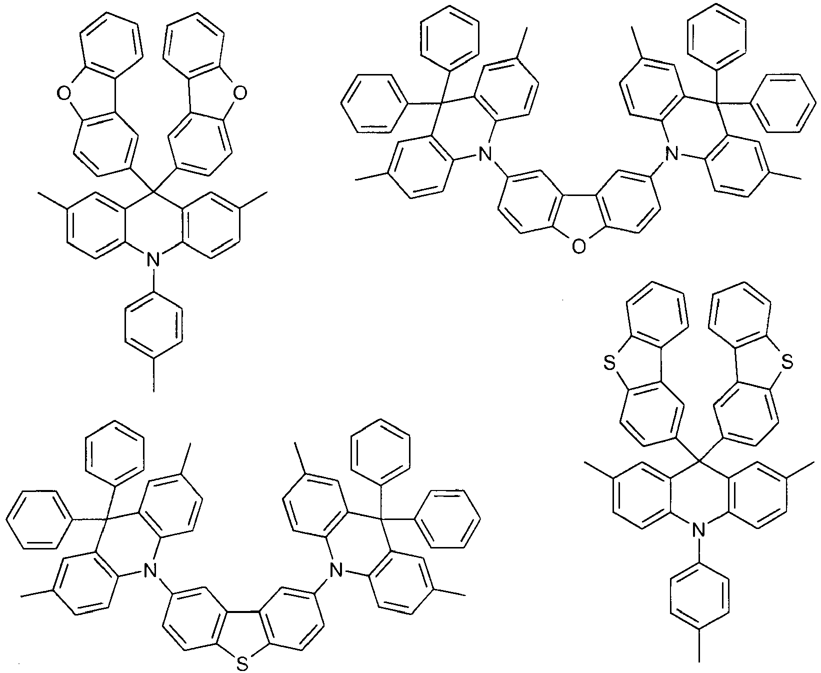

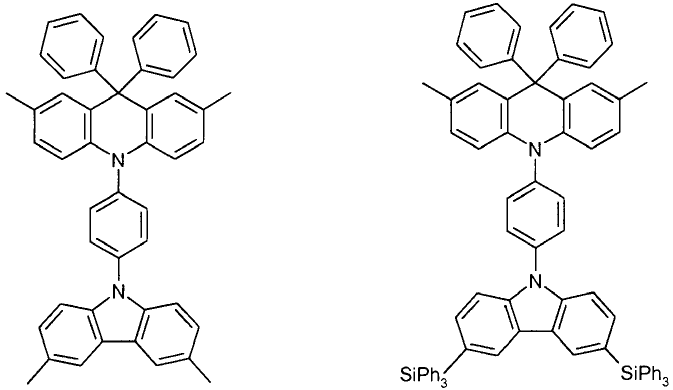

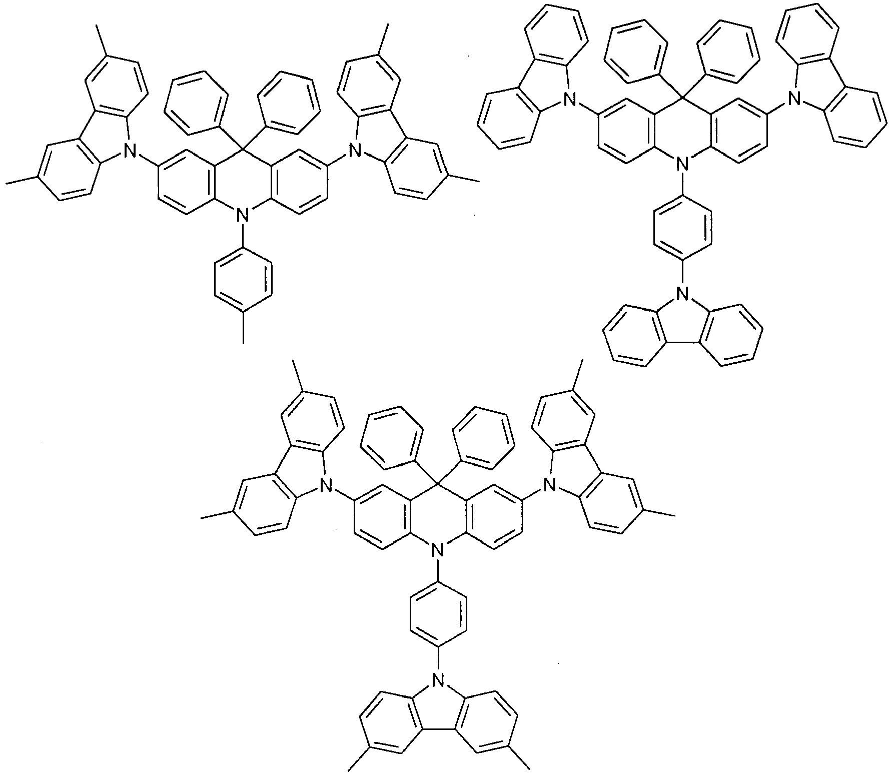

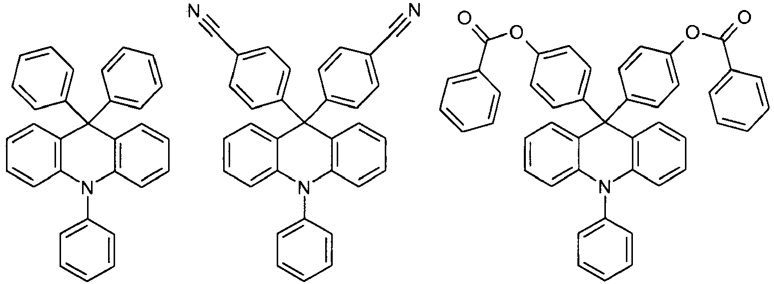

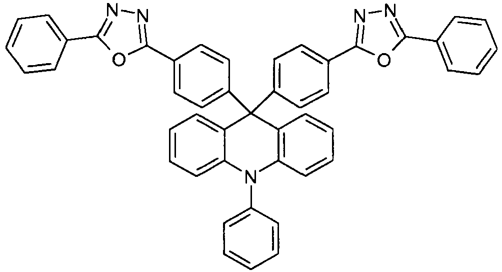

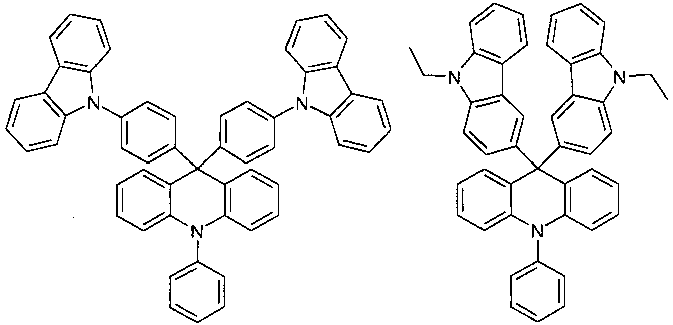

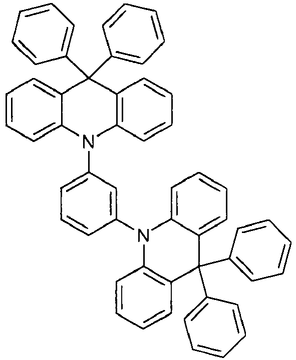

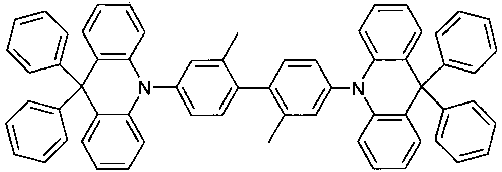

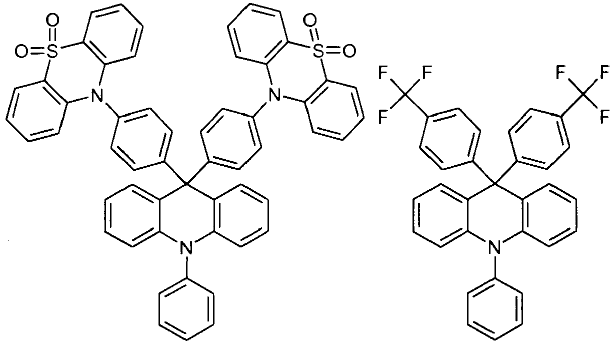

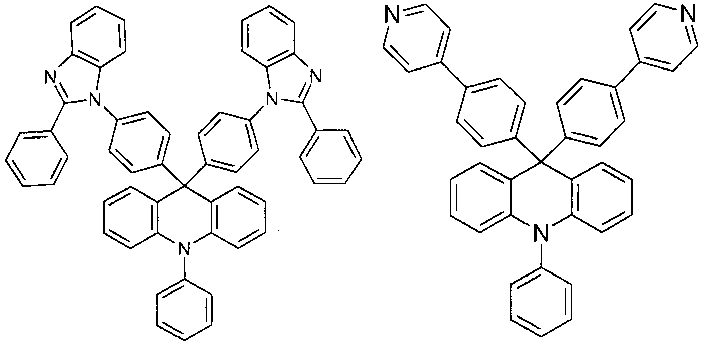

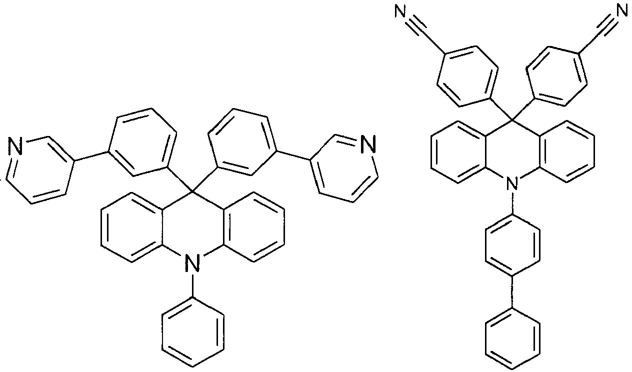

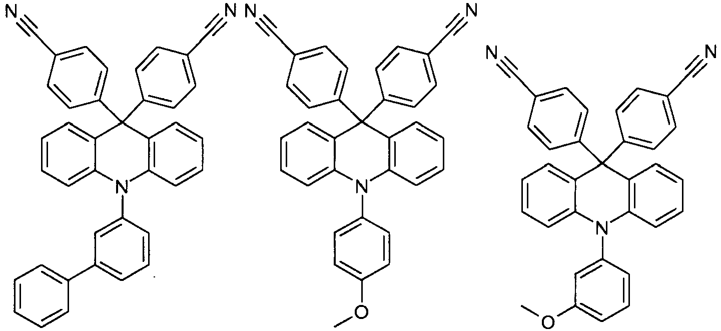

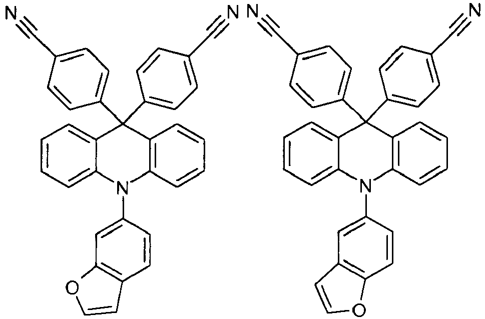

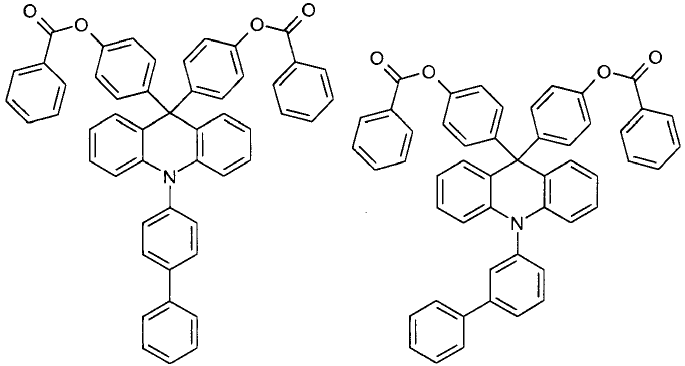

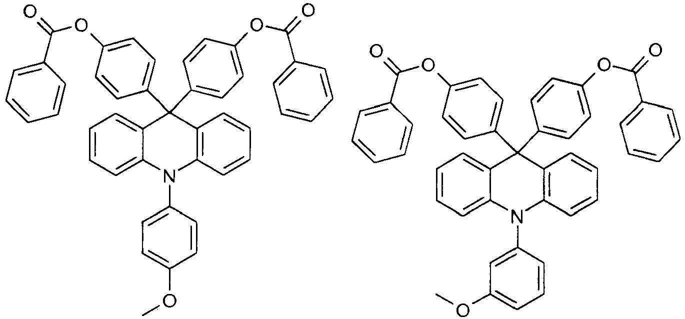

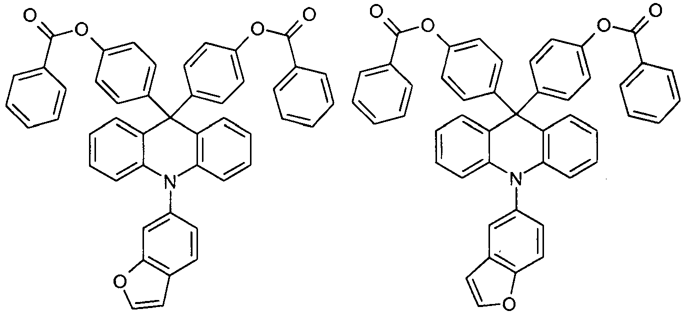

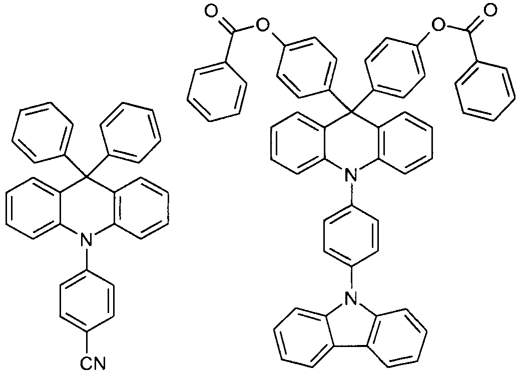

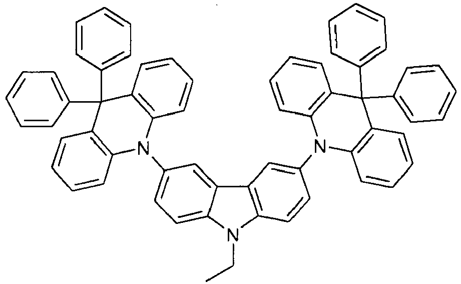

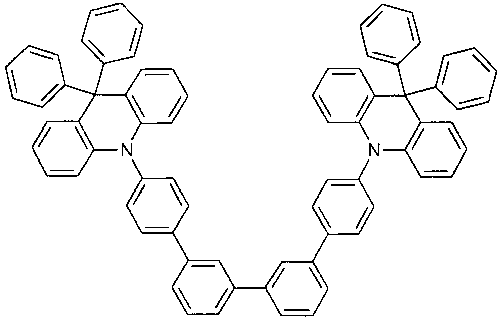

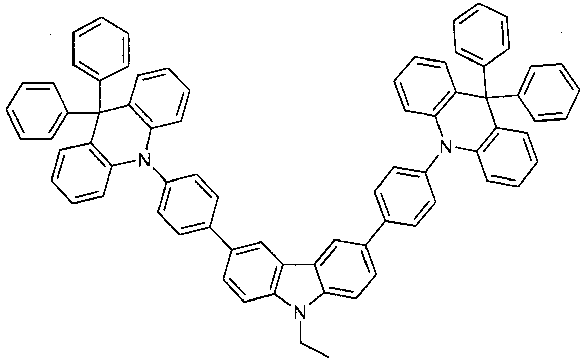

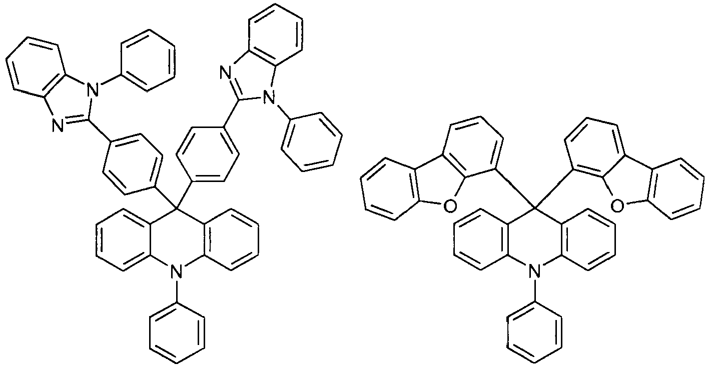

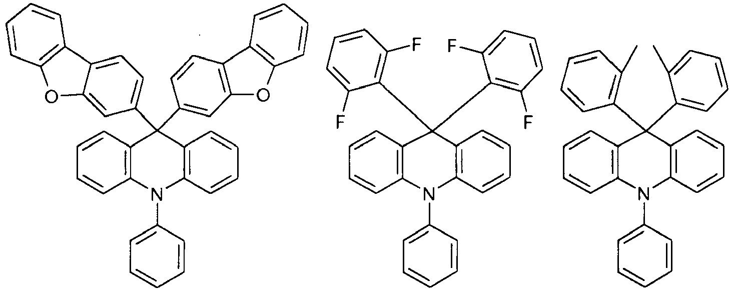

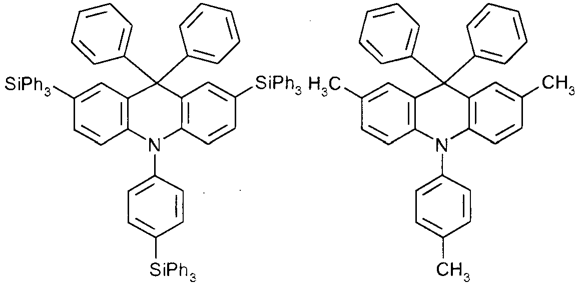

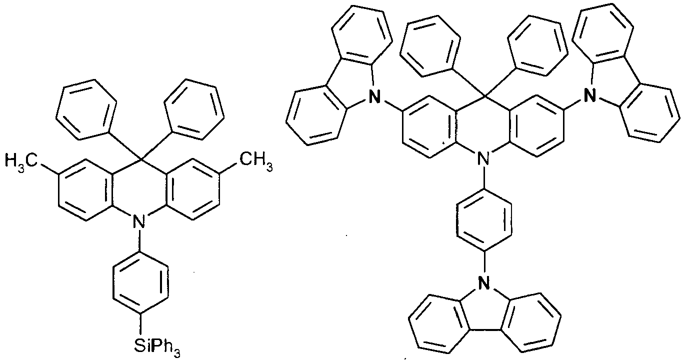

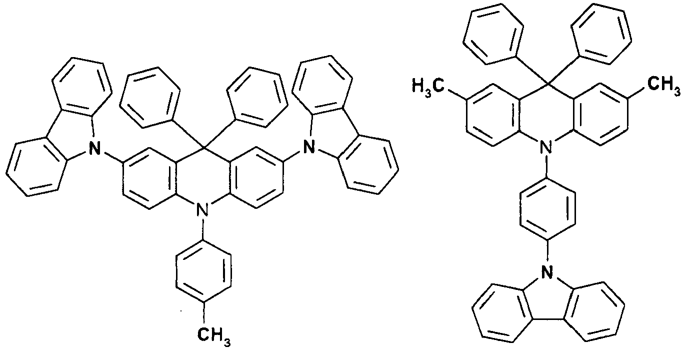

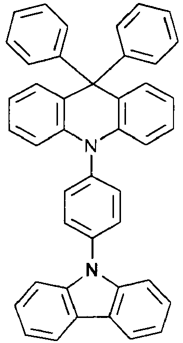

Die Herstellung der Acridinderivate erfolgt nach dem Fachmann bekannten Verfahren, bzw. Verfahren, die von dem Fachmann bekannten Verfahren abgeleitet sind. Geeignete Herstellungsverfahren sind zum Beispiel in der in der Beschreibungseinleitung genannten Literatur angegeben.

Beispielsweise können die Acridinderivate der Formel (I) gemäß dem folgenden Schema 1 hergestellt werden:

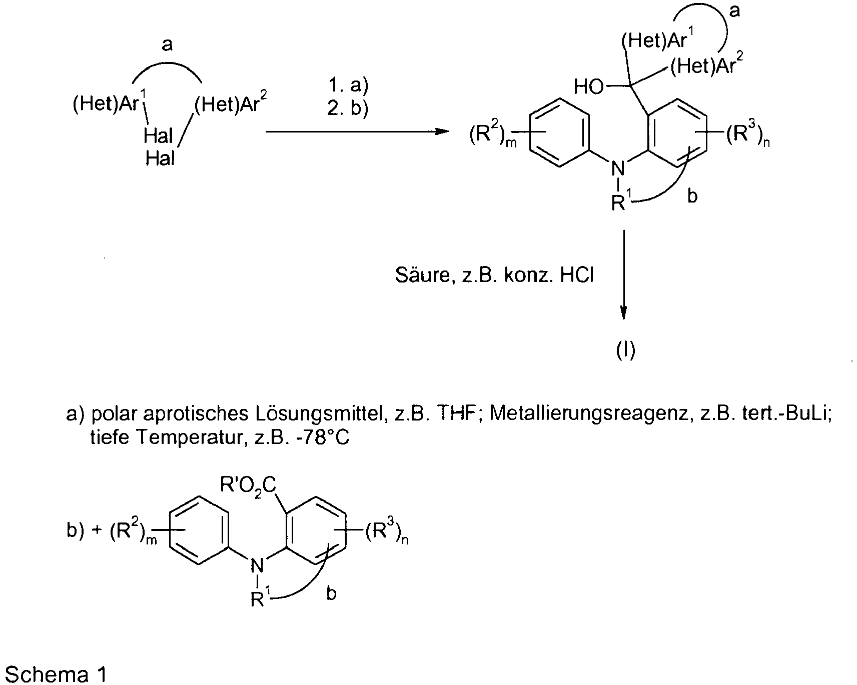

Die Reste und Symbole in Schema 1 haben die vorstehend bezüglich der Verbindungen der Formel I genannten Bedeutungen und Hal bedeutet Halogen, z.B. Fluor, Chlor, Brom, Iod, bevorzugt Chlor oder Brom, besonders bevorzugt Brom.

Die Verfahrensschritte und Reaktionsbedingungen sowie geeignete polar aprotische Lösungsmittel zur Durchführung des in Schema 1 dargestellten Herstellungsverfahrens sind dem Fachmann bekannt.

In dem folgenden Schema 2 ist beispielhaft ein Verfahren zur Herstellung von 9,9-Di(4-(N-carbazolyl)-phenyl-N-phenyl-9,10-dihydroacridin dargestellt:

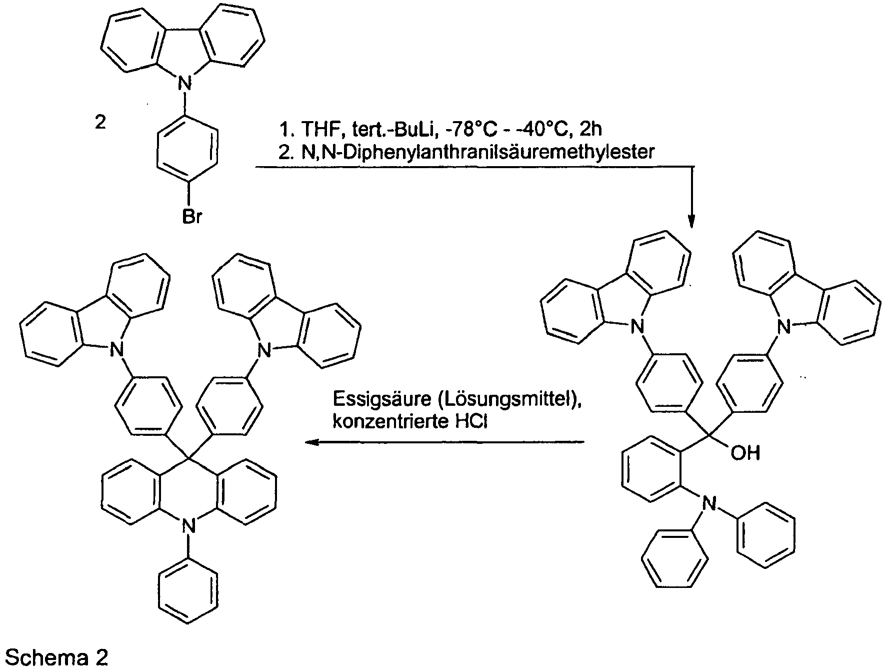

Die Acridinderivate der Formel (I) sind hervorragend für den Einsatz als Matrixmaterialien in der Licht-emittierenden Schicht in organischen Leuchtdioden sowie als Elektronenblocker in OLEDs geeignet, worin als Emitter-Verbindungen ein oder mehrere Triplett-Emitter eingesetzt werden, sowie als Elektronenblocker in einer der Licht-emittierenden Schicht eines OLEDs direkt benachbarten Blockschicht für Elektronen, wobei die Licht-emittierende Schicht einen oder mehrere Triplett-Emitter enthält.

Ein weiterer Gegenstand der vorliegenden Erfindung ist daher eine OLED enthaltend mindestens ein Acridinderivat der Formel (I) in der Licht-emittierenden Schicht neben mindestens einer Emitter-Verbindung, wobei es sich um mindestens einen Triplett-Emitter handelt.

Ein weiterer Gegenstand der vorliegenden Erfindung ist des Weiteren eine OLED enthaltend mindestens ein Acridinderivat der Formel (I) in einer Blockschicht für Elektronen, wobei die Blockschicht für Elektronen bevorzugt in direktem Kontakt mit der Licht-emittierenden Schicht der OLED steht und die Licht-emittierende Schicht der OLED mindestens einen Triplett-Emitter enthält.

Es ist des Weiteren möglich, dass die Acridinderivate der Formel (I) sowohl als Matrixmaterial als auch als Elektronenblocker in einer OLED eingesetzt werden. Dabei handelt es sich bei den in einer OLED als Matrixmaterial sowie als Elektronenblocker eingesetzten Acridinderivaten der Formel (I) bevorzugt um unterschiedliche Acridinderivate der Formel (I).

Ein weiterer Gegenstand der vorliegenden Erfindung ist eine Licht-emittierende Schicht enthaltend mindestens eine Emitter-Verbindung und mindestens ein Matrixmaterial, wobei als Matrixmaterial mindestens ein Acridinderivat der Formel (I) eingesetzt wird. Bevorzugte Acridinderivate der Formel (I) sind bereits vorstehend genannt. Bei der mindestens einen Emitterverbindung handelt es sich um einen Triplett-Emitter. Geeignete Triplett-Emitter sind dem Fachmann bekannt. Bevorzugte Triplett Emitter sind nachstehend genannt.

Ein weiterer Gegenstand der vorliegenden Anmeldung ist eine Blockschicht für Elektronen enthaltend mindestens ein Acridinderivat der Formel (I), wobei bevorzugte Acridinderivate der Formel (I) bereits vorstehend genannt sind.

Die Eignung der Acridinderivate der Formel (I) als Elektronenblocker- oder Matrixmaterial ist u. a. abhängig von den elektronischen Verhältnissen in der OLED und dem Substitutionsmuster der Acridinderivate der Formel (I). Im Allgemeinen hat das Matrixmaterial eine stärkere Elektronenaffinität als das Elektronenblockermaterial. Die Elektronenaffinittät kann über das Reduktionspotential z. B. in einer cyclovoltametrischen Messung bestimmt werden, wobei geeignete Messanordnungen dem Fachmann bekannt sind.

Ein weiterer Gegenstand der vorliegenden Erfindung ist eine OLED enthaltend mindestens eine erfindungsgemäße Licht-emittierende Schicht.

Die Verwendung der Acridinderivate der Formel (I) als Matrixmaterialien und/oder Elektronenblocker soll hierbei nicht ausschließen, dass diese Verbindungen selbst auch Licht emittieren. Die erfindungsgemäß verwendeten Matrixmaterialien bzw. Elektronenblocker bewirken aber, dass im Normalfall bei Verbindungen, welche als Emitter in OLEDs eingesetzt werden, eine Zunahme der Leuchtdichte und Quantenausbeute gegenüber sonst üblichen Matrixmaterialien bzw. Elektronenblockern erreicht wird.

Viele der bevorzugt eingesetzten Emitter-Verbindungen basieren auf Metallkomplexen, wobei z.B. die Komplexe der Metalle Ru, Rh, Ir, Pd, Pt Re, Os, Cu und Au, insbesondere die Komplexe der Metalle Ru, Rh, Ir, Pd und Pt, vor allem die Komplexe des Ir Bedeutung erlangt haben. Die erfindungsgemäß verwendeten Acridinderivate der Formel I sind besonders als Matrixmaterialien und/oder Elektronenblockermaterialien für Emitter auf Basis solcher Metallkomplexe geeignet. Insbesondere sind sie für die Verwendung als Matrixmaterialien und/oder Elektronenblockermaterialien zusammen mit Komplexen des Ru, Rh, Ir, Pd und Pt, besonders bevorzugt für die Verwendung zusammen mit Komplexen des Ir geeignet.

Geeignete Metallkomplexe zur Verwendung zusammen mit den Acridinderivaten der Formel I als Matrixmaterialien und/oder Elektronenblockermaterialien in OLEDs sind z.B. in den Schriften WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006/103874 A1, WO 2005/123873 A1 und WO 2006/121811 A1 beschrieben.

Des Weiteren sind die blaues Licht emittierenden Iridiumkomplexe Iridium(III)bis[(4,6-di-fluorophenyl)pyridinato-N,C^{2'}]picolinat (Flrpic), das z.B. in S. Lamansky et al., J. Am. Soc. 2001, 123, 4304 und C. Adachi et al., Appl. Phys. Lett. 2001, 79, 2082 offenbart ist, und Iridium(III)bis(4',6'-difluorophenylpyridinato)tetrakis(1-pyrazolyl)borat (FIr6), das z.B. in R. J. Holmes et al., Appl. Phys. Lett. 2003, 83, 3818 offenbart ist, geeignet.

Weitere geeignete Metallkomplexe sind die kommerziell erhältlichen Metallkomplexe Tris(2-phenylpyridin)iridium(III), Iridium(III)tris(2-(4-tolyl)pyridinato-N,C²'), Iridium(III)tris(1-phenylisochinolin), Iridium(III)bis(2-2'-benzo-thienyl)pyridinato-N,C³')(acetylacetonat), Iridium(III)bis(2-(4,6-difluorophenyl)pyridinato-N,C²)piolinat, Iridium(III)bis(1-phenylisochinolin)(acetylacetaonat), Iridium(III)bis(di-benzo[f,h]chinoxalin)(acetylacetaonat) und Iridium(III)bis(2-methyldi-benzo[f,h]chinoxalin)(acetylacetonat).

Des Weiteren sind die folgenden kommerziell erhältlichen Materialien geeignet: Tris(dibenzoylacetonato)-mono(phenanthrolin)-europium(III), Tris(dibenzoylmethan)-mono(phenanthrolin)europium(III), Tris(dibenzoylmethan)-mono(5-aminophenan-throlin)europium(III), Tris(di-2-naphthoylmethan) mono(phenanthrolin) europium(III), Tris(4-bromobenzoylmethan)-mono(phenanthrolin)-europium(III), Tris(di-biphenyl-methan)-mono(phenanthrolin)-europium(III), Tris(dibenzoylmethan)-mono(4,7-diphenyl-phenanthrolin)-europium(III), Tris(dibenzoylmethan)-mono(4,7-di-methylphenanthrolin)-europium(III), Tris(dibenzoylmethan)-mono(4,7-dimethyl-phenanthrolin-disulfonsäure)-europium(III)-dinatriumsalz, Tris[di(4-(2-(2-ethoxy-ethoxy)ethoxy)benzoylmethan)]-mono(phenanthrolin)-europium(III) und Tris[di[4-(2-(2-ethoxyethoxy)ethoxy)benzoyl-methan)]mono-(5-aminophenanthrolin)-europium(III).

Des Weiteren sind die folgenden Komplexe als Emittermaterialien zum gemeinsamen Einsatz mit den Acridinderivaten der Formel (I) in OLEDs geeignet:

Homoleptische Komplexe der allgemeinen Formel (II): 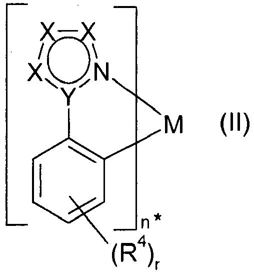 worin bedeuten:
- X: N, NH, NR⁴, CH, CR⁴, O oder S;
- Y: C oder N;
- R⁴: jeweils unabhängig ein Substituent ausgewählt aus der Gruppe bestehend aus Alkyl, Aryl, Heteroaryl, Alkoxy, Aryloxy, Arylcarbonyloxy, Silyl und Halogen, wobei bevorzugte Alkyl, Aryl, Heteroaryl, Alkoxy, Aryloxy, Aryloxycarbonyloxy, Silyl und Halogen-Gruppen vorstehend genannt sind. Ganz besonders bevorzugte Reste R⁴ sind substituiertes oder unsubstituiertes Phenyl, wobei substituiertes Phenyl bevorzugt mit Alkylsubstituenten wie C₁-C₈-, bevorzugt C₁- bis C₄-Alkylresten, die linear oder verzweigt sein können, besonders bevorzugt Methyl, Ethyl, n-Propyl, iso-Propyl, n-Butyl, iso-Butyl, sec-Butyl oder tert-Butyl substituiertes Phenyl bedeutet; Methyl; Methoxy; CF₃; CN; Silyl (bevorzugt Triphenylsilyl, Diphenylmethylsilyl, Phenyldimethylsilyl);
- M: Ir, Rh, Pt, Pd oder Ru, bevorzugt Ir;
- n*: in Abhängigkeit von der Wertigkeit von M 2, 3 oder 4, bevorzugt - wenn M Ir bedeutet - 3;
- r: 0, 1, 2, 3 oder 4, bevorzugt 0, 1 oder 2, besonders bevorzugt 0 oder 1, wobei in den Fällen, wenn r 0 bedeutet, alle substituierbaren Positionen des Arylrests Wasserstoffatome tragen.

Bevorzugte homoleptische Komplexe der Formel (II) weisen die folgenden Strukturen auf: 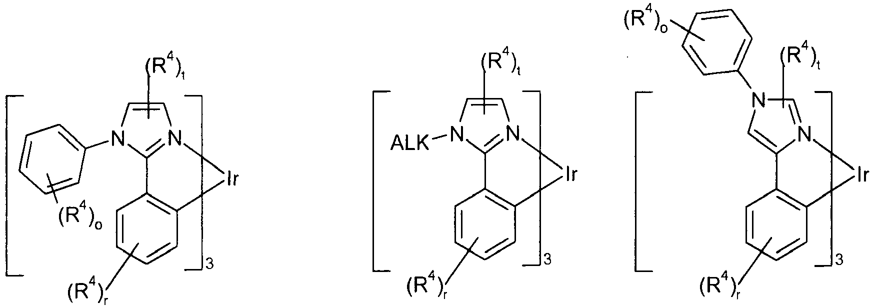 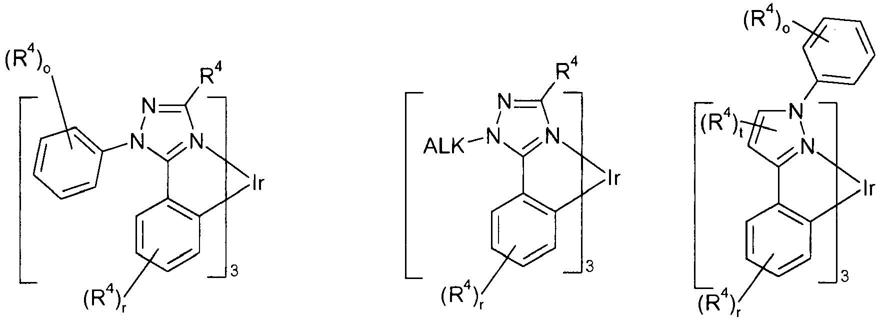 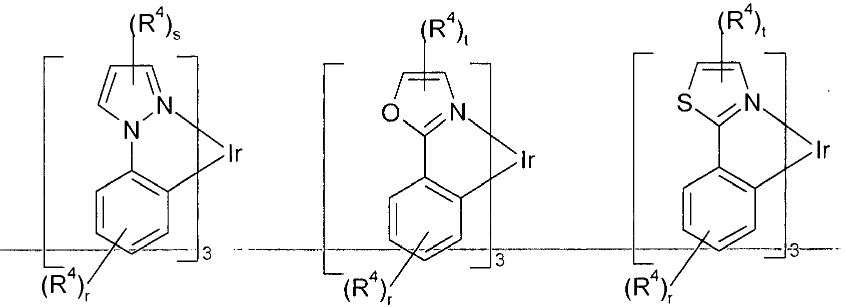 worin bedeuten:
- R⁴: jeweils unabhängig ein Substituent ausgewählt aus der Gruppe bestehend aus Alkyl, Aryl, Heteroaryl, Alkoxy, Aryloxy, Arylcarbonyloxy, Silyl und Halogen, wobei bevorzugte Alkyl, Aryl, Heteroaryl, Alkoxy, Aryloxy, Aryloxycarbonyloxy, Silyl und Halogen-Gruppen vorstehend genannt sind. Ganz besonders bevorzugte Reste R⁴ sind substituiertes oder unsubstituiertes Phenyl, wobei substituiertes Phenyl bevorzugt mit Alkylsubstituenten wie C₁-C₈-, bevorzugt C₁- bis C₄-Alkylresten, die linear oder verzweigt sein können, besonders bevorzugt Methyl, Ethyl, n-Propyl, iso-Propyl, n-Butyl, iso-Butyl, sec-Butyl oder tert-Butyl substituiertes Phenyl bedeutet; Methyl; Methoxy; CF₃; CN; Silyl (bevorzugt Triphenylsilyl, Diphenylmethylsilyl, Phenyldimethylsilyl);
- ALK: ein Alkylrest, bevorzugt ein C₁- bis C₄-Alkylrest, der linear oder verzweigt sein kann, besonders bevorzugt Methyl, Ethyl, n-Propyl, iso-Propyl, n-Butyl, iso-Butyl, sec-Butyl oder tert-Butyl;
- o: 0, 1, 2, 3, 4, 5, bevorzugt 0, 1, 2, 3, besonders bevorzugt 0 oder 1,
- r: 0, 1, 2, 3, 4, bevorzugt 0, 1 oder 2, besonders bevorzugt 0 oder 1;
- s: 0, 1, 2, 3, bevorzugt 0 oder 1;
- t: 0, 1, 2, bevorzugt 0 oder 1;
wobei in den Fällen, worin o, p, r, s oder t 0 bedeuten, die vorstehend genannten Grundgerüste an ihren substituierbaren Positionen Wasserstoffatome tragen.

Heteroleptische Komplexe der allgemeinen Formel (III): 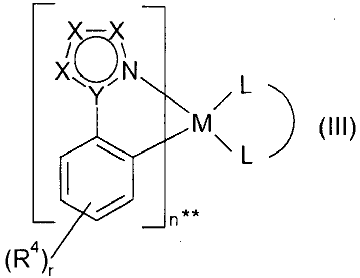 worin die Symbole und Reste X, Y, M, R⁴ und r jeweils unabhängig die bezüglich der allgemeinen Formel (II) genannten Bedeutungen aufweisen, und
- n**: in Abhängigkeit von der Wertigkeit von M 1, 2 oder 3, bevorzugt - wenn M Ir bedeutet - 2; 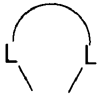
ein monoanionischer bidentater Ligand, zum Beispiel Acetylacetonat und dessen Derivate, Picolinat und dessen Derivate, Schiffsche Basen, Aminosäuren sowie Liganden der folgenden allgemeinen Formeln: 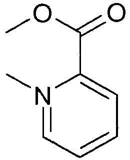 oder 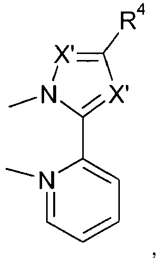 worin X' unabhängig voneinander N oder CH bedeutet und R⁴ die vorstehend genannten Bedeutungen aufweist.

Bevorzugte heteroleptische Komplexe der Formel (III) weisen die folgenden Strukturen auf: 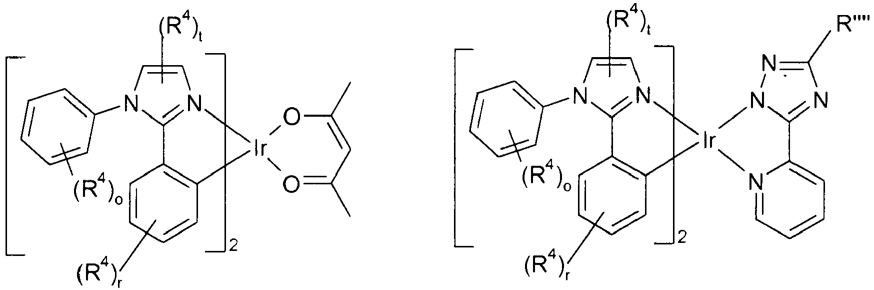 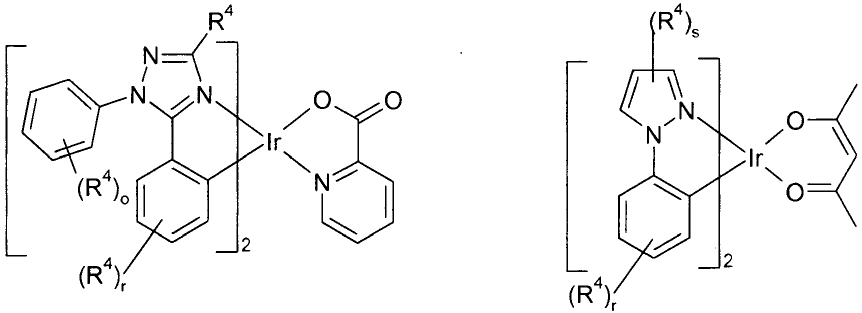 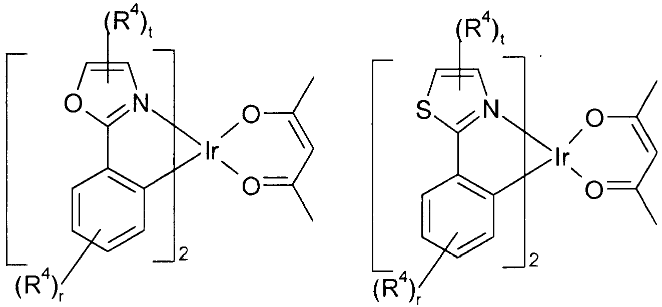 worin die Reste und Indizes R⁴, o, r, s und t die vorstehend bezüglich der bevorzugten Verbindungen der Formel (II) genannten Bedeutungen aufweisen.

Homoleptische Komplexe der allgemeinen Formel (IV): 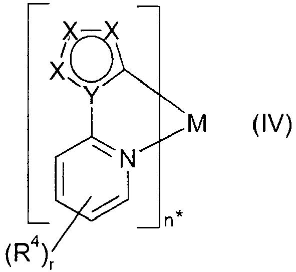 worin die Symbole und Reste X, Y, M, R⁴, n* und r jeweils unabhängig die bezüglich der allgemeinen Formel (II) genannten Bedeutungen aufweisen.

Bevorzugte homoleptische Komplexe der Formel (IV) weisen die folgenden Strukturen auf: 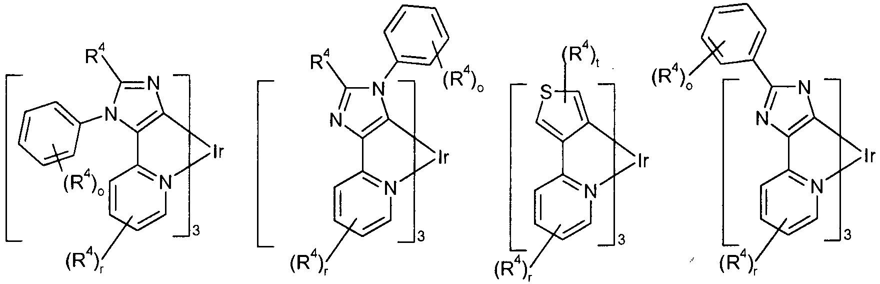 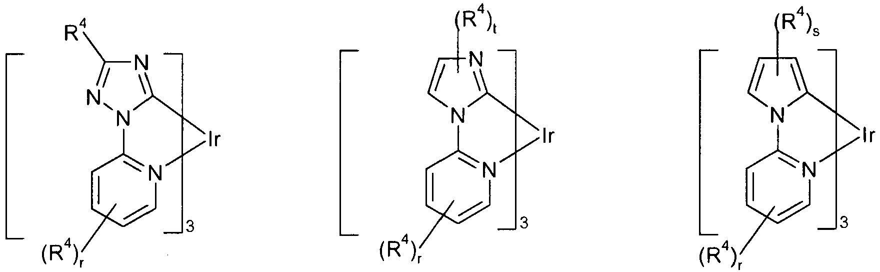 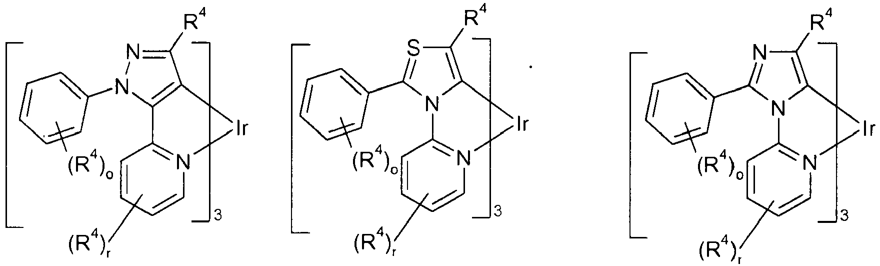 worin die Reste und Indizes R⁴, o, r, s und t die vorstehend bezüglich der bevorzugten Verbindungen der Formel (II) genannten Bedeutungen aufweisen.

Heteroleptische Komplexe der allgemeinen Formel (V): 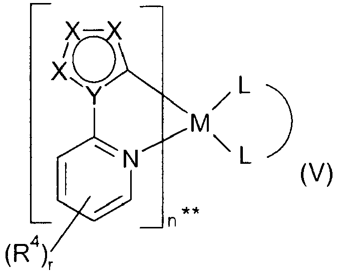 worin die Symbole und Reste X, Y, M, R⁴, n**, r und 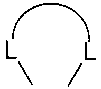 jeweils unabhängig die bezüglich der allgemeinen Formel (III) genannten Bedeutungen aufweisen.

Bevorzugte heteroleptische Komplexe der Formel (V) weisen die folgenden Strukturen auf: 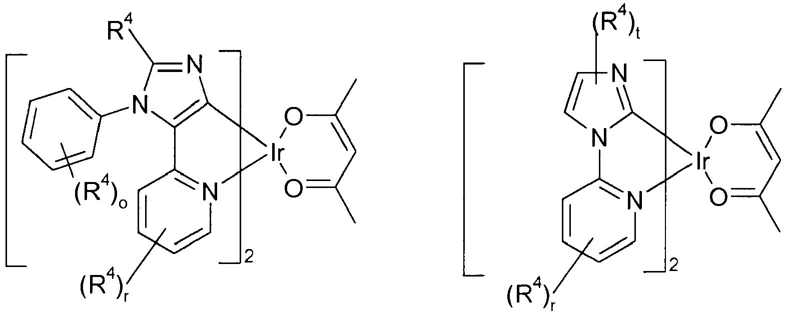 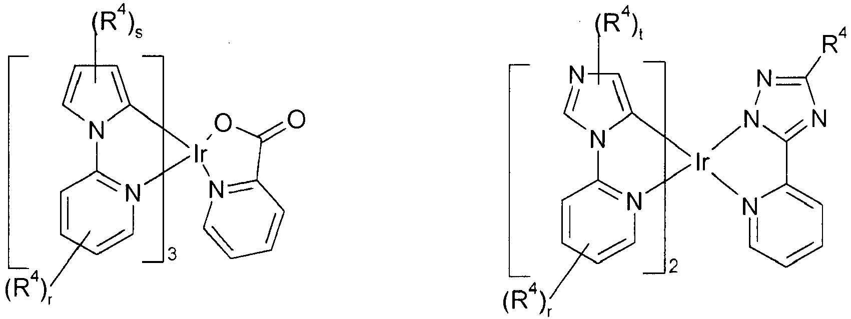 worin die Reste und Indizes R⁴, o, r, s und t die vorstehend bezüglich der bevorzugten Verbindungen der Formel (III) genannten Bedeutungen aufweisen.

Die Herstellung der vorstehend genannten Metallkomplexe erfolgt gemäß dem Fachmann bekannten Verfahren. Die Stöchiometrien und Reaktionsbedingungen sind für den Fachmann auf Basis der vorstehend genannten Patentanmeldungen problemlos zu ermitteln.

In einer bevorzugten Ausführungsform der vorliegenden Erfindung werden die Acridinderivate der Formel (I) in der Licht-emittierenden Schicht als Matrixmaterial gemeinsam mit Carbenkomplexen als Triplett-Emitter eingesetzt, d.h. besonders bevorzugte Triplett-Emitter sind Carbenkomplexe. Geeignete Carbenkomplexe sind dem Fachmann bekannt und in einigen der vorstehend genannten Anmeldungen und nachstehend genannt. In einer weiteren bevorzugten Ausführungsform werden die Acridinderivate der Formel (I) als Elektronenblockermaterial gemeinsam mit Carbenkomplexen als Triplett-Emitter eingesetzt. Die Acridinderivate der Formel (I) können des Weiteren sowohl als Matrixmaterialien als auch als Elektronenblockermaterialien gemeinsam mit Carbenkomplexen als Triplett-Emitter eingesetzt werden.

Geeignete Metallkomplexe zur Verwendung zusammen mit den Acridinderivaten der Formel I als Matrixmaterialien und/oder Elektronenblockermaterialien in OLEDs sind somit z.B. auch Carben-Komplexe, wie sie in WO 2005/019373 A2, WO 2006/056418 A2 und WO 2005/113704 und in den älteren nicht vorveröffentlichten Europäischen Anmeldungen EP 06 112 228.9 und EP 06 112 198.4 beschrieben sind. Insbesondere enthalten geeignete Metallkomplexe zur Verwerdung zusammen mit den Acridinderivaten der Formel I als Matrixmaterialien und/oder Elektronenblockermaterialien in OLEDs Carbenliganden der nachfolgenden, unter anderem in WO 2005/019373 A2 offenbarten Strukturen (die Bezeichnung der im Folgenden verwendeten Variablen wurde aus der Anmeldung WO 2005/019373 A2 übernommen; im Hinblick auf die genauere Definition der Variablen wird ausdrücklich auf diese Anmeldung verwiesen): 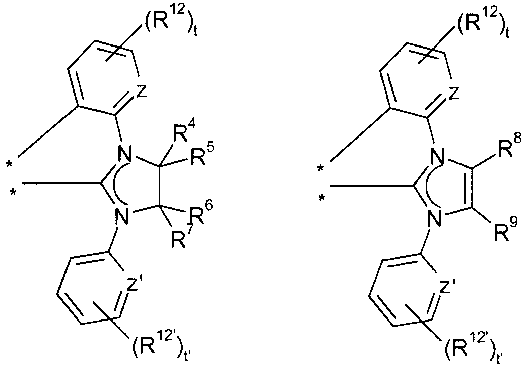 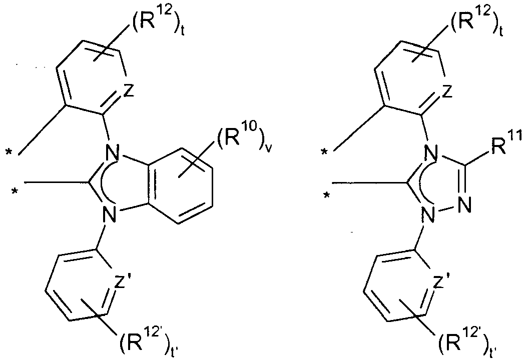 worin bedeuten:
- *: die Anbindungsstellen des Liganden an das Metallzentrum;
- z, z': gleich oder verschieden, CH oder N;
- R¹², R^{12'}: gleich oder verschieden, ein Alkyl-, Aryl-, Heteroaryl- oder Alkenylrest, bevorzugt ein Alkyl- oder Arylrest oder jeweils 2 Reste R¹² bzw. R^{12'} bilden gemeinsam einen anellierten Ring, der gegebenenfalls mindestens ein Heteroatom, bevorzugt N, enthalten kann, bevorzugt bilden jeweils 2 Reste R¹² bzw. R^{12'} gemeinsam einen anellierten aromatischen C₆-Ring, wobei an diesen, bevorzugt sechsgliedrigen, aromatischen Ring gegebenenfalls ein oder mehrere weitere aromatische Ringe anelliert sein können, wobei jede denkbare Anellierung möglich ist, und die anellierten Reste wiederum substituiert sein können; oder R¹² bzw. R^{12'} bedeutet einen Rest mit Donor- oder Akzeptorwirkung, bevorzugt ausgewählt aus der Gruppe bestehend aus Halogenresten, bevorzugt F, Cl, Br, besonders bevorzugt F; Alkoxy-, Aryloxy-, Carbonyl-, Ester-, Aminogruppen, Amidresten, CHF₂, CH₂F, CF₃, CN, Thiogruppen und SCN;
t und t' gleich oder verschieden, bevorzugt gleich, 0 bis 3, wobei, wenn t bzw. t' > 1 ist, die Reste R¹² bzw. R^{12'} gleich oder verschieden sein können, bevorzugt ist t bzw. t' 0 oder 1, der Rest R¹² bzw. R^{12'} befindet sich, wenn t bzw. t' 1 ist, in ortho-, meta- oder para-Position zur Verknüpfungsstelle mit dem dem Carbenkohlenstoffatom benachbarten Stickstoffatom;
- R⁴, R⁵, R⁶, R⁷, R⁸, R⁹ und R¹¹: Wasserstoff, Alkyl, Aryl, Heteroaryl, Alkenyl oder ein Substituent mit Donor- oder Akzeptorwirkung, bevorzugt ausgewählt aus Halogenresten, bevorzugt F, Cl, Br, besonders bevorzugt F, Alkoxyresten, Aryloxyresten, Carbonylresten, Esterresten, Aminresten, Amidresten, CH₂F-Gruppen, CHF₂-Gruppen, CF₃-Gruppen, CN-Gruppen, Thiogruppen und SCN-Gruppen, bevorzugt Wasserstoff, Alkyl, Heteroaryl oder Aryl,
- R¹⁰: Alkyl, Aryl, Heteroaryl oder Alkenyl, bevorzugt Alkyl, Heteroaryl oder Aryl, oder jeweils 2 Reste R¹⁰ bilden gemeinsam einen anellierten Ring, der gegebenenfalls mindestens ein Heteroatom, bevorzugt Stickstoff, enthalten kann, bevorzugt bilden jeweils 2 Reste R¹⁰ gemeinsam einen anellierten aromatischen C₆-Ring, wobei an diesen, bevorzugt sechsgliedrigen, aromatischen Ring gegebenenfalls ein oder mehrere weitere aromatische Ringe anelliert sein können, wobei jede denkbare Anellierung möglich ist, und die anellierten Reste wiederum substituiert sein können; oder R¹⁰ bedeutet einen Rest mit Donor- oder Akzeptorwirkung, bevorzugt ausgewählt aus der Gruppe bestehend aus Halogenresten, bevorzugt F, Cl, Br, besonders bevorzugt F; Alkoxy-, Aryloxy-, Carbonyl-, Ester-, Aminogruppen, Amidresten, CHF₂, CH₂F, CF₃, CN, Thiogruppen und SCN
- v: 0 bis 4, bevorzugt 0, 1 oder 2, ganz besonders bevorzugt 0, wobei, wenn v 0 ist, die vier Kohlenstoffatome des Arylrests in Formel c, die gegebenenfalls mit R¹⁰ substituiert sind, Wasserstoffatome tragen.

Insbesondere enthalten geeignete Metallkomplexe zur Verwendung zusammen mit den Acridinderivaten der Formel I als Matrixmaterialien und/oder Elektronenblockermaterialien in OLEDs Ir-Carbenkomplexe der nachfolgenden, in WO 2005/019373 A2 offenbarten Strukturen: 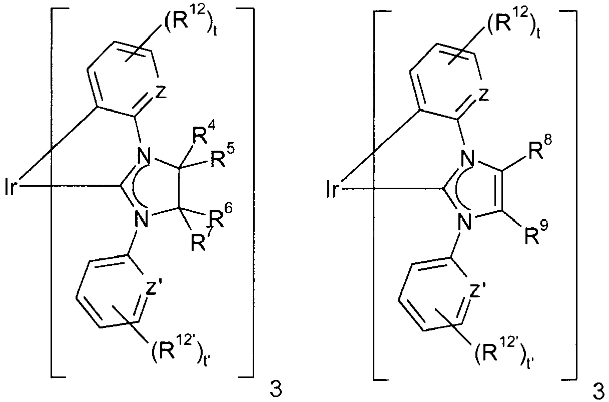 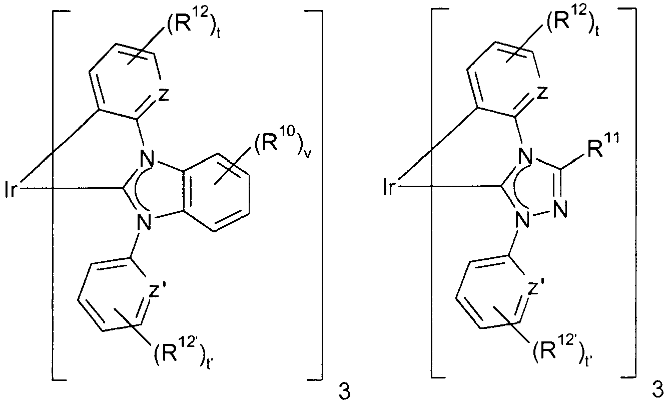 wobei die Variablen die bereits vorstehend genannten Bedeutungen aufweisen.

Weitere geeignete Metallkomplexe zur Verwendung zusammen mit den Acridinderivaten der Formel I als Matrixmaterialien und/oder Elektronenblockermaterialien in OLEDs sind insbesondere auch in WO 2006/056418 A2 offenbarte Strukturen (die Bezeichnung der im Folgenden verwendeten Variablen wurde aus der Anmeldung WO 2006/056418 A2 übernommen; im Hinblick auf die genauere Definition der Variablen wird ausdrücklich auf diese Anmeldung verwiesen): 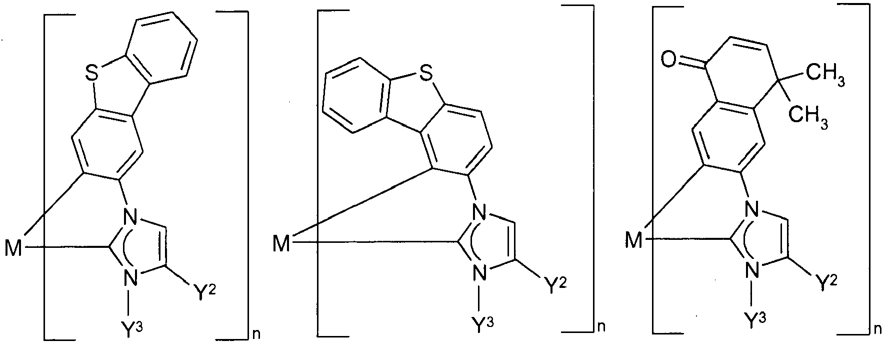 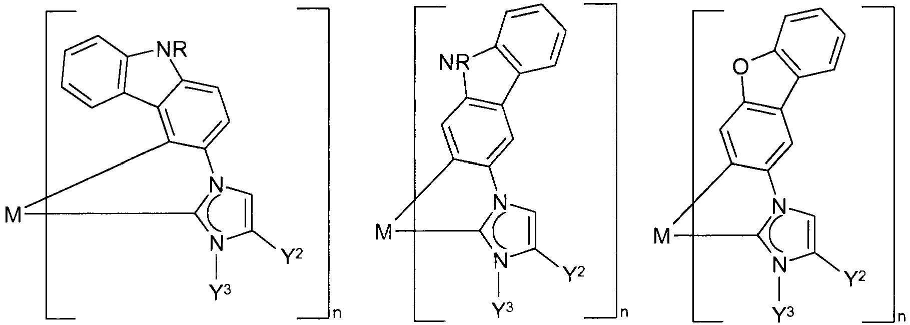 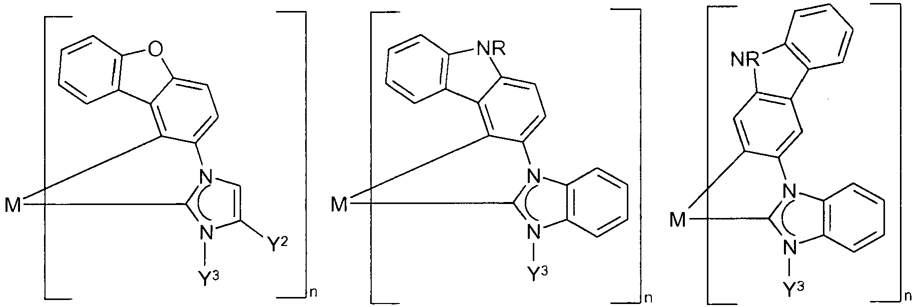 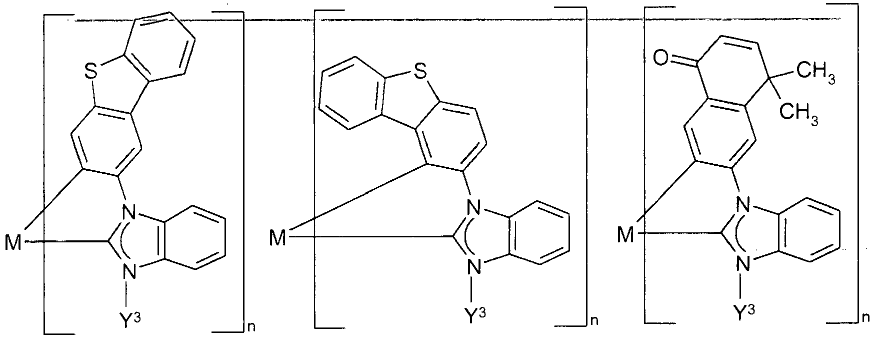 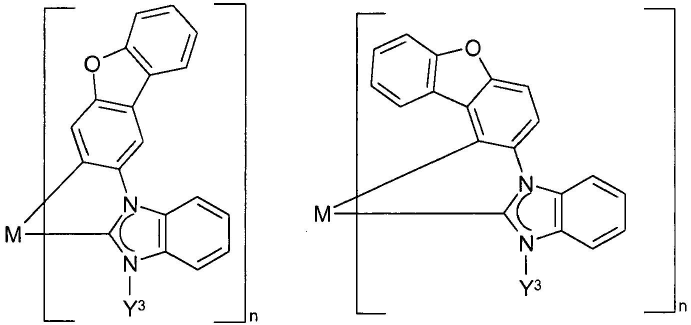 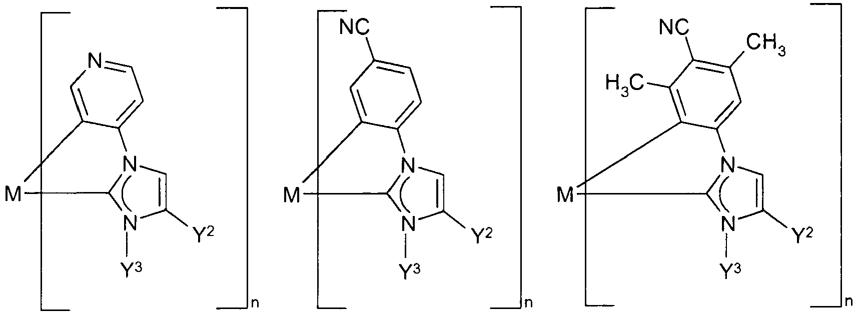 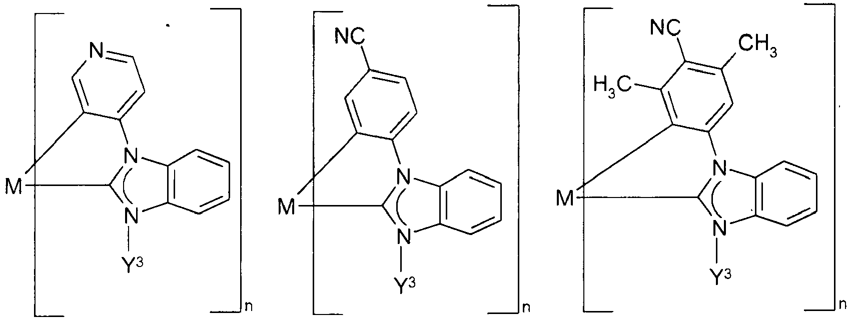 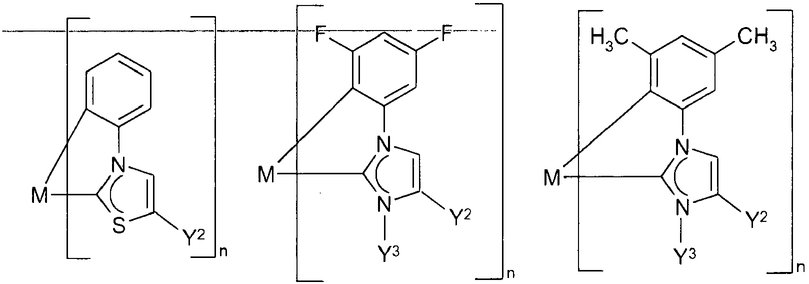 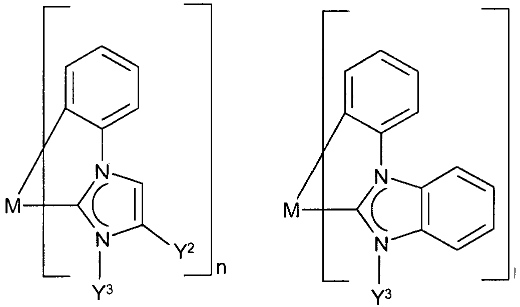 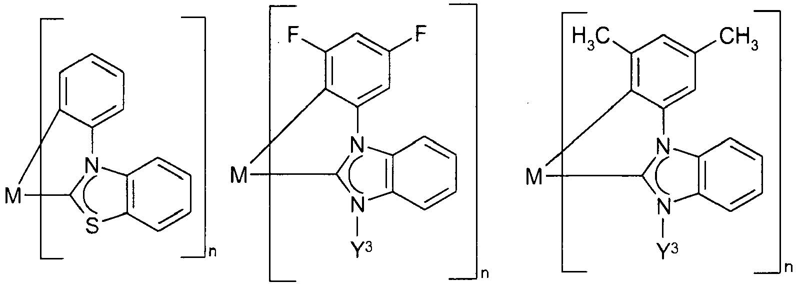 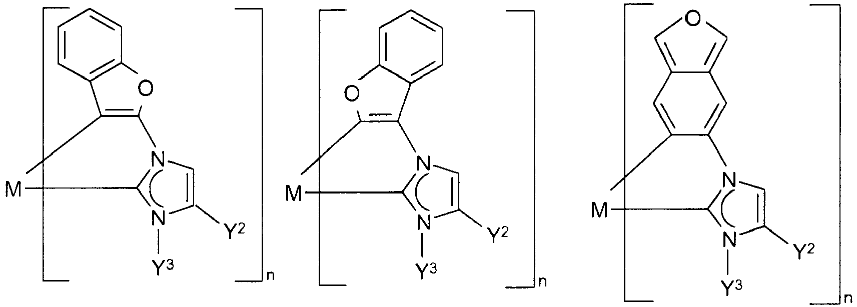 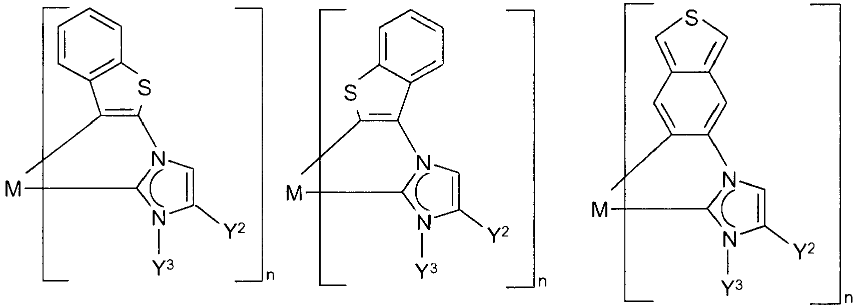 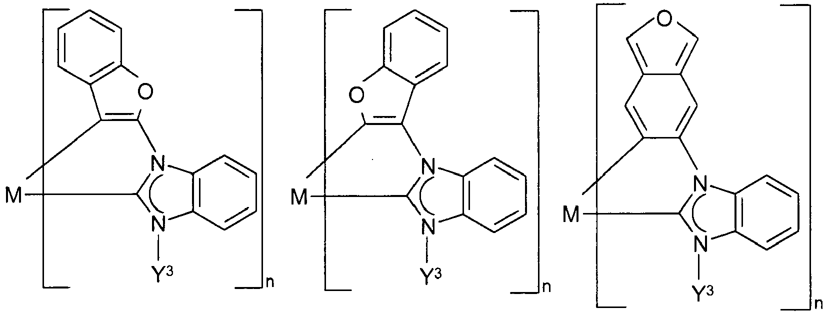 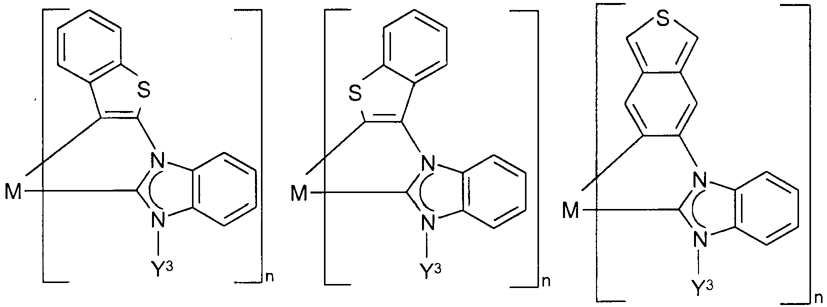 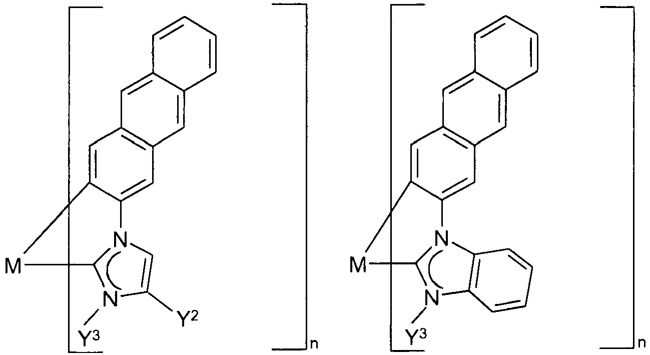 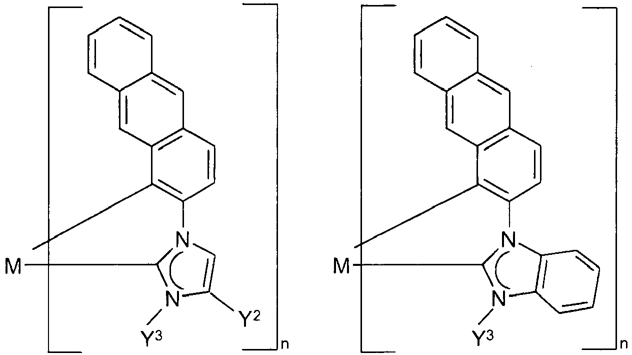 worin M für Ru(III), Rh(III), Ir(III), Pd(II) oder Pt(II) steht, n für Ru(III), Rh(III) und Ir(III) den Wert 3, für Pd(II) und Pt(II) den Wert 2 annimmt und Y² und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Weitere geeignete Metallkomplexe zur Verwendung zusammen mit den Acridinderivaten der Formel I als Matrixmaterialien und/oder Elektronenblockermaterialien in OLEDs sind insbesondere auch: 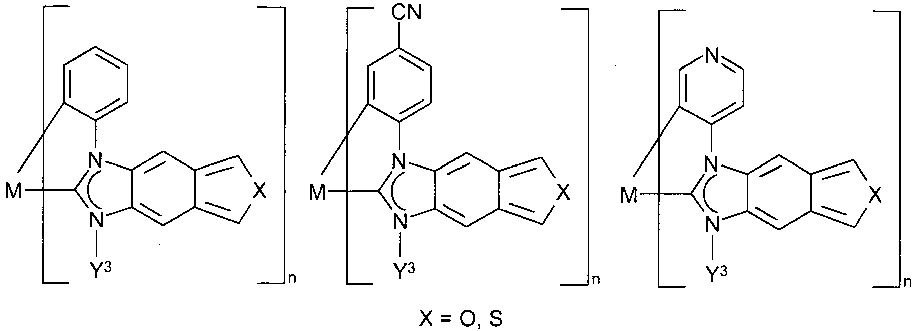 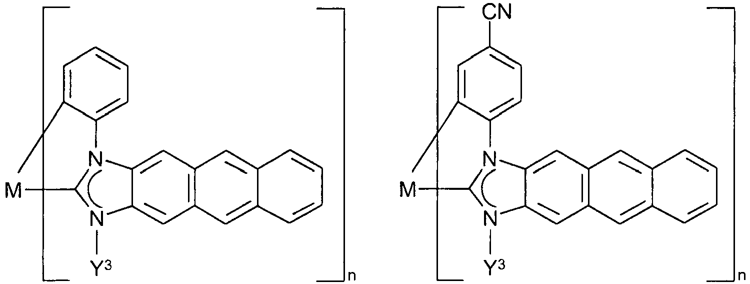 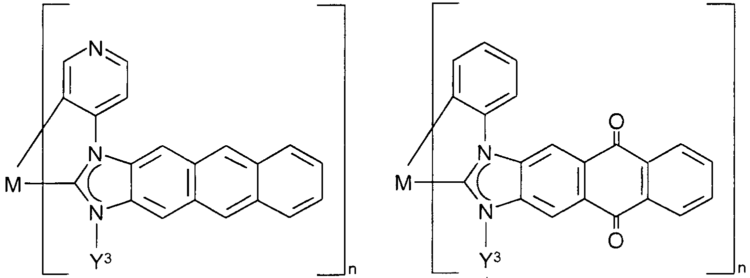 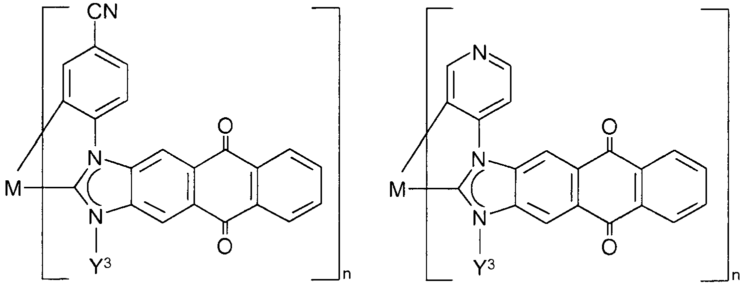 worin M für Ru(III), Rh(III), Ir(III), Pd(II) oder Pt(II) steht, n für Ru(III), Rh(III) und Ir(III) den Wert 3, für Pd(II) und Pt(II) den Wert 2 annimmt und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Weitere geeignete Metallkomplexe zur Verwendung zusammen mit den Acridinderivaten der Formel I als Matrixmaterialien und/oder Elektronenblockermaterialien in OLEDs sind insbesondere auch: 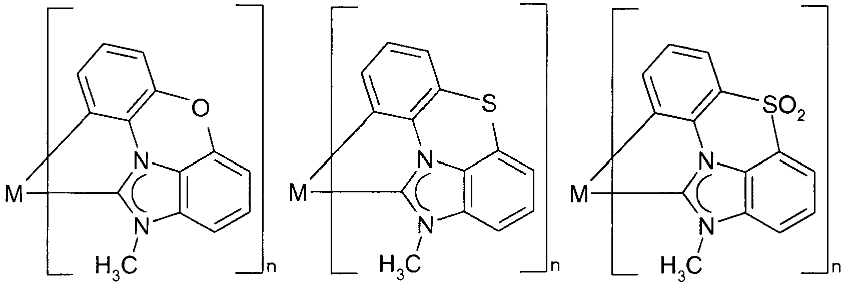 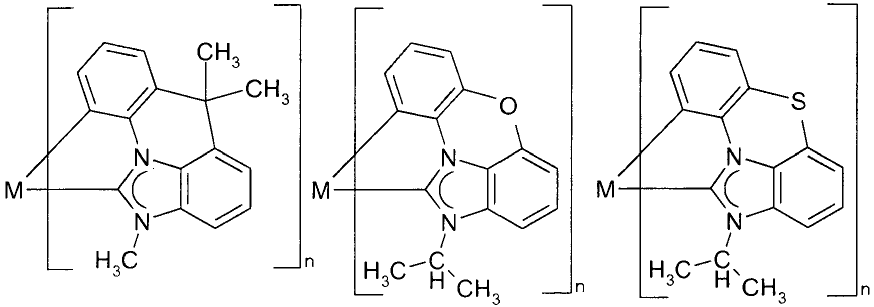 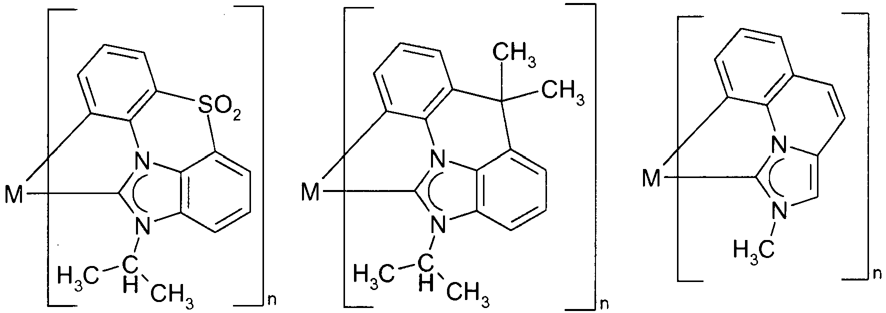 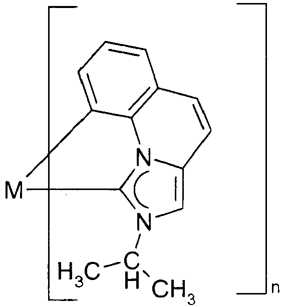 worin M für Ru(III), Rh(III) und insbesondere Ir(III), Pd(II) oder Pt(II) steht, n für Ru(III), Rh(III) und Ir(III) den Wert 3 und für Pd(II) und Pt(II) den Wert 2 annimmt.

Weitere geeignete Metallkomplexe zur Verwendung zusammen mit den Acridinderivaten der Formel I als Matrixmaterialien und/oder Elektronenblockermaterialien in OLEDs sind insbesondere auch: 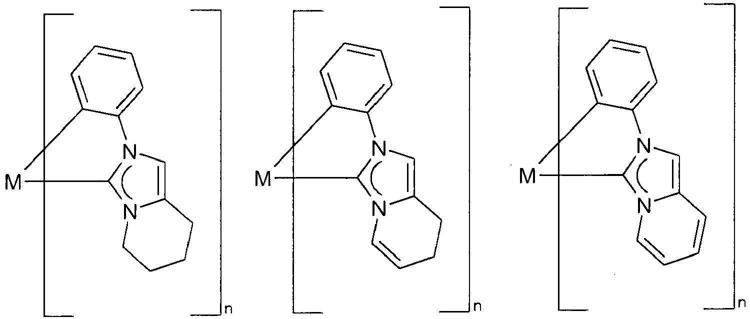 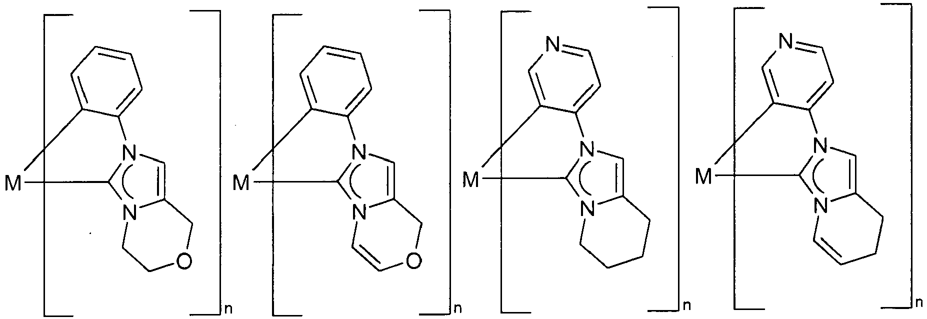 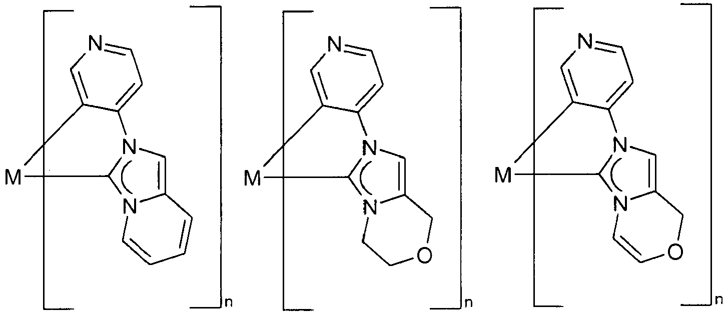 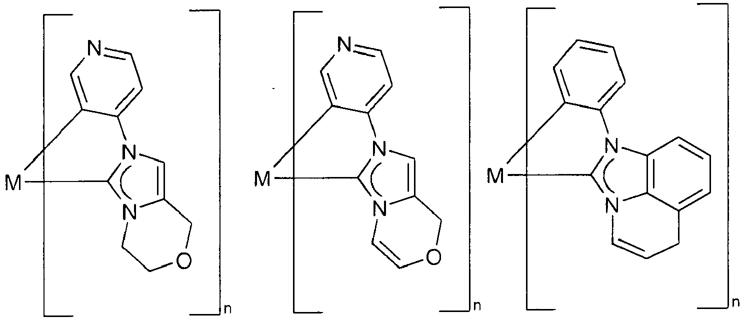 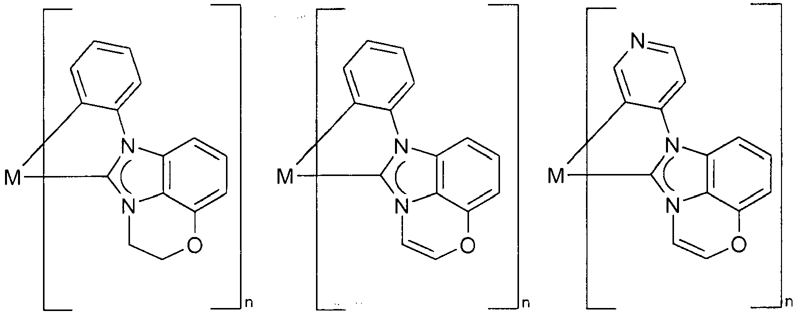 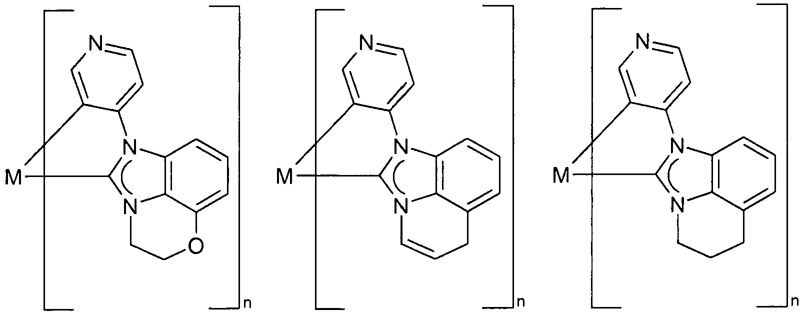 worin M für Ru(III), Rh(III) und insbesondere Ir(III), Pd(II) oder Pt(II) steht, n für Ru(III), Rh(III) und Ir(III) den Wert 3 und für Pd(II) und Pt(II) den Wert 2 annimmt.

Des Weiteren kommen auch Komplexe mit verschiedenen Carbenliganden und/oder mit mono- oder dianionischen Liganden, die sowohl mono- als auch bidentat sein können, in Frage.

Anhand der nachfolgenden Tabelle seien schematisch Komplexe ML'(L")₂ mit dreiwertigen Metallzentren und zwei verschiedenen Carbenliganden L' und L" genannt

| L' | L" | L' | L" | L' | L" | L' | L" |
|---|---|---|---|---|---|---|---|
| L¹ | L² | L³ | L⁴ | L⁷ | L⁵ | L⁵ | L³ |
| L¹ | L³ | L³ | L⁵ | L⁷ | L⁴ | L⁵ | L² |
| L¹ | L⁴ | L³ | L⁶ | L⁷ | L³ | L⁵ | L¹ |
| L¹ | L⁵ | L³ | L⁷ | L⁷ | L² | L⁴ | L³ |
| L¹ | L⁶ | L⁴ | L⁵ | L⁷ | L¹ | L⁴ | L² |
| L¹ | L⁷ | L⁴ | L⁶ | L⁶ | L⁵ | L⁴ | L¹ |
| L² | L³ | L⁴ | L⁷ | L⁶ | L⁴ | L³ | L² |
| L² | L⁴ | L⁵ | L⁶ | L⁶ | L³ | L³ | L¹ |
| L² | L⁵ | L⁵ | L⁷ | L⁶ | L² | L² | L¹ |
| L² | L⁶ | L⁶ | L⁷ | L⁶ | L¹ | | |
| L² | L⁷ | L⁷ | L⁶ | L⁵ | L⁴ | | |

wobei M beispielsweise für Ru(III), Rh(III) oder Ir(III), insbesondere Ir(III), und L' und L" beispielsweise für Liganden ausgewählt aus der Gruppe der Liganden L¹ bis L⁷ 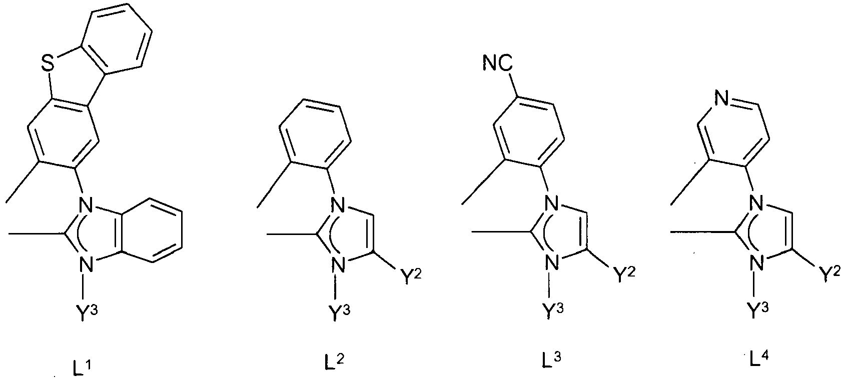 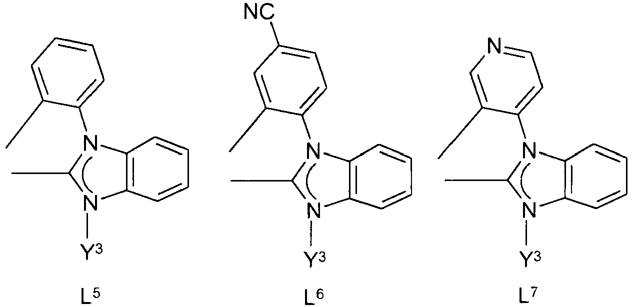 stehen, Y² Wasserstoff, Methyl, Ethyl, n-Propyl, iso-Propyl oder tert.-Butyl und Y³ Methyl, Ethyl, n-Propyl, iso-Propyl oder tert.-Butyl. bezeichnet.

Ein Vertreter dieser Komplexe mit verschiedenen Carbenliganden (L' = L⁴ mit Y² = Wasserstoff und Y³ = Methyl; L" = L² mit Y² = Wasserstoff und Y³ = Methyl) ist beispielsweise:

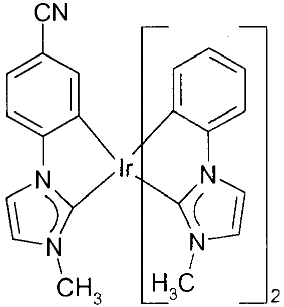

Selbstverständlich können in den als Emitter in den Matrixmaterialien und/oder gemeinsam mit den Elektronenblockermaterialien der Formel I verwendeten Komplexen dreiwertiger Metallzentren (etwa im Falle von Ru(III), Rh(III) oder Ir(III)) auch alle drei Carbenliganden verschieden voneinander sein.

Beispiele für Komplexe dreiwertiger Metallzentren M mit Liganden L (hier monoanionischer, bidentater Ligand) als "Zuschauerliganden" sind LML'L", LM(L')₂ und L₂ML', worin M etwa für Ru(III), Rh(III) oder Ir(III), insbesondere Ir(III), steht, und L' und L" die zuvor aufgeführte Bedeutung besitzen. Für die Kombination von L' und L" in den Komplexen LML'L" ergibt sich hierbei:

| L' | L" | L' | L" |
|---|---|---|---|
| L¹ | L² | L³ | L⁴ |
| L¹ | L³ | L³ | L⁵ |
| L¹ | L⁴ | L³ | L⁶ |
| L¹ | L⁵ | L³ | L⁷ |
| L¹ | L⁶ | L⁴ | L⁵ |
| L¹ | L⁷ | L⁴ | L⁶ |
| L² | L³ | L⁴ | L⁷ |
| L² | L⁴ | L⁵ | L⁶ |
| L² | L⁵ | L⁵ | L⁷ |
| L² | L⁶ | L⁶ | L⁷ |
| L² | L⁷ | | |

Als Liganden L kommen vor allem das Acetylacetonat und dessen Derivate. das Picolinat, Schiffsche Basen, Aminosäuren sowie die in WO 02/15645 A1 genannten bidentaten monoanionischen Liganden in Frage; insbesondere sind das Acetylacetonat und Picolinat von Interesse. Im Falle der Komplexe L₂ML' können die Liganden L gleich oder verschieden sein.

Ein Vertreter dieser Komplexe mit verschiedenen Carbenliganden (L' = L⁴ mit Y² = Wasserstoff und Y³ = Methyl; L" = L² mit Y² = Wasserstoff und Y³ = Methyl) ist beispielsweise: 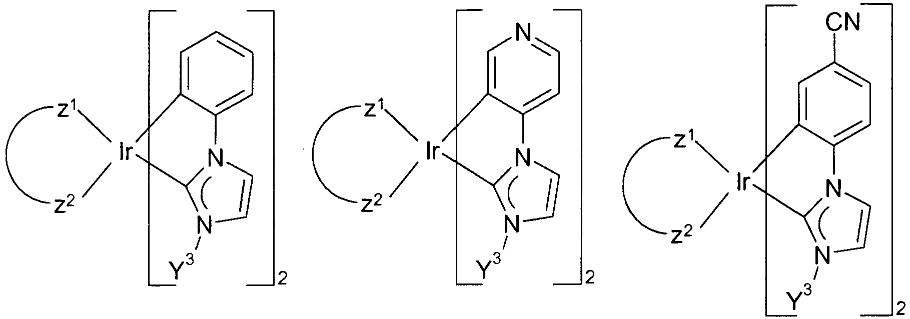 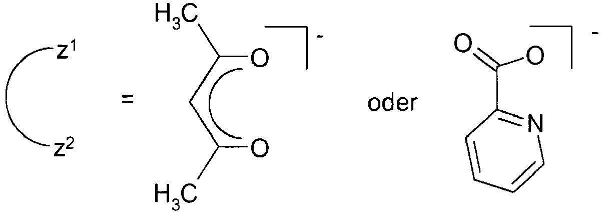 worin z¹ und z² im Symbol 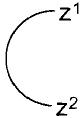 für die beiden Zähne des Liganden L stehen. Y³ bezeichnet Wasserstoff, Methyl, Ethyl, n-Propyl, iso-Propyl oder tert.-Butyl, insbesondere Methyl, Ethyl, n-Propyl oder iso-Propyl.

Weitere insbesondere als Emitter-Verbindungen geeignete Metallkomplexe zur Verwendung gemeinsam mit den Acridinderivaten der Formel I als Matrixmaterialien und/oder Elektronenblockermaterialien in OLEDs sind: 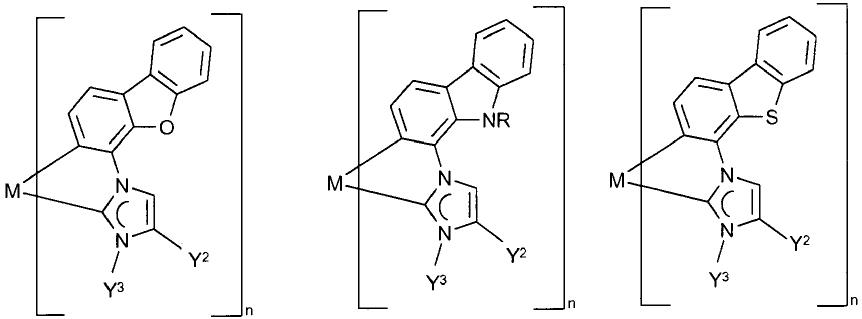 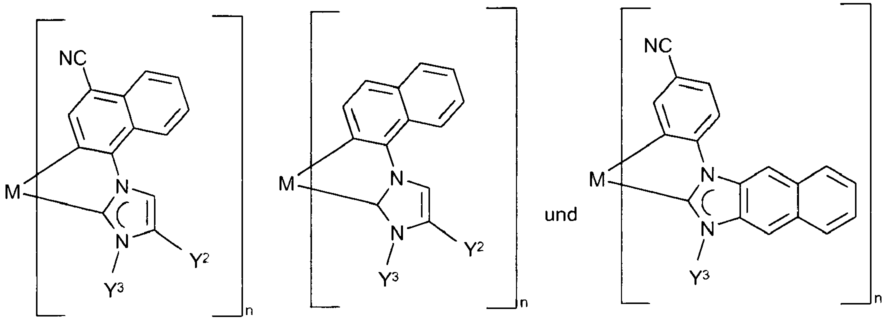 sowie 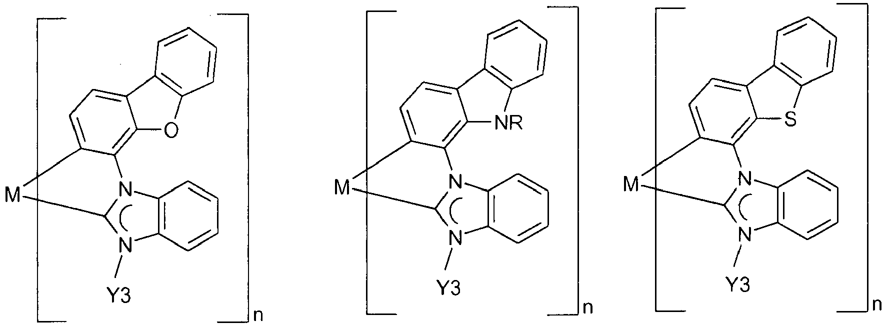 worin M für Ru(III), Rh(III), Ir(III), Pd(II) oder Pt(II) steht, n für den Fall, dass M Ru(III), Rh(III) und Ir(III) bedeutet, den Wert 3 und für den Fall, dass M Pd(II) und Pt(II) bedeutet, den Wert 2 annimmt und Y² und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Des Weiteren sind die folgenden speziellen Metallkomplexe für den Einsatz in OLEDs, insbesondere als Emitter-Verbindungen, gemeinsam mit den Verbindungen der Formel I als Matrixmaterialien geeignet: 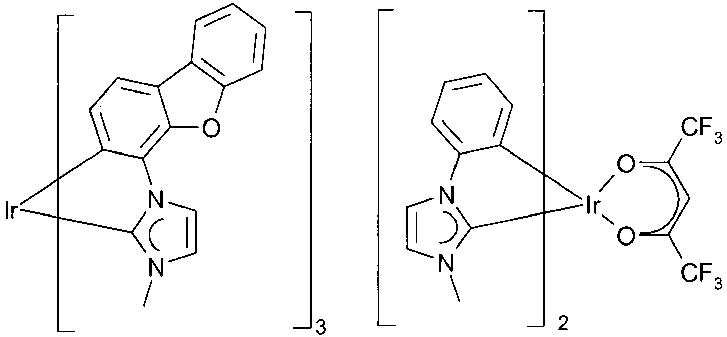 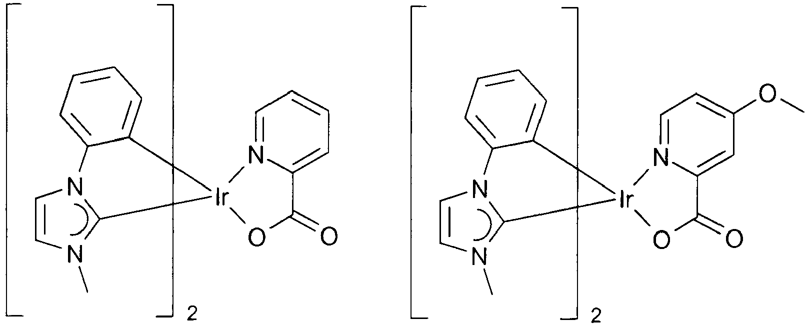 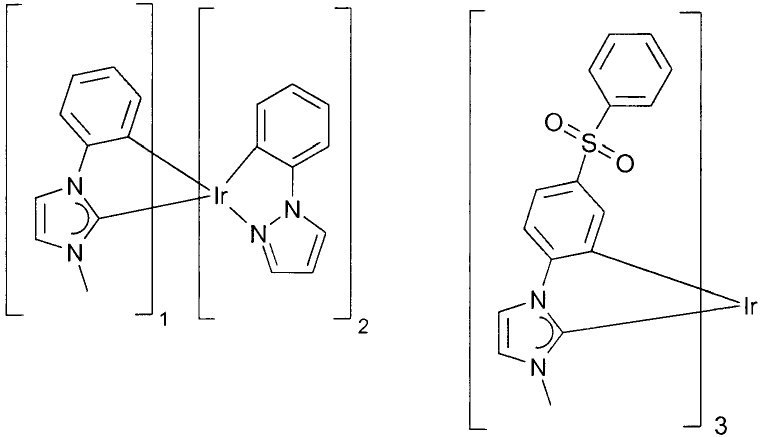 und

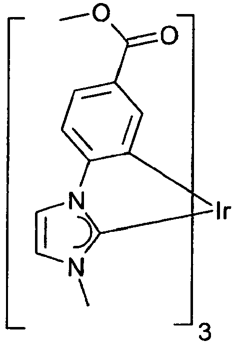

Die Herstellung der vorstehend genannten Carbenkomplexe erfolgt gemäß dem Fachmann bekannten Verfahren. Die Stöchiometrien und Reaktionsbedingungen sind für den Fachmann auf Basis der vorstehend genannten Patentanmeldungen betreffend Carbenkomplexe und deren Herstellungsverfahren problemlos zu ermitteln. Zusätzlich sind im Beispielteil der vorliegenden Anmeldung Verfahren zur Herstellung einiger der vorstehend genannten Carbenkomplexe angegeben. Die nicht ausdrücklich in den Beispielen beschriebenen Carbenkomplexe können in Analogie zu den im Beispielteil beschriebenen Verfahren hergestellt werden.

Wird mindestens ein Acridinderivat der Formel (I) gemeinsam mit einem Triplett-Emitter, besonders bevorzugt gemeinsam mit einem Carben-Komplex als Triplett-Emitter, in der Licht-emittierenden Schicht eines OLEDs eingesetzt, beträgt der Anteil des mindestens einen Acridinderivats der Formel (I) in der Licht-emittierenden Schicht im Allgemeinen 50 bis 99 Gew.-%, bevorzugt 60 bis 95 Gew.-%, besonders bevorzugt 70 bis 90 Gew.-%. Der Anteil der Emitter-Verbindung in der Licht-emittierenden Schicht beträgt im Allgemeinen 1 bis 50 Gew.-%, bevorzugt 5 bis 40 Gew.-%, besonders bevorzugt 10 bis 30 Gew.-%, wobei die Anteile an dem mindestens einen Acridinderivat der Formel (I) und der mindestens einen Emitter-Verbindung im Allgemeinen 100 Gew.-% ergeben. Es ist jedoch auch möglich, dass die Licht-emittierende Schicht neben dem mindestens einen Acridinderivat der Formel (I) und der mindestens einen Emitter-Verbindung weitere Substanzen enthält, zum Beispiel weiteres Verdünnungsmaterial, wobei geeignetes Verdünnungsmaterial nachstehend genannt wird.

Organische Leuchtdioden (OLEDs) sind grundsätzlich aus mehreren Schichten aufgebaut, z. B.:
1. Anode
2. Löcher-transportierende Schicht
3. Licht-emittierende Schicht
4. Elektronen-transportierende Schicht
5. Kathode

Es sind auch von dem vorstehend genannten Aufbau verschiedene Schichtenfolgen möglich, die dem Fachmann bekannt sind. Beispielsweise ist es möglich, dass das OLED nicht alle der genannten Schichten aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transprotierende Schicht) und (4) (Elektronen-transprotierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Die Acridinderivate der Formel I können als Elektronen-blockierende Materialien und/oder Matrixmaterialien eingesetzt werden.

Die Acridinderivate der Formel I können dabei als alleiniges Matrixmaterial - ohne weitere Zusätze - in der Licht-emittierenden Schicht vorliegen. Es ist jedoch ebenfalls möglich, dass neben den erfindungsgemäß eingesetzten Acridinderivaten der Formel I weitere Verbindungen in der Licht-emittierenden Schicht vorliegen. Beispielsweise kann ein fluoreszierender Farbstoff anwesend sein, um die Emissionsfarbe des vorhandenen Emittermoleküls zu verändern. Des Weiteren kann ein Verdünnungsmaterial eingesetzt werden. Dieses Verdünnungsmaterial kann ein Polymer sein, zum Beispiel Poly(N-vinylcarbazol) oder Polysilan. Das Verdünnungsmaterial kann jedoch ebenfalls ein kleines Molekül sein, zum Beispiel 4,4'-N,N'-Dicarbazolbiphenyl (CBP = CDP) oder tertiäre aromatische Amine. Wenn ein Verdünnungsmaterial eingesetzt wird, beträgt der Anteil der erfindungsgemäß eingesetzten Verbindungen der Formel I in der Licht-emittierenden Schicht im Allgemeinen immer noch mindestens 40 Gew.-%, bevorzugt 50 bis 100 Gew.-% bezogen auf das Gesamtgewicht der Verbindungen der Formel I und Verdünnungsmittel.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein, in die aus der Elektrode Löcher injiziert werden, und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der Elektronen-transportierenden Schicht sollte mit der Arbeitsfunktion der Kathode angeglichen sein.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIIIa. Wenn die Anode lichtdurchlässig sein soll, werden im Allgemeinen gemischte Metalloxide der Gruppen IIb, IIIb und IVb des Periodensystems der Elemente (alte IUPAC-Version) eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus tris-[N-(1-naphthyl)-N-(phenylamino)]triphenylamin(1-NaphDATA), 4,4'-Bis[N-(1-naphthyl)-N-phenyl-amino]biphenyl (α-NPD), N,N'-Diphenyl-N,N'-bis(3-methylphenyl)-[1,1'-biphenyl]-4,4'-diamin (TPD), 1,1-Bis[(di-4-tolylamino)phenyl]-cyclohexan (TAPC), N,N'-Bis(4-methylphenyl)-N,N'-Bis(4-ethylphenyl)-[1,1'-(3,3'-dimelhyl)bipnenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB), 4,4',4"-tris(N,N-Diphenylamino)triphenylamin (TDTA), Porphyrinverbindungen und Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können die vorstehend als Emittermaterialien genannten Carben-Komplexe als Lochtransportmaterialien eingesetzt werden, wobei die Bandlücke des mindestens einen Lochtransportmaterials im Allgemeinen größer ist als die Bandlücke des eingesetzten Emittermaterials. Dabei ist unter Bandlücke im Sinne der vorliegenden Anmeldung die Triplett-Energie zu verstehen.

Geeignete Elektronentransportmaterialien für die Schicht (4) der erfindungsgemäßen OLEDs umfassen mit oxinoiden Verbindungen chelatisierte Metalle wie Tris(8-chinolinolato)aluminium (Alq₃), Verbindungen auf Phenanthrolinbasis wie 2,9-Dimethyl-4,7-diphenyl-1,10-phenanthrolin (DDPA = BCP) oder 4,7-Diphenyl-1,10-phenanthrolin (DPA) und Azolverbindungen wie 2,2',2"-(1,3,5-phenylen)tris-[1-phenyl-1H-benzimidazol] (TPBI), 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Von den vorstehend als Lochtransportmaterialien und Elektronentransportmaterialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochtransportmaterialien mit Elektronenakzeptoren dotiert werden, zum Beispiel können Phthalocyanine bzw. Arylamine wie TPD oder TDA mit Tetrafluortetracyanchinodimethan (F4-TCNQ) dotiert werden. Die Elektronentransportmaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten): A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (5) ist eine Elektrode, die zur Einführung von Elektronen oder negativen Ladungsträgern dient. Geeignete Materialien für die Kathode sind ausgewählt aus der Gruppe bestehend aus Alkalimetallen der Gruppe la, zum Beispiel Li, Cs, Erdalkalimetallen der Gruppe IIa, zum Beispiel Calcium, Barium oder Magnesium, Metallen der Gruppe IIb des Periodensystems der Elemente (alte IUPAC-Version), umfassend die Lanthaniden und Aktiniden, zum Beispiel Samarium. Des Weiteren können auch Metalle wie Aluminium oder Indium, sowie Kombinationen aller genannten Metalle eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Es ist jedoch auch möglich, dass das OLED nicht alle der genannten Schichten (1) bis (5) aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transportiende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren kann jede der genannten Schichten des erfindungsgemäßen OLEDs aus zwei oder mehreren Schichten ausgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz und Lebensdauer zu erhalten.

Die Herstellung des erfindungsgemäßen OLEDs kann nach dem Fachmann bekannten Methoden erfolgen. Im Allgemeinen wird das erfindungsgemäße OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden.

Im Allgemeinen haben die verschiedenen Schichten folgende Dicken: Anode (1) 500 bis 5000 Å, bevorzugt 1000 bis 2000 Å; Löcher-transportierende Schicht (2) 50 bis 1000 Å, bevorzugt 200 bis 800 Å, Licht-emittierende Schicht (3) 10 bis 1000 Å, bevorzugt 100 bis 800 Å, Elektronen transportierende Schicht (4) 50 bis 1000 Å, bevorzugt 200 bis 800 Å, Kathode (5) 200 bis 10.000 Å, bevorzugt 300 bis 5000 Å. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt.

Durch erfindungsgemäßen Einsatz der Acridinderivate der Formel I als Matrixmaterialien in der Licht-emittierenden Schicht in OLEDs können OLEDs mit hoher Effizienz erhalten werden. Die Effizienz der erfindungsgemäßen OLEDs kann des Weiteren durch Optimierung der anderen Schichten verbessert werden. Beispielsweise können hoch effiziente Kathoden wie Ca oder Ba, gegebenenfalls in Kombination mit einer Zwischenschicht aus LiF, eingesetzt werden. Geformte Substrate und neue Löchertranspor-tierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Digitalkameras, Fahrzeugen sowie Zielanzeigen an Bussen und Bahnen.

Weiterhin können die Acridinderivate der Formel I in OLEDs mit inverser Struktur als Matrixmaterial in der Licht-emittierenden Schicht eingesetzt werden. Der Aufbau von inversen OLEDs und die üblicherweise darin eingesetzten Materialien sind dem Fachmann bekannt.

Die nachfolgenden Beispiele erläutern die Erfindung zusätzlich.

### Beispiele

### A) Verfahren zur Herstellung von ausgewählten Carbenkomplexen

### Ir(DBF-MIC)₃ (V5)

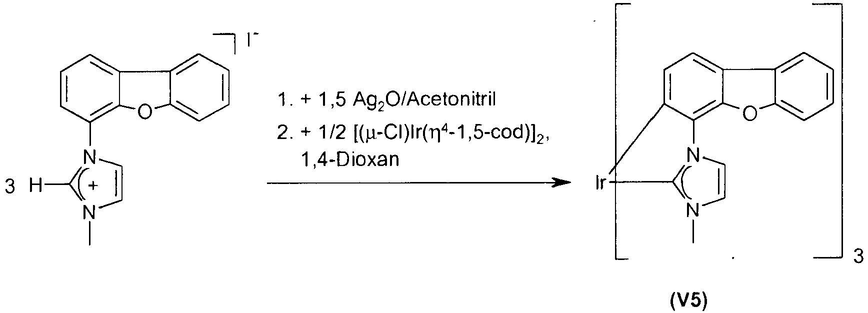

3.48 g (9.2 mmol) Imidazoliumiodid und 1.07 g (4.6 mmol) Silberoxid werden in 60 ml Acetonitril suspendiert und bei Raumtemperatur über Nacht gerührt. Anschließend wird die Suspension zur Trockene eingeengt, mit 100 ml 1,4-Dioxan aufgenommen und zu einer Lösung aus 0.62 g (0.92 mmol) [(µ -Cl)(η⁴-1,5-cod)Ir]₂ und 60 ml 1,4-Dioxan innerhalb von einer halben Stunde dosiert. Im Anschluss wird eine Stunde bei Raumtemperatur, zwei Stunden bei 70°C und 18 Stunden unter Rückfluss gerührt. Nach dem Abkühlen wird das Reaktionsgemisch zur Trockene eingeengt, mit Dichlormethan extrahiert und der Extrakt einer säulen-chromatographischen Aufreinigung (Eluent: 1. Methylenchlorid : Cyclohexan 2:1 zur Isolierung des Isomerengemisches und 2. Essigsäureethylester : Cyclohexan 1:1 zur Trennung der Isomeren, Verhältnis der Isomeren im Reaktionsgemisch : *mer*/*fac* ca. 3/1) unterzogen. Man erhält ca. 0.61 g (35%) *mer-*Isomer und 0.1 g (6%) *fac*-Isomer als hellgelbes Pulver.

### meridionales Isomer:

¹H-NMR: (DMSO, 500 MHz): 8.40 (d, *J*= 2.0 Hz, 1 H, CH), 8.34 (d, *J*= 2.1 Hz, 1 H, CH), 8.22 (d, *J* = 2.1 Hz, 1H, CH), 7.90 - 7.85 (m, 1 H, CH), 7.69 - 7.65 (m, 1 H, CH), 7.43 - 7.13 (m, 16H, CH), 6.75 (d, ³*J*_{H,H} = 7.5 Hz, 1 H, CH), 6.68 (d, ³*J*_{H,H} = 7.7 Hz, 1 H, CH), 6.56 (d, ³*J*_{H},_{H} = 7.6 Hz, 1 H, CH), 3.13 (s, 3H, CH₃), 3.05 (s, 3H, CH₃), 2.99 (s, 3H, CH₃).

### faciales Isomer:

¹H-NMR: (DMSO, 500 MHz): 8.28 (d, *J*= 1.95 Hz, 3H, CH), 7.88-7.89 (m, 3H, CH), 7.67-7.68 (m, 3H, CH), 7.43 - 7.12 (m, 12H, CH), 6.50 (d, *J*_{H,H}= 7.5 Hz, 3H, CH), 3.14 (s, 9H, CH₃).

### [(PMIC)₂IrCI]₂ (V6)

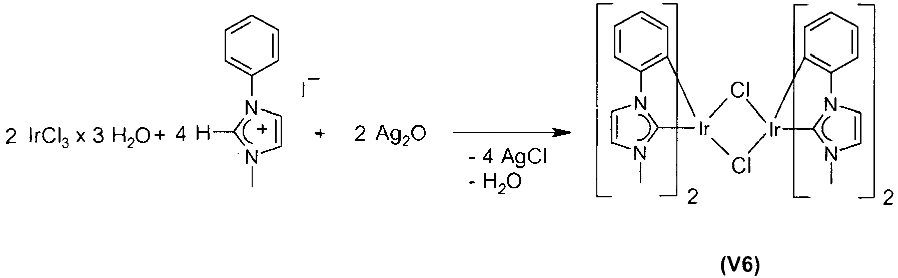

4.29 g (18.5 mmol) Silberoxid, 9.47g (33.1 mmol) Imidazoliumiodid und 3.56 g (10.1 mmol) Iridiumtrichloridtrihydrat werden in 350 ml 2-Ethoxyethanol suspendiert und 15 h bei 120°C im Dunkeln gerührt. Danach entfernt man das Lösungsmittel im Vakuum und extrahiert den Rückstand mit Methylenchlorid. Der Extrakt wird auf ca. ein Viertel seines Volumens eingeengt und mit Methanol versetzt. Der ausfallende Niederschlag wird abfiltriert und getrocknet. Man erhält 1.7 g [(PMIC)₂IrCI]₂(31%).

¹H-NMR: (CD₂Cl₂, 500 MHz): δ = 7.59 (d, *J* = 2.3 Hz, 4H, CH), 7.17 (d, *J* = 1.7 Hz, 4H, CH), 6.99 (d, ³*J*_{H,H} = 7.2 Hz, 4H, CH), 6.73 (pt, ³*J*_{H,H} = 7.5 Hz, 4H, CH), 6.45 (pt, ³*J*_{H,H} = 7.9 Hz, 4H, CH), 6.09 (d, ³*J*_{H,H} = 7.3 Hz, 4H, CH), 3.91 (s, 12H, CH₃).

### (PMIC)₂Irpic (V7)

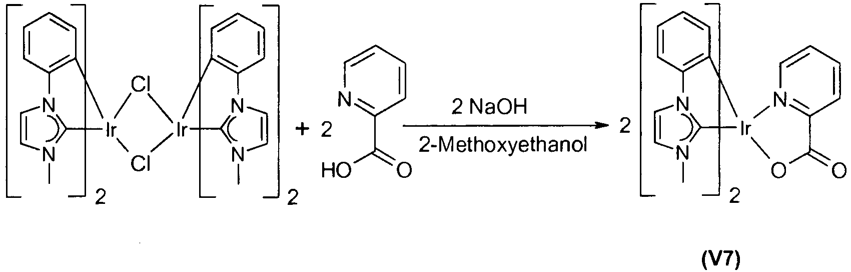

Eine Lösung von 0.41 g (3.32 mmol) Picolinsäure in Methoxyethanol (30 ml) wird innerhalb von 10 min mit 3.32 ml Natronlauge (1 M, 3.32 mmol) versetzt und 15 min bei Raumtemperatur gerührt. Danach wird das Reaktionsgemisch innerhalb von 10 min zu einer Suspension von 0.9 g (0.83 mmol) [(PMIC)₂IrCI]₂ in Methoxyethanol (80 ml) gegeben. Es wird 15 min bei Raumtemperatur gerührt und anschließend 21 h am Rückfluss erhitzt. Nach dem Abkühlen wird das Reaktionsgemisch mit Wasser (300 ml) versetzt. Der dabei entstandene Niederschlag wird abfiltriert, getrocknet und einer säulenchromatographischen Reinigung unterworfen (Eluent: Ethylacetat/ Methanol = 1/0.25). Man erhält 0.64 g (PMIC)₂IrPic (61%).

¹H-NMR: (CD₂Cl₂, 500 MHz): δ = 3.00 (s, 3H, CH₃), 3.86 (s, 3H, CH₃), 6.31-6.33 (m, 1H, CH), 6.42-6.44 (m, 1H, CH), 6.59-6.63 (m, 2H, CH), 6.83-6.88 (m, 2H, CH), 6.90-6.91 (m, 1 H, CH), 6.98-6.99 (m, 1 H, CH), 7.08 (d, *J* = 7.8 Hz, 2H, CH), 7.21-7.24 (m, 1H, CH), 7.46-7.47 (m, 2H, CH), 7.80-7.83 (dt, *J* = 7.7 Hz, *J* = 1.5 Hz, 1 H, CH), 7.92-7.93 (m, 1H, CH), 8.13-8.15 (m, 1 H, CH).

### (PMIC)₂IrPicOMe (V8)

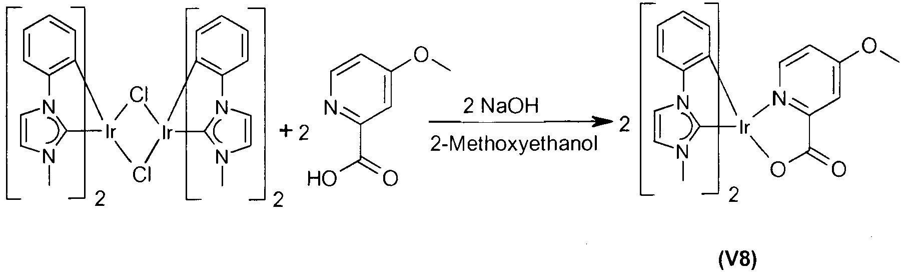

Eine Suspension von 0.68 g (4.44 mmol) 4-Methoxypicolinsäure in 70 ml 2-Methoxyethanol wird innerhalb von 10 min mit 4.44 ml Natronlauge (1M, 4.44 mmol) versetzt. Es wird 15 min bei Raumtemperatur gerührt, bevor man das Gemisch langsam zu einer Suspension aus 1.2g (1.11 mmol) [(PMIC)₂IrCI]₂ und 80ml 2-Methoxyethanol gibt. Es wird 15 min bei Raumtemperatur gerührt und anschließend 21 h am Rückfluss erhitzt. Nach dem Abkühlen wird das Reaktionsgemisch mit Wasser (600 ml) versetzt. Der dabei entstandene Niederschlag wird abfiltriert, getrocknet und säulenchromatographisch gereinigt (Eluent: Ethylacetat/Methanol = 1/0.25). Man erhält 0.93 g PMIC₂IrPicOMe (64 %).

¹H-NMR: (CD₂Cl₂, 500 MHz): δ = 3.07 (s, 3H, CH₃), 3.85 (s, 3H, CH₃), 3.91 (s, 3H, CH₃), 6.33 (dd, *J* = 7.3 Hz, *J* = 1.4 Hz, 1 H, CH), 6.42 (dd, *J* = 7.4 Hz, *J* = 1.4 Hz, 1 H, CH), 6.57-6.61 (m, 2H, CH), 6.74 (dd, *J* = 6.3 Hz, *J* = 2.9 Hz, 1 H, CH), 6.81-6.86 (m, 2H, CH), 6.92 (d, *J* = 2.1 Hz, 1 H, CH), 6.98 (d, *J* = 2.1 Hz, 1 H, CH), 7.07 (dd, *J* = 4.8 Hz, *J =* 1.3 Hz, 1 H, CH), 7.08 (dd, *J* = 4.6 Hz, *J* = 1.3 Hz, 1 H, CH), 7.46 (dd, *J* = 4.1 Hz, *J* = 2.1 Hz, 2H, CH), 7.65 (d, *J* = 6.2 Hz, 1 H, CH), 7.72 (d, *J* = 2.5 Hz, 1 H, CH).

### (PMIC)₂Ir(acac-F₆) (V9)

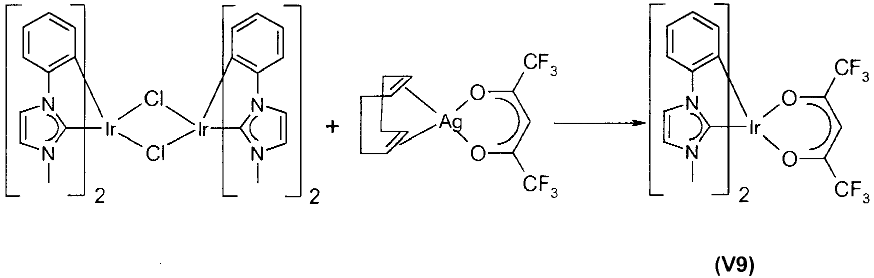

Eine Lösung von 0.22 g (0.2 mmol) [(PMIC)₂IrCI]₂ in 60 ml Methylenchlorid wurden mit einer Lösung von 0.17 g (0.4 mmol) (cod)Ag(acac-F₆) in 30 ml Methylenchlorid versetzt. Es wurde 2 h unter Rückfluss und 18 h bei Raumtemperatur gerührt. Dann wurde das Lösungsmittel im Vakuum entfernt und der Rückstand säulenchromatographisch gereinigt (Eluent CH₂Cl₂). Man erhielt 0.28 g (**96%**) rotes Pulver.

¹H-NMR: (CD₂Cl₂, 500 MHz): δ = 7.50 (s, 2H), 7.11 (s, 2H), 7.03 (m, 2H), 6.81 (m, 2H), 6.56 (m, 2H), 6.19 (m, 2H), 5.98 (s, 1 H), 3.79 (s, 6H).

### mer-Tris-[1-(4'-phenylsulfonylphenyl)-3-methylbenzimidazol-2-yliden-C2,C2]-iridium(III)(V10)

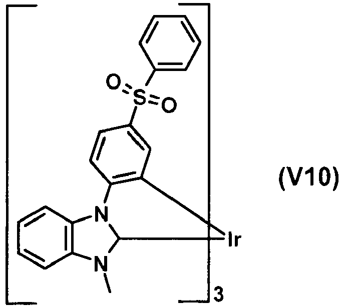

### 1-(4'-Phenylsulfonphenyl)-benzimidazol

Zu einer Lösung von Benzimidazol (11.8 g, 0.10 mol) in DMF (500 mL) wird bei Raumtemperatur unter Stickstoff Natriumhydrid (60% in Mineralöl, 4.4 g, 0.11 mol) gegeben und 10 min gerührt. Die Mischung wird mit 4-Chlorphenyl-phenylsulfon (26.1 g, 0.10 mol) versetzt und 16 h bei 100 °C gerührt. Nach erneuter Zugabe von Natriumhydrid (60% in Mineralöl, 2.0 g, 0.05 mol) bei Raumtemperatur wird die Mischung 16 h bei 130 °C gerührt. Nach Abkühlung auf Raumtemperatur wird die Mischung auf Eiswasser gegeben. Ausgefallenes Produkt wird abfiltriert und mit Wasser gewaschen. Ausbeute: 91%.

¹H-NMR (*d₆*-DMSO, 400 MHz): δ = 7.35 (m_{c}, 2H), 7.64-7.82 (m, 5H), 7.98 (d, 2H), 8.06 (d, 2H), 8.20 (d, 2H) 8.67 (s, 1 H).

### 1-(4'-Phenylsulfonphenyl)-3-methylbenzimidazolium-tetrafluoroborat

Eine Lösung von 1-(4'-Phenylsulfonphenyl)-benzimidazol (6.7 g, 20 mmol) in Dichlormethan (100 mL) wird bei -10 °C mit Trimethyloxonium-tetrafluoroborat (3.3 g, 22 mmol) versetzt und 16 h unter Argon gerührt. Nach Zugabe von Ethanol wird der entstandene Niederschlag abfiltriert und mit kaltem Petrolether gewaschen. Ausbeute: 80%.

¹H-NMR (*d₆*-DMSO, 400 MHz): δ = 4.17 (s, 3H), 7.67-7.83 (m, 5H), 7.94 (d, 1H), 8.04-8.12 (m, 4H), 8.15 (d, 1H), 8.36 (d, 2H), 10.14 (s, 1H).

### mer-Tris-[1-(4'-phenylsulfonylphenyl)-3-methylbenzimidazol-2-yliden-C2,C2']-iridium(III)(V11)

Eine Suspension von 1-(4'-Phenylsulfonphenyl)-3-methylbenzimidazolium-tetrafluoroborat (4.4 g, 10 mmol) in Dioxan (100 mL) wird unter Argon bei Raumtemperatur mit KHMDS (0.5 M in Toluol, 20mL, 10 mmol) versetzt und 15 min gerührt. Nach Zugabe von 1,5-Cyclooctadien-iridium(I)chlorid-Dimer (0.7 g, 1 mmol) wird die Mischung 16 h unter Rückfluß gerührt. Nach Abkühlung auf Raumtemperatur wird der Niederschlag abfiltriert und mit Methyl-tert-butylether gewaschen. Die vereinigten Filtrate werden zur Trockne eingeengt und säulenchromatographisch aufgereinigt (Aluminiumoxid, Dichlormethan, Butanon). Ausbeute: 56%.

¹H-NMR (*d₆*-DMSO, 400 MHz): δ = 2.82 (s, 3H), 2.99 (s, 3H), 3.15 (s, 3H), 6.61 (d, 1H), 6.98 (d, 1 H), 7.01 (d, 1 H), 7.30-7.72 (m, 27H), 8.09-8.16 (m, 3H), 8.35-8.44 (m, 3H).

### Tris-[1-(4'-methoxycarbonylphenyl)-3-methylimidazol-2-yliden-C2,C2]-iridium(III)

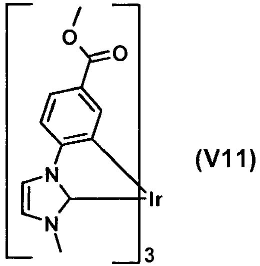

### 1-(4'-Methoxycarbonylphenyl)-imidazol

Eine Mischung von Imidazol (132 g, 1.9 mol), 4-Fluorbenzoesäuremethylester (170 mL, 1.3 mol) und Kaliumcarbonat (357 g, 2.6 mol) in DMSO (200 mL) wird 3 h bei 120 °C gerührt. Nach Abkühlung auf Raumtemperatur wird die Mischung auf Eiswasser gegeben. Ausgefallenes Produkt wird abfiltriert und mit Wasser gewaschen. Ausbeute: 59%.

¹H-NMR (*d₆*-DMSO, 400 MHz): δ = 3.89 (s, 3H), 7.17 (m_{c}, 1H), 7.86 (d,. 2H), 7.90 (m_{c}, 1H), 8.08 (d, 2H), 8.44 (m_{c}, 1H).

### 1-(4'-Methoxycarbonylphenyl)-3-methylimidazolium-iodid

Eine Lösung von 1-(4'-Methoxycarbonylphenyl)-imidazol (153 g, 0.76 mol) in THF/Methanol 1:1 (600 mL) wird mit Methyliodid (196 mL, 2.27 mol) versetzt und 16 h bei Raumtemperatur unter Argon gerührt. Nach Aufkonzentartion der Lösung wird das ausgefallene Produkt abfiltriert und mit THF gewaschen. Ausbeute: 59%.

¹H-NMR (*d₆*-DMSO, 400 MHz): δ = 3.90 (s, 3H), 3.95 (s, 3H), 7.94 (d, 2H), 7.98 (m_{c}, 1H), 8.21 (d, 2H), 8.38 (m_{c}, 1 H), 9.89 (s, 1 H).

### Tris-[1-(4'-methoxycarbonylphenyl)-3-methylimidazol-2-yliden-C2,C2']-iridium(III)

Eine Suspension von 1-(4'-Methoxycarbonylphenyl)-3-methylimidazolium-iodid (148.0 g, 431 mmol) und Silber(I)oxid (50.4 g, 216 mmol) in Dioxan (400 mL) wird 16 h bei Raumtemperatur unter Argon gerührt. Die Mischung wird mit 1,5-Cyclooctadien-iridium(I)chlorid-Dimer (33.2 g, 43 mmol) versetzt und 16 h unter Rückfluß gerührt. Nach Abkühlung auf Raumtemperatur wird der Niederschlag abfiltriert und mit Dichlormethan gewaschen. Die vereinigten Filtrate werden zur Trockne eingeengt und säulenchromatographisch aufgereinigt (Aluminiumoxid, Essigester/Methanol 1:1). Mischfraktionen, die das Produkt als mer- und fac-Isomer enthalten, werden zur Trockne eingeengt und in Aceton/Methanol 1:1 gelöst. Die Lösung wird mit 1 M Salzsäure versetzt und 16 h unter Rückfluß gerührt. Das ausgefallene mer-Isomer wird abfiltriert und mit wenig Aceton gewaschen. Ausbeute: 45%.

*mer*-isomer: ¹H-NMR (*d₆*-DMSO, 400 MHz): δ = 2.89 (s, 3H), 2.96 (s, 3H), 2.99 (s, 3H), 3.60 (s, 3H), 3.67 (s, 3H), 3.69 (s, 3H), 7.12 (s, 1 H), 7.18 (s, 1 H), 7.24 (s, 1 H), 7.27 (s, 1 H), 7.29 (s, 1 H), 7.31 (s, 1H),7.38-7.49 (m, 6H), 7.98 (s, 1 H), 8.04 (s, 1 H), 8.06 (s, 1 H).

*fac*-isomer: ¹H-NMR (*d₆*-DMSO, 400 MHz): δ = 3.00 (s, 9H), 3.60 (s, 9H), 7.06 (d, 3H), 7.21 (d, 3H), 7.43-7.51 (m, 6H), 7.99 (d, 3H).

### Iridium(III)bis [(2-phenyl)-2-pyrazolinato-N,C²)](1-phenyl-3-methyl-imidazolin-2-yliden-C,C² (V12)

Die Verbindung (V12) wird in Analogie zu Beispiel 3 in US 2005/0260441 A1 hergestellt mit dem Unterschied, dass anstelle von [(F2ppz)₂IrCI]₂ [(ppz)₂IrCI]₂ eingesetzt wird.

### B) Veriahren zur Herstellung von beispielhaften Acridinderivafen

### B1) Synthese von 9.9-Di(4-(N-carbazolyl)-phenyl)-N-Phenyl-9,10-dihydroacridin

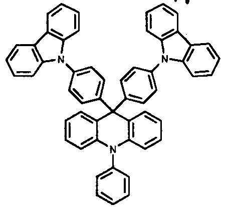

4-Bromphenyl-carbazol (2.1 g, 6.6 mmol) werden in THF (24 ml) gelöst und bei -78 °C mit tert-Butlyllithium (14.5 mmol in Hexan) versetzt. Die Temperatur wird innerhalb von 2 Stunden auf -40 °C angehoben. Darauf wird mit einer Lösung aus N,N-Diphenylanthranilsäuremethylester (1.0 g, 3.3 mmol) versetzt und über Nacht bei Raumtemperatur gerührt. Nach chromatographischer Aufreinigung an Kieselgel werden 597 mg Di-(4-N-carbazolylphenyl)-(2-(N,N-Diphenylamino)-phenyl)-methanol erhalten

Di-(4-N-carbazolylphenyl)-(2-(N,N-Diphenylamino)-phenyl)-methanol (0.12 g, 0.16 mmol) wird in Essigsäure (2 mL) gelöst und mit konzentrierter HCl-Lösung (0.1 mL) versetzt. Das Gemisch wird anschließend 1 Stunde auf 100 °C erwärmt und nach dem Abkühlen auf Raumtemperatur wässrig aufgearbeitet. Nach chromatographischer Reinigung an Kieselgel werden 99 mg 9,9-Di(4-(N-carbazolyl)-phenyl)-N-Phenyl-9,10-dihydroacridin erhalten.

¹H-NMR (400 MHz, CD₂Cl₂): δ = 6.55 (d, J = 8.2 Hz, 2 H), 7.02 (dd, J = 7.4 Hz, 2 H), 7.13-7.26 (m, 7 H), 7.29 (dd, J = 7.4 Hz, 4 H), 7.37 (d, J = 8.5 Hz, 4 H), 7.44 (dd, J = 7.7 Hz, 4 H), 7.52-7.58 (m, 8 H), 7.59-7.64 (m, 2 H), 8.16 (d, J = 8.2 Hz, 4 H).

UV (PMMA): λₘₐₓ = 236, 292, 326 (Schulter), 340 (Schulter) nm

### Weitere Beispiele (Acridinderivate)

### B2) 9,9-Di(3-(N-Ethylcarbazolyl)-N-Phenyl-9,10-dihydroacridin

Die Synthese erfolgt analog der beispielhaften Synthese gemäß B1.

¹H-NMR(400 MHz, CD₂Cl₂): δ = 1.45 (t, J = 7.1 Hz, 6 H), 4.39 (q, J = 7.1 Hz, 4 H), 6.43 (dd, J = 8.2, 1.1 Hz, 2 H), 6.88 (ddd, J = 8.0, 7.1, 1.3 Hz, 2 H), 7.02-7.12 (m, 8 H), 7.28 (dd, J = 8.8, 1.1 Hz, 2 H), 7.36 (d, J = 8.5 Hz, 2 H), 7.39-7.46 (m, 5 H), 7.48-7.53 (m, 2 H), 7.69 (d, J = 1.9 Hz, 2 H), 7.81 (ddd, J = 7.4, 1.1, 1.1 Hz, 2 H).

UV (PMMA): λₘₐₓ = 240, 270, 301, 350 (Schulter) nm

### B3) 9.9-Diphenyl-N-Phenyl-9,10-dihydroacridin

Die Synthese erfolgt analog der beispielhaften Synthese gemäß B1.

¹H-NMR(400 MHz, CD₂Cl₂): δ = 6.40 (d, J = 8.0 Hz, 2 H), 6.83-6.88 (m, 4 H), 6.99-7.05 (m, 6 H), 7.07-7.10 (m, 2 H), 7.22-7.29 (m, 6 H), 7.47 (dddd, J = 7.5, 7.5, 1.5, 1.5 Hz, 1H), 7.54 (dddd, J = 7.5, 7.5, 1.5, 1.5 Hz, 2 H).

UV (PMMA): λₘₐₓ = 234, 294 nm

### B4) 9,9-Di(2-methylphenyl)-N-Phenyl-9,10-dihydroacridin

Die Synthese erfolgt analog der beispielhaften Synthese gemäß B1.

¹H-NMR (400 MHz, CD₂Cl₂): δ = 1.89 (s, 6 H), 6,49(d, J = 8.2 Hz, 2 H), 6.58 (d, J = 8.2 Hz, 2 H), 6.85 (dddd, J = 7.4, 7.4, 1.1, 1.1 Hz, 2 H), 6.90-6.93 (m, 4 H), 7.01-7.08 (m, 4 H), 7.15-7.24 (m, 4 H), 7.41-7.52 (m, 3 H).

UV (PMMA): λₘₐₓ = 231, 288 nm

### B5) 9,9-Di(2,5-difluorphenyl)-N-Phenyl-9,10-dihydroacridin

Die Synthese erfolgt analog der beispielhaften Synthese gemäß B1.

¹H-NMR (400 MHz, CD2Cl2): δ 6.35 (d, J = 8.2 Hz, 2 H), 6.78 (dddd, J = 8.0, 8.0, 1.3, 1.3 Hz, 2 H), 6.81-6.88 (m, 4 H), 6.98 (dddd, J = 8.5, 7.4, 1.3, 1.3 Hz, 2 H), 7.20 (brd, J = 8.0 Hz, 2 H), 7.24-7.30 (m, 4 H), 7.49-7.54 (m, 1 H), 7.62 (brdd, J = 7.7, 7.7 Hz, 2 H).

UV (PMMA): λₘₐₓ = 236, 280, 324 (Schulter) nm

## Patentansprüche

1. Verwendung von Acridinderivaten der allgemeinen Formel (I) als Matrixmaterialien in einer Licht-emittierenden Schicht von organischen Leuchtdioden und/oder als Elektronenblocker in organischen Leuchtdioden 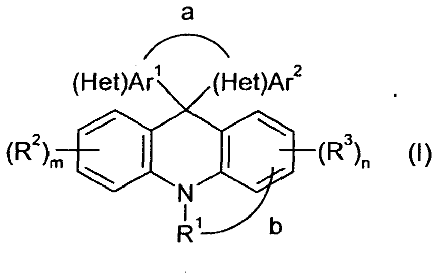 worin bedeuten:
(Het)Ar¹, (Het)Ar² unabhängig voneinander ein unsubstituierter oder substituierter Arylrest oder ein unsubstituierter oder substituierter Heteroarylrest;
R¹ ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest;
R², R³ unabhängig voneinander ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest oder ein Silylrest;
n, m unabhängig voneinander 0, 1, 2, 3 oder 4, wobei in dem Fall, wenn n bzw. m 0 sind, alle mit R² bzw. R³ substituierbaren Positionen des Acridin-Grundgerüsts Wasserstoff bedeuten; 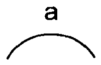
nicht vorhanden; 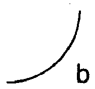 eine optionale Brücke aus 0, 1 oder 2 Atomen, die den Rest R¹ kovalent mit einem der substituierbaren Kohlenstoffatome des Acridin-Gerüsts verknüpft,
**dadurch gekennzeichnet, dass** die Acridinderivate der Formel (I) eine Energie des niedrigsten Triplettzustandes von mindestens 2,6 eV aufweisen, und als Matrixmaterialien und/oder Elektronenblocker für Triplett-Emitter eingesetzt werden.

2. Verwendung nach Anspruch 1, **dadurch gekennzeichnet, dass** der Rest R¹ ein unsubstituierter oder substituierter C₁- bis C₈-Alkylrest, der linear oder verzweigt sein kann, ein unsubstituierter oder substituierter C₆-C₁₄-Arylrest oder ein unsubstituierter oder substituierter Heteroarylrest mit 5 bis 14 Ringatomen ist.

3. Verwendung nach Anspruch 2, **dadurch gekennzeichnet, dass** der Rest R¹ ein unsubstituierter C₁- bis C₄-Alkylrest, der linear oder verzweigt sein kann, ein unsubstituierter oder substituierter Phenylrest, ein unsubstituierter oder substituierter Biphenylrest, ein unsubstituierter oder substituierter Pyridylrest, ein unsubstituierter oder substituierter Pyrimidylrest, ein unsubstituierter oder substituierter Benzofurylrest, ein unsubstituierter oder substituierter Dibenzofurylrest, ein unsubstituierter oder substituierter Benzothienylrest, ein unsubstituierter oder substituierter Dibenzothienylrest, ein unsubstituierter oder substituierter Carbazolylrest oder ein unsubstituierter oder substituierter Triazinrest ist.

4. Verwendung nach einem der Ansprüche 1 bis 3, **dadurch gekennzeichnet, dass** die Reste (Het)Ar¹ und (Het)Ar² unabhängig voneinander ausgewählt sind aus der Gruppe bestehend aus unsubstituierten oder substituierten C₆- bis C₁₄-Arylresten und unsubstituierten oder substituierten Heteroarylresten mit 5 bis 14 Ringatomen.

5. Verwendung nach Anspruch 4, **dadurch gekennzeichnet, dass** die Reste (Het)Ar¹ und (Het)Ar² ausgewählt sind aus der Gruppe bestehend aus unsubstituiertem oder substituiertem Phenyl, unsubstituiertem oder substituiertem Biphenyl, unsubstituiertem oder substituiertem Pyridyl, unsubstituiertem oder substituiertem Pyrimidyl, unsubstituiertem oder substituiertem Triazinyl, unsubstituiertem oder substituiertem Benzofuryl, unsubstituiertem oder substituiertem Benzothienyl, unsubstituiertem oder substituiertem Benzimidazolyl, unsubstituiertem oder substituiertem Dibenzofuryl, unsubstituiertem oder substituiertem Dibenzothienyl und unsubstituiertem oder substituiertem Carbazolyl.

6. Verwendung nach einem der Ansprüche 1 bis 5, **dadurch gekennzeichnet, dass** die Reste und Symbole in den Acridinderivaten der Formel (I) die folgenden Bedeutungen aufweisen:
(Het)Ar¹, (Het)Ar² unabhängig voneinander Phenyl, das unsubstituiert ist oder mit einem oder mehreren Resten ausgewählt aus der Gruppe bestehend aus CN, -OC(O)Ph, Carbazolyl, Pyridyl, CF₃, 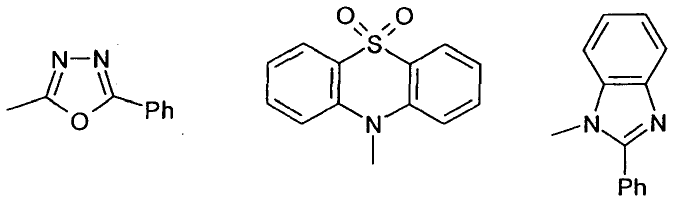 oder 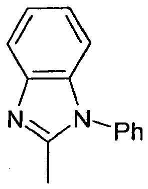 substituiert ist; Pyridyl, Pyrimidyl, Triazinyl, Oxazolyl, Oxadiazolyl, das jeweils unsubstituiert oder mit Phenyl substituiert ist; Benzimidazolyl, das unsubstituiert oder mit Phenyl substituiert ist; Benzofuryl, Dibenzofuryl, Benzothienyl, Dibenzothienyl und Carbazolyl, das jeweils unsubstituiert oder mit Phenyl substituiert ist;
R¹ Phenyl, das unsubstituiert ist oder mit einem oder mehreren Resten ausgewählt aus der Gruppe bestehend aus CH₃, CN, OCH₃, Carbazolyl, 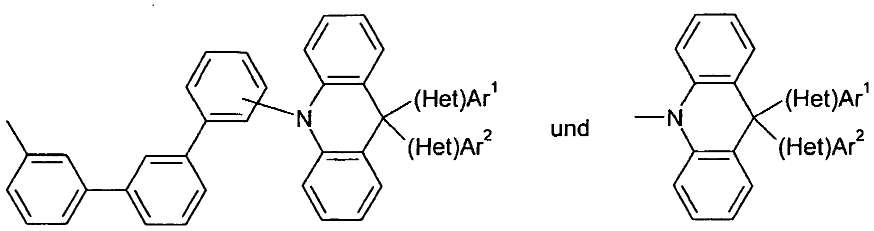 substituiert ist; Biphenyl, Benzofuryl oder ein Rest der Formel 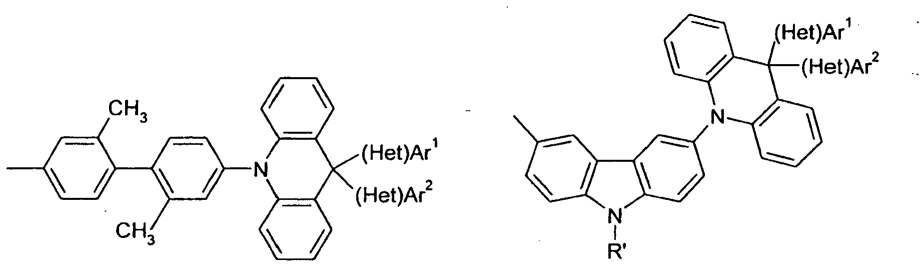 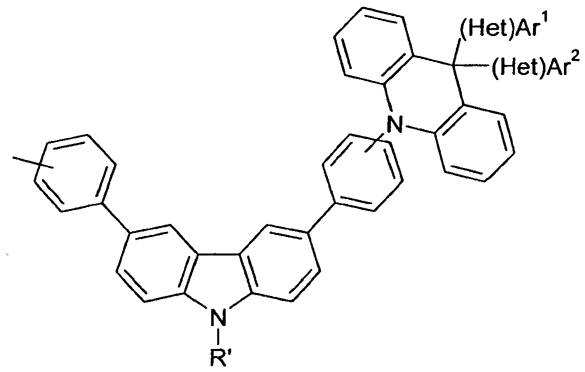 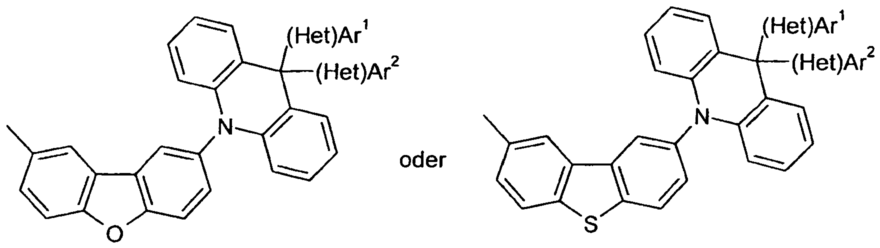 wobei (Het)Ar¹ und (Het)Ar² vorstehend definiert sind, und
R' ein unsubstituierter oder substituierter Alkylrest, der linear oder verzweigt sein kann, ein unsubstituierter oder substituierter Cycloalkylrest, ein unsubstituierter oder substituierter Heterocycloalkylrest, ein unsubstituierter oder substituierter Arylrest, ein unsubstituierter oder substituierter Heteroarylrest ist;
n, m 0; 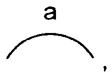 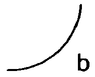
nicht vorhanden.

7. Verwendung nach einem der Ansprüche 1 bis 6, **dadurch gekennzeichnet, dass** (Het)Ar¹ und (Het)Ar² in den Acridinderivaten der Formel (I) identisch sind.

8. Licht-emittierende Schicht enthaltend mindestens einen Triplett- Emitter und mindestens ein Matrixmaterial, **dadurch gekennzeichnet, dass** als Matrixmaterial mindestens ein Acridinderivat der Formel (I) gemäß einem der Ansprüche 1 bis 7 eingesetzt wird.

9. Organische Leuchtdiode enthaltend mindestens eine Licht-emittierende Schicht gemäß Anspruch 8 oder enthaltend mindestens ein Acridinderivat der Formel (I) gemäß einem der Ansprüche 1 bis 7 in einer Blockschicht für Elektronen, wobei die Blockschicht für Elektronen in direktem Kontant mit der Licht-emittierenden Schicht der organischen Leuchtdiode steht und die Licht-emittierende Schicht der organischen Leuchtdiode mindestens einen Triplett-Emitter enthält.

10. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen und Beleuchtungseinheiten enthaltend mindestens eine organische Leuchtdiode gemäß Anspruch 9.

## Claims

1. The use of acridine derivatives of the general formula (I) as matrix materials in a light-emitting layer of organic light-emitting diodes and/or as electron blockers in organic light-emitting diodes 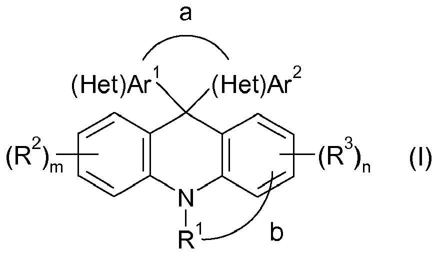 in which:
(Het)Ar¹, (Het)Ar² are each independently an unsubstituted or substituted aryl radical or an unsubstituted or substituted heteroaryl radical;
R¹ is an unsubstituted or substituted alkyl radical which may be linear or branched, an unsubstituted or substituted cycloalkyl radical, an unsubstituted or substituted heterocycloalkyl radical, an unsubstituted or substituted aryl radical, an unsubstituted or substituted heteroaryl radical;
R², R³ are each independently an unsubstituted or substituted alkyl radical which may be linear or branched, an unsubstituted or substituted cycloalkyl radical, an unsubstituted or substituted heterocycloalkyl radical, an unsubstituted or substituted aryl radical, an unsubstituted or substituted heteroaryl radical, or a silyl radical;
n, m are each independently 0, 1, 2, 3 or 4, where, in the case that n or m is 0, all positions in the acridine base skeleton substitutable by R² or R³ are hydrogen; 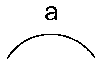
is not present; 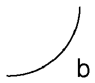 is an optional bridge composed of 0, 1 or 2 atoms which bonds the R¹ radical covalently to one of the substitutable carbon atoms of the acridine skeleton,
wherein the acridine derivatives of the formula (I) have an energy of the lowest triplet state of at least 2.6 eV and are used as matrix materials and/or electron blockers for triplet emitters.

2. The use according to claim 1, wherein the R¹ radical is an unsubstituted or substituted C₁- to C₈-alkyl radical which may be linear or branched, an unsubstituted or substituted C₆-C₁₄-aryl radical or an unsubstituted or substituted heteroaryl radical having from 5 to 14 ring atoms.

3. The use according to claim 2, wherein the R¹ radical is an unsubstituted C₁- to C₄-alkyl radical which may be linear or branched, an unsubstituted or substituted phenyl radical, an unsubstituted or substituted biphenyl radical, an unsubstituted or substituted pyridyl radical, an unsubstituted or substituted pyrimidyl radical, an unsubstituted or substituted benzofuryl radical, an unsubstituted or substituted dibenzofuryl radical, an unsubstituted or substituted benzothienyl radical, an unsubstituted or substituted dibenzothienyl radical, an unsubstituted or substituted carbazolyl radical or an unsubstituted or substituted triazine radical.

4. The use according to any of claims 1 to 3, wherein the (Het)Ar¹ and (Het)Ar² radicals are each independently selected from the group consisting of unsubstituted and substituted C₆- to C₁₄-aryl radicals and unsubstituted and substituted heteroaryl radicals having from 5 to 14 ring atoms.

5. The use according to claim 4, wherein the (Het)Ar¹ and (Het)Ar² radicals are selected from the group consisting of unsubstituted and substituted phenyl, unsubstituted and substituted biphenyl, unsubstituted and substituted pyridyl, unsubstituted and substituted pyrimidyl, unsubstituted and substituted triazinyl, unsubstituted and substituted benzofuryl, unsubstituted and substituted benzothienyl, unsubstituted and substituted benzimidazolyl, unsubstituted and substituted dibenzofuryl, unsubstituted and substituted dibenzothienyl and unsubstituted and substituted carbazolyl.

6. The use according to any of claims 1 to 5, wherein the radicals and symbols in the acridine derivatives of the formula (I) are each defined as follows:
(Het)Ar¹, (Het)Ar² are each independently phenyl which is unsubstituted or substituted by one or more radicals selected from the group consisting of CN, -OC(O)Ph, carbazolyl, pyridyl, CF₃, 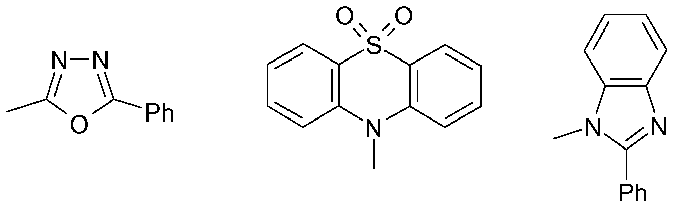 or 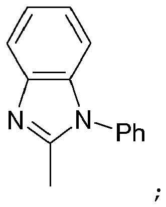 pyridyl, pyrimidyl, triazinyl, oxazolyl, oxadiazolyl, each of which is unsubstituted or substituted by phenyl; benzimidazolyl which is unsubstituted or substituted by phenyl; benzofuryl, dibenzofuryl, benzothienyl, dibenzothienyl and carbazolyl, each of which is unsubstituted or substituted by phenyl;
R¹ is phenyl which is unsubstituted or substituted by one or more radicals selected from the group consisting of CH₃, CN, OCH₃, carbazolyl, 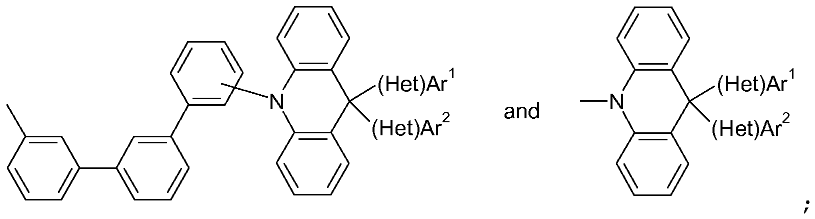 biphenyl, benzofuryl or a radical of the formula 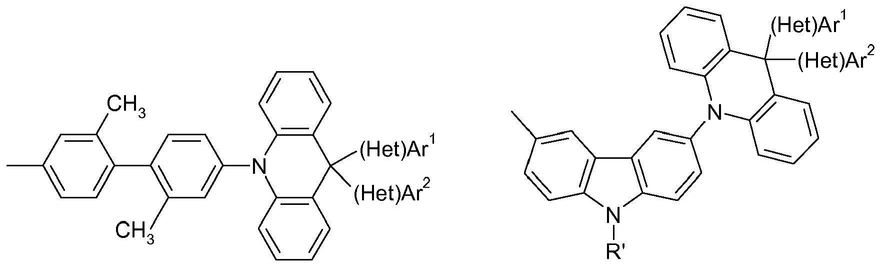 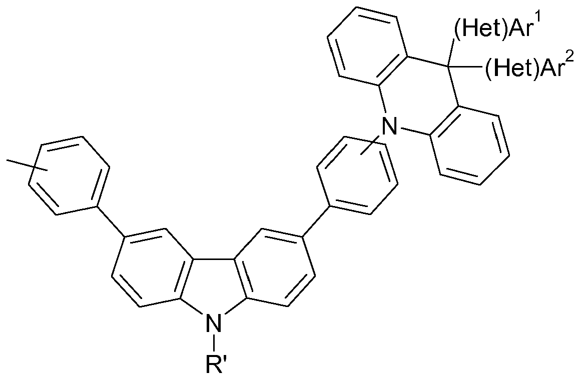 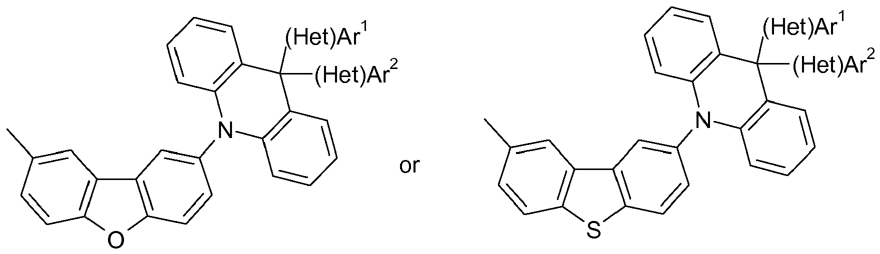 where Het)Ar¹ and (Het)Ar² are each as defined above, and
R' is an unsubstituted or substituted alkyl radical which may be linear or branched, an unsubstituted or substituted cycloalkyl radical, an unsubstituted or substituted heterocycloalkyl radical, an unsubstituted or substituted aryl radical, an unsubstituted or substituted heteroaryl radical;
n, m are each 0; 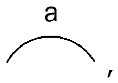 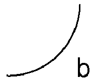
are not present.

7. The use according to any of claims 1 to 6, wherein (Het)Ar¹ and (Het)Ar² in the acridine derivatives of the formula (I) are identical.

8. A light-emitting layer comprising at least one triplet emitter and at least one matrix material, wherein the matrix material used is at least one acridine derivative of the formula (I) according to any of claims 1 to 7.

9. An organic light-emitting diode comprising at least one light-emitting layer according to claim 8 or comprising at least one acridine derivative of the formula (I) according to any of claims 1 to 7 in a blocking layer for electrons, the blocking layer for electrons being in direct contact with the light-emitting layer of the organic light-emitting diode and the light-emitting layer of the organic light-emitting diode comprising at least one triplet emitter.

10. A device selected from the group consisting of stationary visual display units such as visual display units of computers, televisions, visual display units in printers, kitchen appliances and advertising panels, illuminations, information panels and mobile visual display units such as visual display units in cellphones, laptops, digital cameras, vehicles, and destination displays on buses and trains, and illumination units, comprising at least one organic light-emitting diode according to claim 9.

## Revendications

1. Utilisation de dérivés d'acridine de formule générale (I) en tant que matériaux de matrice dans une couche photoémettrice de diodes électroluminescentes organiques et/ou en tant que bloqueurs d'électrons dans des diodes électroluminescentes organiques 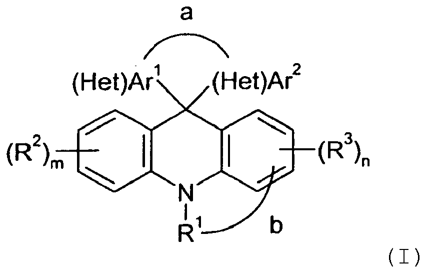 formule dans laquelle :
(Het)Ar¹, (Het)Ar² représentent indépendamment l'un de l'autre, un radical aryle substitué ou non substitué ou un radical hétéroaryle substitué ou non substitué ;
R¹ représente un radical alkyle substitué ou non substitué, qui peut être linéaire ou ramifié, un radical cycloalkyle substitué ou non substitué, un radical hétérocycloalkyle substitué ou non substitué, un radical aryle substitué ou non substitué, un radical hétéroaryle substitué ou non substitué ;
R², R³ représentent, indépendamment l'un de l'autre, un radical alkyle substitué ou non substitué, qui peut être linéaire ou ramifié, un radical cycloalkyle substitué ou non substitué, un radical hétérocycloalkyle substitué ou non substitué, un radical aryle substitué ou non substitué, un radical hétéroaryle substitué ou non substitué ou un radical silyle ;
n, m valent indépendamment l'un de l'autre 0, 1, 2, 3 ou 4, dans le cas où n ou m est égal à 0, toutes les positions substituables par R² ou, respectivement, R³ du squelette de base acridine représentent des atomes d'hydrogène ; 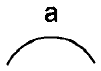
n'est pas présent ; 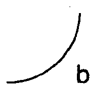 représente un pont optionnel constitué de 0, 1 ou 2 atomes qui relient le radical R¹ par liaison covalente à l'un des atomes de carbone substituables du squelette acridine,
**caractérisée en ce que** les dérivés d'acridine de formule (I) présentent une énergie du plus bas état triplet d'au moins 2,6 eV, et sont utilisés comme matériaux de matrice et/ou bloqueurs d'électrons pour émetteurs triplets.

2. Utilisation selon la revendication 1, **caractérisée en ce que** le radical R¹ est un radical alkyle en C₁-C₈ substitué ou non substitué, qui peut être linéaire ou ramifié, un radical aryle en C₆-C₁₄ substitué ou non substitué ou un radical hétéroaryle substitué ou non substitué ayant de 5 à 14 atomes formant le cycle.

3. Utilisation selon la revendication 2, **caractérisée en ce que** le radical R¹ est un radical alkyle en C₁-C₄ non substitué, qui peut être linéaire ou ramifié, un radical phényle substitué ou non substitué, un radical biphényle substitué ou non substitué, un radical pyridyle substitué ou non substitué, un radical pyrimidyle substitué ou non substitué, un radical benzofuryle substitué ou non substitué, un radical dibenzofuryle substitué ou non substitué, un radical benzothiényle substitué ou non substitué, un radical dibenzothiényle substitué ou non substitué, un radical carbazolyle substitué ou non substitué ou un radical triazine substitué ou non substitué.

4. Utilisation selon l'une quelconque des revendications 1 à 3, **caractérisée en ce que** les radicaux (Het)Ar¹ et (Het)Ar² sont choisis, indépendamment l'un de l'autre, dans le groupe constitué par des radicaux aryle en C₆-C₁₄ substitués ou non substitués et des radicaux hétéroaryle substitués ou non substitués ayant de 5 à 14 atomes de carbone formant le cycle.

5. Utilisation selon la revendication 4, **caractérisée en ce que** les radicaux (Het)Ar¹ et (Het)Ar² sont choisis dans le groupe constitué par un radical phényle substitué ou non substitué, un radical biphényle substitué ou non substitué, un radical pyridyle substitué ou non substitué, un radical pyrimidyle substitué ou non substitué, un radical triazinyle substitué ou non substitué, un radical benzofuryle substitué ou non substitué, un radical benzothiényle substitué ou non substitué, un radical benzimidazolyle substitué ou non substitué, un radical dibenzofuryle substitué ou non substitué, un radical dibenzothiényle substitué ou non substitué et un radical carbazolyle substitué ou non substitué.

6. Utilisation selon l'une quelconque des revendications 1 à 5, **caractérisée en ce que** les radicaux et symboles dans les dérivés d'acridine de formule (I) ont les significations suivantes :
(Het)Ar¹, (Het)Ar² représentent, indépendamment l'un de l'autre, un radical phényle qui est non substitué ou substitué par un ou plusieurs radicaux choisis dans le groupe constitué par CN, -OC(O)Ph, carbazolyle, pyridyle, CF₃, 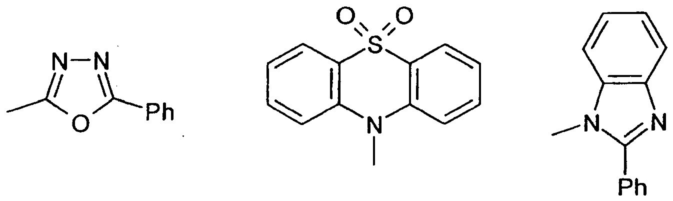 ou 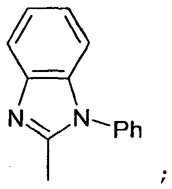 un radical pyridyle, pyrimidyle, triazinyle, oxazolyle, oxadiazolyle, qui dans chaque cas est non substitué ou substitué par phényle ; un radical benzimidazolyle qui est non substitué ou substitué par phényle ; un radical benzofuryle, dibenzofuryle, benzothiényle, dibenzothiényle et carbazolyle, qui dans chaque cas est non substitué ou substitué par phényle ;
R¹ représente un groupe phényle qui est non substitué ou substitué par un ou plusieurs radicaux choisis dans le groupe constitué par CH₃, CN, OCH₃, carbazolyle, 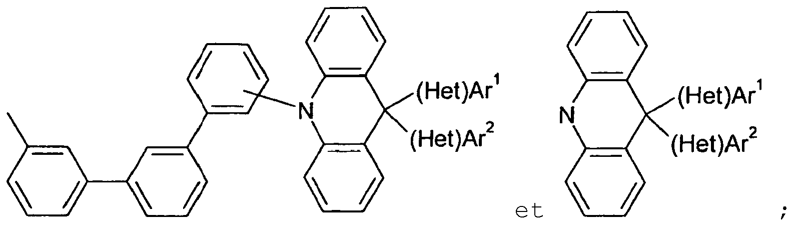 biphényle, benzofuryle ou un radical de formule 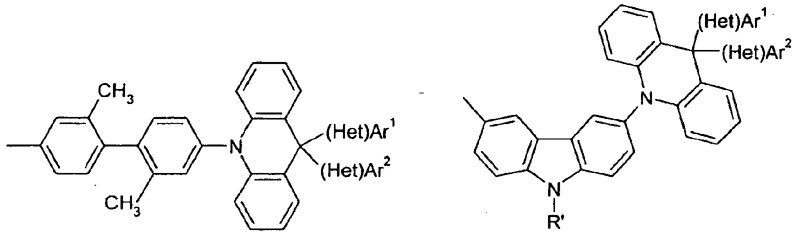 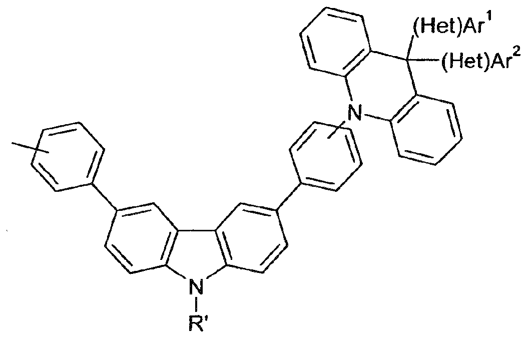 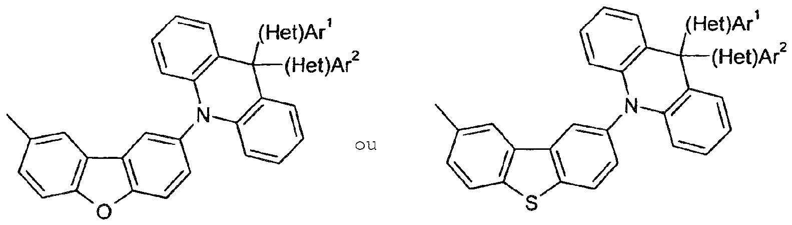 (Het)Ar¹ et (Het)Ar² étant tels que définis précédemment, et
R' étant un radical alkyle substitué ou non substitué, qui peut être linéaire ou ramifié, un radical cycloalkyle substitué ou non substitué, un radical hétérocycloalkyle substitué ou non substitué, un radical aryle substitué ou non substitué, un radical hétéroaryle substitué ou non substitué ;
n, m valent 0 ; 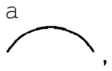 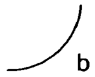
ne sont pas présents.

7. Utilisation selon l'une quelconque des revendications 1 à 6, **caractérisée en ce que** (Het)Ar¹ et (Het)Ar² dans les dérivés d'acridine de formule (I) sont identiques.

8. Couche photoémettrice contenant au moins un émetteur triplet et au moins un matériau de matrice, **caractérisée en ce qu'**on utilise comme matériau de matrice au moins un dérivé d'acridine de formule (I) selon l'une quelconque des revendications 1 à 7.

9. Diodes électroluminescentes organiques contenant au moins une couche photoémettrice selon la revendication 8 ou contenant au moins un dérivé d'acridine de formule (I) selon l'une quelconque des revendications 1 à 7 dans une couche de blocage pour électrons, la couche de blocage pour électrons étant en contact direct avec la couche photoémettrice des diodes électroluminescentes organiques et la couche photoémettrice des diodes électroluminescentes organiques contenant au moins un émetteur triplet.

10. Dispositif choisi dans le groupe constitué par des écrans fixes tels que des écrans d'ordinateurs, des téléviseurs, des écrans dans des imprimantes, des appareils de cuisine ainsi que des panneaux publicitaires, des éclairages, des tableaux indicateurs et des écrans mobiles tels que des écrans dans des téléphones portables, des ordinateurs portables, des appareils photographiques numériques, des véhicules ainsi que des afficheurs de destination dans des autobus et des trains et des unités d'éclairage contenant au moins une diode électroluminescente organique selon la revendication 9.
